# EUROPEAN PATENT APPLICATION

(11) **EP 1 254 952 A1**
(43) Date of publication of application: **06.11.2002**
(21) Application number: 00985950.5
(22) Date of filing: 27.12.2000
(51) Int. Cl.: C12N 5/06, C12N 5/08, C12P 21/08, C12Q 1/02, A61K 35/28, A61K 33/44, A61P 9/06, A61P 9/04, A61K 38/18

(54) **CELLS CAPABLE OF DIFFERENTIATING INTO HEART MUSCLE CELLS**

(30) Priority: 28.12.1999 JP 37282699; 28.02.2000 WO PCT/JP00/01148; 02.11.2000 WO PCT/JP00/07741
(71) Applicant: KYOWA HAKKO KOGYO CO., LTD., Chiyoda-ku, Tokyo 100-8185 (JP)
(72) Inventor: UMEZAWA, Akihiro, Chiba 270-0014 (JP); HATA, Jun-ichi, Tokyo 141-0031 (JP); FUKUDA, Keiichi, Tokyo 176-0006 (JP); OGAWA, Satoshi, Tokyo 157-0066 (JP); SAKURADA, Kazuhiro c/o Tokyo Research Lab., Machida-shi Tokyo 194-8533 (JP); GOJO, Satoshi, Iruma-gun Saitama 350-0414 (JP); YAMADA, Yoji c/o Tokyo Research Lab., Machida-shi Tokyo 194-8533 (JP)
(74) Representative: VOSSIUS & PARTNER
(86) International application number: JP0009323
(87) International publication number: WO01048151

(57) **Abstract**

The present invention relates to methods for isolation, purification, expansion, and differentiation of cells having the potential to differentiate into cardiomyocytes. Furthermore, the present invention relates to methods for proliferating cells having the potential to differentiate into cardiomyocytes and for regulating their differentiation into cardiomyocytes using various cytokines and transcription factors. Moreover, the present invention relates to a method for obtaining surface antigens specific for cells having the potential to differentiate into cardiomyocytes, a method for obtaining genes encoding the surface antigens, a method for obtaining antibodies specific for the surface antigens, and a method for obtaining a protein and a gene controlling the proliferation of cells having the potential to differentiation into cardiomyocytes and their differentiation into cardiomyocytes. Also, the present invention relates to therapeutic agents for various heart diseases containing cells having the potential to differentiate into cardiomyocytes. Still furthermore, the present invention relates to a method for differentiating various cells and tissues such as neural cells, hepatocytes, adipocytes, skeletal muscle cells, vascular endothelial cells and osteoblasts, using cells having the potential to differentiate into cardiomyocytes.

SEQUENCE LISTING
SEQUENCING LISTING
<110> KYOWA HAKKO KOGYO CO., LTD

<111> THE CELL HAVING THE POTENTIALITY OF DIFFERENTIATION INTO
CARDIOMYOCYTES

<130> 11217W03

<140>
<141>

<150> H11-372826
<151> 1999-12-28

<150> PCT-JP00-01148
<151> 2000-02-28

<150> PCT-JP00-07741
<151> 2000-11-02

<160>80

<170> PatentIn Ver.2.0

<210> 1
<211> 411
<212> PRT
<213> Homo sapiens
<400> 1
Met Arg Ala His Pro Gly Gly Gly Arg Cys Cys Pro Glu Gln Glu Glu
1               5                   10                  15
Gly Glu Ser Ala Ala Gly Gly Ser Gly Ala Gly Gly Asp Ser Ala Ile
            20                  25                  30
Glu Gln Gly Gly Gln Gly Ser Ala Leu Ala Pro Ser Pro Val Ser Gly
        35                  40                  45

Val Arg Arg Glu Gly Ala Arg Gly Gly Gly Arg Gly Arg Gly Arg Trp
    50                  55                  60
Lys Gln Ala Gly Arg Gly Gly Gly Val Cys Gly Arg Gly Arg Gly Arg
65                  70                  75                  80
Gly Arg Gly Arg Gly Arg Gly Arg Gly Arg Gly Arg Gly Arg Gly Arg
                85                  90                  95
Pro Pro Ser Gly Gly Ser Gly Leu Gly Gly Asp Gly Gly Gly Cys Gly
            100                 105                 110
Gly Gly Gly Ser Gly Gly Gly Gly Ala Pro Arg Arg Glu Pro Val Pro
        115                 120                 125
Phe Pro Ser Gly Ser Ala Gly Pro Gly Pro Arg Gly Pro Arg Ala Thr
    130                 135                 140
Glu Ser Gly Lys Arg Met Asp Cys Pro Ala Leu Pro Pro Gly Trp Lys
145                 150                 155                 160
Lys Glu Glu Val Ile Arg Lys Ser Gly Leu Ser Ala Gly Lys Ser Asp
                165                 170                 175
Val Tyr Tyr Phe Ser Pro Ser Gly Lys Lys Phe Arg Ser Lys Pro Gln
            180                 185                 190
Leu Ala Arg Tyr Leu Gly Asn Thr Val Asp Leu Ser Ser Phe Asp Phe
        195                 200                 205
Arg Thr Gly Lys Met Met Pro Ser Lys Leu Gln Lys Asn Lys Gln Arg
    210                 215                 220
Leu Arg Asn Asp Pro Leu Asn Gln Asn Lys Gly Lys Pro Asp Leu Asn
225                 230                 235                 240
Thr Thr Leu Pro Ile Arg Gln Thr Ala Ser Ile Phe Lys Gln Pro Val
                245                 250                 255
Thr Lys Val Thr Asn His Pro Ser Asn Lys Val Lys Ser Asp Pro Gln
            260                 265                 270
Arg Met Asn Glu Gln Pro Arg Gln Leu Phe Trp Glu Lys Arg Leu Gln
        275                 280                 285
Gly Leu Ser Ala Ser Asp Val Thr Glu Gln Ile Ile Lys Thr Met Glu
    290                 295                 300
Leu Pro Lys Gly Leu Gln Gly Val Gly Pro Gly Ser Asn Asp Glu Thr
305                 310                 315                 320
Leu Leu Ser Ala Val Ala Ser Ala Leu His Thr Ser Ser Ala Pro Ile
                325                 330                 335

Thr Gly Gln Val Ser Ala Ala Val Glu Lys Asn Pro Ala Val Trp Leu
            340                 345                 350
Asn Thr Ser Gln Pro Leu Cys Lys Ala Phe Ile Val Thr Asp Glu Asp
        355                 360                 365
Ile Arg Lys Gln Glu Glu Arg Val Gln Gln Val Arg Lys Lys Leu Glu
    370                 375                 380
Glu Ala Leu Met Ala Asp Ile Leu Ser Arg Ala Ala Asp Thr Glu Glu
385                 390                 395                 400
Met Asp Ile Glu Met Asp Ser Gly Asp Glu Ala
                405                 410
<210> 2
<211> 1233
<212> DNA
<213> Homo sapiens
<220>
<221> CDS
<223> (1)..(1236)
<400> 2
atg cgc gcg cac ccg ggg gga ggc cgc tgc tgc ccg gag cag gag gag     48
Met Arg Ala His Pro Gly Gly Gly Arg Cys Cys Pro Glu Gln Glu Glu
  1               5                  10                  15
ggg gag agt gcg gcg ggc ggc agc ggc gct ggc ggc gac tcc gcc ata     96
Gly Glu Ser Ala Ala Gly Gly Ser Gly Ala Gly Gly Asp Ser Ala Ile
             20                  25                  30
gag cag ggg ggc cag ggc agc gcg ctc gcc ccg tcc ccg gtg agc ggc    144
Glu Gln Gly Gly Gln Gly Ser Ala Leu Ala Pro Ser Pro Val Ser Gly
         35                  40                  45
gtg cgc agg gaa ggc gct cgg ggc ggc ggc cgt ggc cgg ggg cgg tgg    192
Val Arg Arg Glu Gly Ala Arg Gly Gly Gly Arg Gly Arg Gly Arg Trp
     50                  55                  60
aag cag gcg ggc cgg ggc ggc ggc gtc tgt ggc cgt ggc cgg ggc cgg    240
Lys Gln Ala Gly Arg Gly Gly Gly Val Cys Gly Arg Gly Arg Gly Arg
 65                  70                  75                  80
ggc cgt ggc cgg gga cgg gga cgg ggc cgg ggc cgg ggc cgc ggc cgt    288
Gly Arg Gly Arg Gly Arg Gly Arg Gly Arg Gly Arg Gly Arg Gly Arg
                 85                  90                  95

ccc ccg agt ggc ggc agc ggc ctt ggc ggc gac ggc ggc ggc tgc ggc 336
Pro Pro Ser Gly Gly Ser Gly Leu Gly Gly Asp Gly Gly Gly Cys Gly
            100                 105                 110
ggc ggc ggc agc ggt ggc ggc ggc gcc ccc cgg cgg gag ccg gtc cct 384
Gly Gly Gly Ser Gly Gly Gly Gly Ala Pro Arg Arg Glu Pro Val Pro
        115                 120                 125
ttc ccg tcg ggg agc gcg ggg ccg ggg ccc agg gga ccc cgg gcc acg 432
Phe Pro Ser Gly Ser Ala Gly Pro Gly Pro Arg Gly Pro Arg Ala Thr
    130                 135                 140
gag agc ggg aag agg atg gat tgc ccg gcc ctc ccc ccc gga tgg aag 480
Glu Ser Gly Lys Arg Met Asp Cys Pro Ala Leu Pro Pro Gly Trp Lys
145                 150                 155                 160
aag gag gaa gtg atc cga aaa tct ggg cta agt gct ggc aag agc gat 528
Lys Glu Glu Val Ile Arg Lys Ser Gly Leu Ser Ala Gly Lys Ser Asp
                165                 170                 175
gtc tac tac ttc agt cca agt ggt aag aag ttc aga agc aag cct cag 576
Val Tyr Tyr Phe Ser Pro Ser Gly Lys Lys Phe Arg Ser Lys Pro Gln
            180                 185                 190
ttg gca agg tac ctg gga aat act gtt gat ctc agc agt ttt gac ttc 624
Leu Ala Arg Tyr Leu Gly Asn Thr Val Asp Leu Ser Ser Phe Asp Phe
        195                 200                 205
aga act gga aag atg atg cct agt aaa tta cag aag aac aaa cag aga 672
Arg Thr Gly Lys Met Met Pro Ser Lys Leu Gln Lys Asn Lys Gln Arg
    210                 215                 220
ctg cga aac gat cct ctc aat caa aat aag ggt aaa cca gac ttg aat 720
Leu Arg Asn Asp Pro Leu Asn Gln Asn Lys Gly Lys Pro Asp Leu Asn
225                 230                 235                 240
aca aca ttg cca att aga caa aca gca tca att ttc aaa caa ccg gta 768
Thr Thr Leu Pro Ile Arg Gln Thr Ala Ser Ile Phe Lys Gln Pro Val
                245                 250                 255
acc aaa gtc aca aat cat cct agt aat aaa gtg aaa tca gac cca caa 816
Thr Lys Val Thr Asn His Pro Ser Asn Lys Val Lys Ser Asp Pro Gln
            260                 265                 270
cga atg aat gaa cag cca cgt cag ctt ttc tgg gag aag agg cta caa 864
Arg Met Asn Glu Gln Pro Arg Gln Leu Phe Trp Glu Lys Arg Leu Gln
        275                 280                 285

gga ctt agt gca tca gat gta aca gaa caa att ata aaa acc atg gaa 912
Gly Leu Ser Ala Ser Asp Val Thr Glu Gln Ile Ile Lys Thr Met Glu
    290                 295                 300
cta ccc aaa ggt ctt caa gga gtt ggt cca ggt agc aat gat gag acc 960
Leu Pro Lys Gly Leu Gln Gly Val Gly Pro Gly Ser Asn Asp Glu Thr
305                 310                 315                 320
ctt tta tct gct gtt gcc agt gct ttg cac aca agc tct gcg cca atc 1008
Leu Leu Ser Ala Val Ala Ser Ala Leu His Thr Ser Ser Ala Pro Ile
                325                 330                 335
aca ggg caa gtc tcc gct gct gtg gaa aag aac cct gct gtt tgg ctt 1056
Thr Gly Gln Val Ser Ala Ala Val Glu Lys Asn Pro Ala Val Trp Leu
            340                 345                 350
aac aca tct caa ccc ctc tgc aaa gct ttt att gtc aca gat gaa gac 1104
Asn Thr Ser Gln Pro Leu Cys Lys Ala Phe Ile Val Thr Asp Glu Asp
        355                 360                 365
atc agg aaa cag gaa gag cga gta cag caa gta cgc aag aaa ttg gaa 1152
Ile Arg Lys Gln Glu Glu Arg Val Gln Gln Val Arg Lys Lys Leu Glu
    370                 375                 380
gaa gca ctg atg gca gac atc ttg tcg cga gct gct gat aca gaa gag 1200
Glu Ala Leu Met Ala Asp Ile Leu Ser Arg Ala Ala Asp Thr Glu Glu
385                 390                 395                 400
atg gat att gaa atg gac agt gga gat gaa gcc 1233
Met Asp Ile Glu Met Asp Ser Gly Asp Glu Ala
                405                 410
<210> 3
<211> 196
<212> PRT
<213> Homo sapiens
<400> 3
Met Arg Thr Leu Ala Cys Leu Leu Leu Leu Gly Cys Gly Tyr Leu Ala
1               5                   10                  15
His Val Leu Ala Glu Glu Ala Glu Ile Pro Arg Glu Val Ile Glu Arg
            20                  25                  30
Leu Ala Arg Ser Gln Ile His Ser Ile Arg Asp Leu Gln Arg Leu Leu
        35                  40                  45
Glu Ile Asp Ser Val Gly Ser Glu Asp Ser Leu Asp Thr Ser Leu Arg

    50                  55                  60
Ala His Gly Val His Ala Thr Lys His Val Pro Glu Lys Arg Pro Leu
65                  70                  75                  80
Pro Ile Arg Arg Lys Arg Ser Ile Glu Glu Ala Val Pro Ala Val Cys
                85                  90                  95
Lys Thr Arg Thr Val Ile Tyr Glu Ile Pro Arg Ser Gln Val Asp Pro
            100                 105                 110
Thr Ser Ala Asn Phe Leu Ile Trp Pro Pro Cys Val Glu Val Lys Arg
        115                 120                 125
Cys Thr Gly Cys Cys Asn Thr Ser Ser Val Lys Cys Gln Pro Ser Arg
    130                 135                 140
Val His His Arg Ser Val Lys Val Ala Lys Val Glu Tyr Val Arg Lys
145                 150                 155                 160
Lys Pro Lys Leu Lys Glu Val Gln Val Arg Leu Glu Glu His Leu Glu
                165                 170                 175
Cys Ala Cys Ala Thr Thr Ser Leu Asn Pro Asp Tyr Arg Glu Glu Asp
            180                 185                 190
Thr Asp Val Arg
        195
<210> 4
<211> 588
<212> DNA
<213> Homo sapiens
<220>
<221> CDS
<223> (1)..(591)
<400> 4
atg agg acc ttg gct tgc ctg ctg ctc ctc ggc tgc gga tac ctc gcc 48
Met Arg Thr Leu Ala Cys Leu Leu Leu Leu Gly Cys Gly Tyr Leu Ala
1               5                   10                  15
cat gtt ctg gcc gag gaa gcc gag atc ccc cgc gag gtg atc gag agg 96
His Val Leu Ala Glu Glu Ala Glu Ile Pro Arg Glu Val Ile Glu Arg
            20                  25                  30
ctg gcc cgc agt cag atc cac agc atc cgg gac ctc cag cga ctc ctg 144
Leu Ala Arg Ser Gln Ile His Ser Ile Arg Asp Leu Gln Arg Leu Leu
        35                  40                  45

gag ata gac tcc gta ggg agt gag gat tct ttg gac acc agc ctg aga 192
Glu Ile Asp Ser Val Gly Ser Glu Asp Ser Leu Asp Thr Ser Leu Arg
    50                  55                  60
gct cac ggg gtc cac gcc act aag cat gtg ccc gag aag cgg ccc ctg 240
Ala His Gly Val His Ala Thr Lys His Val Pro Glu Lys Arg Pro Leu
65                  70                  75                  80
ccc att cgg agg aag aga agc atc gag gaa gct gtc ccc gct gtc tgc 288
Pro Ile Arg Arg Lys Arg Ser Ile Glu Glu Ala Val Pro Ala Val Cys
                85                  90                  95
aag acc agg acg gtc att tac gag att cct cgg agt cag gtc gac ccc 336
Lys Thr Arg Thr Val Ile Tyr Glu Ile Pro Arg Ser Gln Val Asp Pro
            100                 105                 110
acg tcc gcc aac ttc ctg atc tgg ccc ccg tgc gtg gag gtg aaa cgc 384
Thr Ser Ala Asn Phe Leu Ile Trp Pro Pro Cys Val Glu Val Lys Arg
        115                 120                 125
tgc acc ggc tgc tgc aac acg agc agt gtc aag tgc cag ccc tcc cgc 432
Cys Thr Gly Cys Cys Asn Thr Ser Ser Val Lys Cys Gln Pro Ser Arg
    130                 135                 140
gtc cac cac cgc agc gtc aag gtg gcc aag gtg gaa tac gtc agg aag 480
Val His His Arg Ser Val Lys Val Ala Lys Val Glu Tyr Val Arg Lys
145                 150                 155                 160
aag cca aaa tta aaa gaa gtc cag gtg agg tta gag gag cat ttg gag 528
Lys Pro Lys Leu Lys Glu Val Gln Val Arg Leu Glu Glu His Leu Glu
                165                 170                 175
tgc gcc tgc gcg acc aca agc ctg aat ccg gat tat cgg gaa gag gac 576
Cys Ala Cys Ala Thr Thr Ser Leu Asn Pro Asp Tyr Arg Glu Glu Asp
            180                 185                 190
acg gat gtg agg 588
Thr Asp Val Arg
        195
<210> 5
<211> 241
<212> PRT
<213> Homo sapiens
<400> 5
<210> 6
<211> 723
<212> DNA
<213> Homo sapiens
<220>
<221> CDS

<223> (1)..(726)
<400> 6
atg aat cgc tgc tgg gcg ctc ttc ctg tct ctc tgc tgc tac ctg cgt    48
ctg gtc agc gcc gag ggg gac ccc att ccc gag gag ctt tat gag atg    96
ctg agt gac cac tcg atc cgc tcc ttt gat gat ctc caa cgc ctg ctg    144
cac gga gac ccc gga gag gaa gat ggg gcc gag ttg gac ctg aac atg    192
acc cgc tcc cac tct gga ggc gag ctg gag agc ttg gct cgt gga aga    240
agg agc ctg ggt tcc ctg acc att gct gag ccg gcc atg atc gcc gag    288
tgc aag acg cgc acc gag gtg ttc gag atc tcc cgg cgc ctc ata gac    336
cgc acc aac gcc aac ttc ctg gtg tgg ccg ccc tgt gtg gag gtg cag    384
cgc tgc tcc ggc tgc tgc aac aac cgc aac gtg cag tgc cgc ccc acc    432
cag gtg cag ctg cga cct gtc cag gtg aga aag atc gag att gtg cgg    480
aag aag cca atc ttt aag aag gcc acg gtg acg ctg gaa gac cac ctg    528
gca tgc aag tgt gag aca gtg gca gct gca cgg cct gtg acc cga agc    576
ccg ggg ggt tcc cag gag cag cga gcc aaa acg ccc caa act cgg gtg 624
acc att cgg acg gtg cga gtc cgc cgg ccc ccc aag ggc aag cac cgg 672
aaa ttc aag cac acg cat gac aag acg gca ctg aag gag acc ctt gga 720
gcc 723
<210> 7
<211> 155
<212> PRT
<213> Homo sapiens
<400> 7
<210> 8
<211> 465
<212> DNA
<213> Homo sapiens
<220>
<221> CDS
<223> (1)..(468)
<400> 8
atg gca gcc ggg agc atc acc acg ctg ccc gcc ttg ccc gag gat ggc    48
ggc agc ggc gcc ttc ccg ccc ggc cac ttc aag gac ccc aag cgg ctg    96
tac tgc aaa aac ggg ggc ttc ttc ctg cgc atc cac ccc gac ggc cga    144
gtt gac ggg gtc cgg gag aag agc gac cct cac atc aag cta caa ctt    192
caa gca gaa gag aga gga gtt gtg tct atc aaa gga gtg tgt gct aac    240
cgt tac ctg gct atg aag gaa gat gga aga tta ctg gct tct aaa tgt    288
gtt acg gat gag tgt ttc ttt ttt gaa cga ttg gaa tct aat aac tac    336
aat act tac cgg tca agg aaa tac acc agt tgg tat gtg gca ttg aaa    384
cga act ggg cag tat aaa ctt gga tcc aaa aca gga cct ggg cag aaa    432
gct ata ctt ttt ctt cca atg tct gct aag agc 465
<210> 9
<211> 324
<212> PRT
<213> Homo sapiens
<400> 9

<210> 10
<211> 972
<212> DNA
<213> Homo sapiens
<220>
<221> CDS
<223> (1)..(975)
<400> 10
atg ttc ccc agc cct gct ctc acg ccc acg ccc ttc tca gtc aaa gac     48

  1               5                  10                  15
atc cta aac ctg gaa cag cag cag cgc agc ctg gct gcc gcc gga gag     96

             20                  25                  30
ctc tct gcc cgc ctg gag gcg acc ctg gcg ccc tcc tcc tgc atg ctg    144

         35                  40                  45
gcc gcc ttc aag cca gag gcc tac gct ggg ccc gag gcg gct gcg ccg    192

     50                  55                  60
ggc ctc cca gag ctg cgc gca gag ctg ggc cgc gcg cct tca ccg gcc    240


65                  70                  75                  80
aag tgt gcg tct gcc ttt ccc gcc gcc ccc gcc ttc tat cca cgt gcc     288

                85                  90                  95
tac agc gac ccc gac cca gcc aag gac cct aga gcc gaa aag aaa gag     336

            100                 105                 110
ctg tgc gcg ctg cag aag gcg gtg gag ctg gag aag aca gag gcg gac     384

        115                 120                 125
aac gcg gag cgg ccc cgg gcg cga cgg cgg agg aag ccg cgc gtg ctc     432

    130                 135                 140
ttc tcg cag gcg cag gtc tat gag ctg gag cgg cgc ttc aag cag cag     480

145                 150                 155                 160
cgg tac ctg tcg gcc ccc gaa cgc gac cag ctg gcc agc gtg ctg aaa     528

                165                 170                 175
ctc acg tcc acg cag gtc aag atc tgg ttc cag aac cgg cgc tac aag     576

            180                 185                 190
tgc aag cgg cag cgg cag gac cag act ctg gag ctg gtg ggg ctg ccc     624

        195                 200                 205
ccg ccg ccg ccg ccg cct gcc cgc agg atc gcg gtg cca gtg ctg gtg     672

    210                 215                 220
cgc gat ggc aag cca tgc cta ggg gac tcg gcg ccc tac gcg cct gcc     720

225                 230                 235                 240
tac ggc gtg ggc ctc aat ccc tac ggt tat aac gcc tac ccc gcc tat     768

                245                 250                 255
ccg ggt tac ggc ggc gcg gcc tgc agc cct ggc tac agc tgc act gcc     816


            260                 265                 270
gct tac ccc gcc ggg cct tcc cca gcg cag ccg gcc act gcc gcc gcc 864

        275                 280                 285
aac aac aac ttc gtg aac ttc ggc gtc ggg gac ttg aat gcg gtt cag 912

    290                 295                 300
agc ccc ggg att ccg cag agc aac tcg gga gtg tcc acg ctg cat ggt 960

305                 310                 315                 320
atc cga gcc tgg 972

324
<210> 11
<211> 442
<212> PRT
<213> Homo sapiens
<400> 11
<210> 12
<211> 1326
<212> DNA
<213> Homo sapiens
<220>
<221> CDS
<223> (1)..(1329)
<400> 12
atg tat cag agc ttg gcc atg gcc gcc aac cac ggg ccg ccc ccc ggt 48
gcc tac cag gcg ggc ggc ccc ggc ccc ttc atg cac ggc gcg ggc gcc 96
gcg tcc tcg cca gtc tac ctg ccc aca ccg cgg gtg ccc tcc tcc gtt 144
ctg ggc ctg tcc tac ctc cag ggc gga ggc gcg ggc tct gcg tcc gga 192
ggc ccc tcg ggc ggc agc ccc ggt ggg gcc gcg tct ggt gcg ggg ccc 240
ggg acc cag cag ggc agc ccg gga tgg agc cag gcg gga gcg acc gga 288
gcc gct tac acc ccg ccg ccg gtg tcg ccg cgc ttc tcc ttc ccg ggg 336
acc acc ggg tcc ctg gcg gcg gcg gcg gcg gct gcc gcc gcc cgg gaa 384
gct gcg gcc tac agc agt ggc ggc gga gcg gcg ggt gcg ggc ctg gcg 432
ggc cgc gag cag tac ggg cgc gcc ggc ttc gcg ggc tcc tac tcc agc 480
ccc tac ccg gct tac atg gcc gac gtg ggc gcg tcc tgg gcc gca gcc 528
gcc gcc gcc tcc gcc ggc ccc ttc gac agc ccg gtc ctg cac agc ctg 576
ccc ggc cgg gcc aac ccg gcc gcc cga cac ccc aat ctc gat atg ttt 624
gac gac ttc tca gaa ggc aga gag tgt gtc aac tgt ggg gct atg tcc 672
acc ccg ctc tgg agg cga gat ggg acg ggt cac tat ctg tgc aac gcc 720
tgt ggc ctc tac cac aag atg aac ggc atc aac cgg ccg ctc atc aag 768
cct cag cgc cgg ctg tcc gcc tcc cgc cga gtg ggc ctc tcc tgt gcc 816
aac tgc cag acc acc acc acc acg ctg tgg cgc cgc aat gcg gag ggc 864
gag cct gtg tgc aat gcc tgc ggc ctc tac atg aag ctc cac ggg gtg 912
ccc agg cct ctt gca atg cgg aaa gag ggg atc caa acc aga aaa cgg 960
aag ccc aag aac ctg aat aaa tct aag aca cca gca gct cct tca ggc 1008
agt gag agc ctt cct ccc gcc agc ggt gct tcc agc aac tcc agc aac 1056
gcc acc acc agc agc agc gag gag atg cgt ccc atc aag acg gag cct 1104
ggc ctg tca tct cac tac ggg cac agc agc tcc gtg tcc cag acg ttc 1152
tca gtc agt gcg atg tct ggc cat ggg ccc tcc atc cac cct gtc ctc 1200
tcg gcc ctg aag ctc tcc cca caa ggc tat gcg tct ccc gtc agc cag 1248
tct cca cag acc agc tcc aag cag gac tct tgg aac agt ctg gtc ttg 1296
gcc gac agt cac ggg gac ata atc act gcg 1326
<210> 13
<211> 507
<212> PRT
<213> Homo sapiens
<400> 13
Met Gly Arg Lys Lys Ile Gln Ile Thr Arg Ile Met Asp Glu Arg Asn
1               5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Ser Asn Lys Leu Phe Gln Tyr Ala Ser Thr Asp Met Asp Lys
    50                  55                  60

Val Leu Leu Lys Tyr Thr Glu Tyr Asn Glu Pro His Glu Ser Arg Thr
65                  70                  75                  80
Asn Ser Asp Ile Val Glu Ala Leu Asn Lys Lys Glu His Arg Gly Cys
                85                  90                  95
Asp Ser Pro Asp Pro Asp Thr Ser Tyr Val Leu Thr Pro His Thr Glu
            100                 105                 110
Glu Lys Tyr Lys Lys Ile Asn Glu Glu Phe Asp Asn Met Met Arg Asn
        115                 120                 125
His Lys Ile Ala Pro Gly Leu Pro Pro Gln Asn Phe Ser Met Ser Val
    130                 135                 140
Thr Val Pro Val Thr Ser Pro Asn Ala Leu Ser Tyr Thr Asn Pro Gly
145                 150                 155                 160
Ser Ser Leu Val Ser Pro Ser Leu Ala Ala Ser Ser Thr Leu Thr Asp
                165                 170                 175
Ser Ser Met Leu Ser Pro Pro Gln Thr Thr Leu His Arg Asn Val Ser
            180                 185                 190
Pro Gly Ala Pro Gln Arg Pro Pro Ser Thr Gly Asn Ala Gly Gly Met
        195                 200                 205
Leu Ser Thr Thr Asp Leu Thr Val Pro Asn Gly Ala Gly Ser Ser Pro
    210                 215                 220
Val Gly Asn Gly Phe Val Asn Ser Arg Ala Ser Pro Asn Leu Ile Gly
225                 230                 235                 240
Ala Thr Gly Ala Asn Ser Leu Gly Lys Val Met Pro Thr Lys Ser Pro
                245                 250                 255
Pro Pro Pro Gly Gly Gly Asn Leu Gly Met Asn Ser Arg Lys Pro Asp
            260                 265                 270
Leu Arg Val Val Ile Pro Pro Ser Ser Lys Gly Met Met Pro Pro Leu
        275                 280                 285
Ser Glu Glu Glu Glu Leu Glu Leu Asn Thr Gln Arg Ile Ser Ser Ser
    290                 295                 300
Gln Ala Thr Gln Pro Leu Ala Thr Pro Val Val Ser Val Thr Thr Pro
305                 310                 315                 320
Ser Leu Pro Pro Gln Gly Leu Val Tyr Ser Ala Met Pro Thr Ala Tyr
                325                 330                 335
Asn Thr Asp Tyr Ser Leu Thr Ser Ala Asp Leu Ser Ala Leu Gln Gly
            340                 345                 350

Phe Asn Ser Pro Gly Met Leu Ser Leu Gly Gln Val Ser Ala Trp Gln
        355                 360                 365
Gln His His Leu Gly Gln Ala Ala Leu Ser Ser Leu Val Ala Gly Gly
    370                 375                 380
Gln Leu Ser Gln Gly Ser Asn Leu Ser Ile Asn Thr Asn Gln Asn Ile
385                 390                 395                 400
Ser Ile Lys Ser Glu Pro Ile Ser Pro Pro Arg Asp Arg Met Thr Pro
                405                 410                 415
Ser Gly Phe Gln Gln Gln Gln Gln Gln Gln Gln Gln Gln Gln Pro Pro
            420                 425                 430
Pro Pro Pro Gln Pro Gln Pro Gln Pro Pro Gln Pro Gln Pro Arg Gln
        435                 440                 445
Glu Met Gly Arg Ser Pro Val Asp Ser Leu Ser Ser Ser Ser Ser Ser
    450                 455                 460
Tyr Asp Gly Ser Asp Arg Glu Asp Pro Arg Gly Asp Phe His Ser Pro
465                 470                 475                 480
Ile Val Leu Gly Arg Pro Pro Asn Thr Glu Asp Arg Glu Ser Pro Ser
                485                 490                 495
Val Lys Arg Met Arg Met Asp Ala Trp Val Thr
            500                 505
<210> 14
<211> 1521
<212> DNA
<213> Homo sapiens
<220>
<221> CDS
<223> (1)..(1524)
<400> 14
atg ggg cgg aag aaa ata caa atc aca cgc ata atg gat gaa agg aac 48
Met Gly Arg Lys Lys Ile Gln Ile Thr Arg Ile Met Asp Glu Arg Asn
1               5                   10                  15
cga cag gtc act ttt aca aag aga aag ttt gga tta atg aag aaa gcc 96
Arg Gln Val Thr Phe Thr Lys Arg Lys Phe Gly Leu Met Lys Lys Ala
            20                  25                  30
tat gaa ctt agt gtg ctc tgt gac tgt gaa ata gca ctc atc att ttc 144
Tyr Glu Leu Ser Val Leu Cys Asp Cys Glu Ile Ala Leu Ile Ile Phe

        35                  40                  45
aac agc tct aac aaa ctg ttt caa tat gct agc act gat atg gac aaa 192
Asn Ser Ser Asn Lys Leu Phe Gln Tyr Ala Ser Thr Asp Met Asp Lys
    50                  55                  60
gtt ctt ctc aag tat aca gaa tat aat gaa cct cat gaa agc aga acc 240
Val Leu Leu Lys Tyr Thr Glu Tyr Asn Glu Pro His Glu Ser Arg Thr
65                  70                  75                  80
aac tcg gat att gtt gag gct ctg aac aag aag gaa cac aga ggg tgc 288
Asn Ser Asp Ile Val Glu Ala Leu Asn Lys Lys Glu His Arg Gly Cys
                85                  90                  95
gac agc cca gac cct gat act tca tat gtg cta act cca cat aca gaa 336
Asp Ser Pro Asp Pro Asp Thr Ser Tyr Val Leu Thr Pro His Thr Glu
            100                 105                 110
gaa aaa tat aaa aaa att aat gag gaa ttt gat aat atg atg cgg aat 384
Glu Lys Tyr Lys Lys Ile Asn Glu Glu Phe Asp Asn Met Met Arg Asn
        115                 120                 125
cat aaa atc gca cct ggt ctg cca cct cag aac ttt tca atg tct gtc 432
His Lys Ile Ala Pro Gly Leu Pro Pro Gln Asn Phe Ser Met Ser Val
    130                 135                 140
aca gtt cca gtg acc agc ccc aat gct ttg tcc tac act aac cca ggg 480
Thr Val Pro Val Thr Ser Pro Asn Ala Leu Ser Tyr Thr Asn Pro Gly
145                 150                 155                 160
agt tca ctg gtg tcc cca tct ttg gca gcc agc tca acg tta aca gat 528
Ser Ser Leu Val Ser Pro Ser Leu Ala Ala Ser Ser Thr Leu Thr Asp
                165                 170                 175
tca agc atg ctc tct cca cct caa acc aca tta cat aga aat gtg tct 576
Ser Ser Met Leu Ser Pro Pro Gln Thr Thr Leu His Arg Asn Val Ser
            180                 185                 190
cct gga gct cct cag aga cca cca agt act ggc aat gca ggt ggg atg 624
Pro Gly Ala Pro Gln Arg Pro Pro Ser Thr Gly Asn Ala Gly Gly Met
        195                 200                 205
ttg agc act aca gac ctc aca gtg cca aat gga gct gga agc agt cca 672
Leu Ser Thr Thr Asp Leu Thr Val Pro Asn Gly Ala Gly Ser Ser Pro
    210                 215                 220
gtg ggg aat gga ttt gta aac tca aga gct tct cca aat ttg att gga 720
Val Gly Asn Gly Phe Val Asn Ser Arg Ala Ser Pro Asn Leu Ile Gly

225                 230                 235                 240
gct act ggt gca aat agc tta ggc aaa gtc atg cct aca aag tct ccc     768
Ala Thr Gly Ala Asn Ser Leu Gly Lys Val Met Pro Thr Lys Ser Pro
                245                 250                 255
cct cca cca ggt ggt ggt aat ctt gga atg aac agt agg aaa cca gat     816
Pro Pro Pro Gly Gly Gly Asn Leu Gly Met Asn Ser Arg Lys Pro Asp
            260                 265                 270
ctt cga gtt gtc atc ccc cct tca agc aag ggc atg atg cct cca cta     864
Leu Arg Val Val Ile Pro Pro Ser Ser Lys Gly Met Met Pro Pro Leu
        275                 280                 285
tcg gag gaa gag gaa ttg gag ttg aac acc caa agg atc agt agt tct     912
Ser Glu Glu Glu Glu Leu Glu Leu Asn Thr Gln Arg Ile Ser Ser Ser
    290                 295                 300
caa gcc act caa cct ctt gct acc cca gtc gtg tct gtg aca acc cca     960
Gln Ala Thr Gln Pro Leu Ala Thr Pro Val Val Ser Val Thr Thr Pro
305                 310                 315                 320
agc ttg cct ccg caa gga ctt gtg tac tca gca atg ccg act gcc tac     1008
Ser Leu Pro Pro Gln Gly Leu Val Tyr Ser Ala Met Pro Thr Ala Tyr
                325                 330                 335
aac act gat tat tca ctg acc agc gct gac ctg tca gcc ctt caa ggc     1056
Asn Thr Asp Tyr Ser Leu Thr Ser Ala Asp Leu Ser Ala Leu Gln Gly
            340                 345                 350
ttc aac tcg cca gga atg ctg tcg ctg gga cag gtg tcg gcc tgg cag     1104
Phe Asn Ser Pro Gly Met Leu Ser Leu Gly Gln Val Ser Ala Trp Gln
        355                 360                 365
cag cac cac cta gga caa gca gcc ctc agc tct ctt gtt gct gga ggg     1152
Gln His His Leu Gly Gln Ala Ala Leu Ser Ser Leu Val Ala Gly Gly
    370                 375                 380
cag tta tct cag ggt tcc aat tta tcc att aat acc aac caa aac atc     1200
Gln Leu Ser Gln Gly Ser Asn Leu Ser Ile Asn Thr Asn Gln Asn Ile
385                 390                 395                 400
agc atc aag tcc gaa ccg att tca cct cct cgg gat cgt atg acc cca     1248
Ser Ile Lys Ser Glu Pro Ile Ser Pro Pro Arg Asp Arg Met Thr Pro
                405                 410                 415
tcg ggc ttc cag cag cag cag cag cag cag cag cag cag cag ccg ccg     1296
Ser Gly Phe Gln Gln Gln Gln Gln Gln Gln Gln Gln Gln Gln Pro Pro

                420                 425                 430
cca cca ccg cag ccc cag cca caa ccc ccg cag ccc cag ccc cga cag    1344
Pro Pro Pro Gln Pro Gln Pro Gln Pro Pro Gln Pro Gln Pro Arg Gln
            435                 440                 445
gaa atg ggg cgc tcc cct gtg gac agt ctg agc agc tct agt agc tcc    1392
Glu Met Gly Arg Ser Pro Val Asp Ser Leu Ser Ser Ser Ser Ser Ser
        450                 455                 460
tat gat ggc agt gat cgg gag gat cca cgg ggc gac ttc cat tct cca    1440
Tyr Asp Gly Ser Asp Arg Glu Asp Pro Arg Gly Asp Phe His Ser Pro
465                 470                 475                 480
att gtg ctt ggc cga ccc cca aac act gag gac aga gaa agc cct tct    1488
Ile Val Leu Gly Arg Pro Pro Asn Thr Glu Asp Arg Glu Ser Pro Ser
                485                 490                 495
gta aag cga atg agg atg gac gcg tgg gtg acc                        1521
Val Lys Arg Met Arg Met Asp Ala Trp Val Thr
                500                 505
<210> 15
<211> 365
<212> PRT
<213> Homo sapiens
<400> 15
Met Gly Arg Lys Lys Ile Gln Ile Ser Arg Ile Leu Asp Gln Arg Asn
  1               5                  10                  15
Arg Gln Val Thr Phe Thr Lys Arg Lys Phe Gly Leu Met Lys Lys Ala
                 20                  25                  30
Tyr Glu Leu Ser Val Leu Cys Asp Cys Glu Ile Ala Leu Ile Ile Phe
             35                  40                  45
Asn Ser Ala Asn Arg Leu Phe Gln Tyr Ala Ser Thr Asp Met Asp Arg
         50                  55                  60
Val Leu Leu Lys Tyr Thr Glu Tyr Ser Glu Pro His Glu Ser Arg Thr
 65                  70                  75                  80
Asn Thr Asp Ile Leu Glu Thr Leu Lys Arg Arg Gly Ile Gly Leu Asp
                 85                  90                  95
Gly Pro Glu Leu Glu Pro Asp Glu Gly Pro Glu Glu Pro Gly Glu Lys
                100                 105                 110
Phe Arg Arg Leu Ala Gly Glu Gly Gly Asp Pro Ala Leu Pro Arg Pro

115                 120                 125
Arg Leu Tyr Pro Ala Ala Pro Ala Met Pro Ser Pro Asp Val Val Tyr
    130                 135                 140
Gly Ala Leu Pro Pro Pro Gly Cys Asp Pro Ser Gly Leu Gly Glu Ala
145                 150                 155                 160
Leu Pro Ala Gln Ser Arg Pro Ser Pro Phe Arg Pro Ala Ala Pro Lys
                165                 170                 175
Ala Gly Pro Pro Gly Leu Val His Pro Leu Phe Ser Pro Ser His Leu
            180                 185                 190
Thr Ser Lys Thr Pro Pro Pro Leu Tyr Leu Pro Thr Glu Gly Arg Arg
        195                 200                 205
Ser Asp Leu Pro Gly Gly Leu Ala Gly Pro Arg Gly Gly Leu Asn Thr
    210                 215                 220
Ser Arg Ser Leu Tyr Ser Gly Leu Gln Asn Pro Cys Ser Thr Ala Thr
225                 230                 235                 240
Pro Gly Pro Pro Leu Gly Ser Phe Pro Phe Leu Pro Gly Gly Pro Pro
                245                 250                 255
Val Gly Ala Glu Ala Trp Ala Arg Arg Val Pro Gln Pro Ala Ala Pro
            260                 265                 270
Pro Arg Arg Pro Pro Gln Ser Ala Ser Ser Leu Ser Ala Ser Leu Arg
        275                 280                 285
Pro Pro Gly Ala Pro Ala Thr Phe Leu Arg Pro Ser Pro Ile Pro Cys
    290                 295                 300
Ser Ser Pro Gly Pro Trp Gln Ser Leu Cys Gly Leu Gly Pro Pro Cys
305                 310                 315                 320
Ala Gly Cys Pro Trp Pro Thr Ala Gly Pro Gly Arg Arg Ser Pro Gly
                325                 330                 335
Gly Thr Ser Pro Glu Arg Ser Pro Gly Thr Ala Arg Ala Arg Gly Asp
            340                 345                 350
Pro Thr Ser Leu Gln Ala Ser Ser Glu Lys Thr Gln Gln
        355                 360
<210> 16
<211> 1095
<212> DNA
<213> Homo sapiens
<220>

<221> CDS
<223> (1)..(1098)
<400> 16
atg ggg agg aaa aaa atc cag atc tcc cgc atc ctg gac caa agg aat 48
Met Gly Arg Lys Lys Ile Gln Ile Ser Arg Ile Leu Asp Gln Arg Asn
1               5                   10                  15
cgg cag gtg acg ttc acc aag cgg aag ttc ggg ctg atg aag aag gcc 96
Arg Gln Val Thr Phe Thr Lys Arg Lys Phe Gly Leu Met Lys Lys Ala
            20                  25                  30
tat gag ctg agc gtg ctc tgt gac tgt gag ata gcc ctc atc atc ttc 144
Tyr Glu Leu Ser Val Leu Cys Asp Cys Glu Ile Ala Leu Ile Ile Phe
        35                  40                  45
aac agc gcc aac cgc ctc ttc cag tat gcc agc acg gac atg gac cgt 192
Asn Ser Ala Asn Arg Leu Phe Gln Tyr Ala Ser Thr Asp Met Asp Arg
    50                  55                  60
gtg ctg ctg aag tac aca gag tac agc gag ccc cac gag agc cgc acc 240
Val Leu Leu Lys Tyr Thr Glu Tyr Ser Glu Pro His Glu Ser Arg Thr
65                  70                  75                  80
aac act gac atc ctc gag acg ctg aag cgg agg ggc att ggc ctc gat 288
Asn Thr Asp Ile Leu Glu Thr Leu Lys Arg Arg Gly Ile Gly Leu Asp
                85                  90                  95
ggg cca gag ctg gag ccg gat gaa ggg cct gag gag cca gga gag aag 336
Gly Pro Glu Leu Glu Pro Asp Glu Gly Pro Glu Glu Pro Gly Glu Lys
            100                 105                 110
ttt cgg agg ctg gca ggc gaa ggg ggt gat ccg gcc ttg ccc cga ccc 384
Phe Arg Arg Leu Ala Gly Glu Gly Gly Asp Pro Ala Leu Pro Arg Pro
        115                 120                 125
cgg ctg tat cct gca gct cct gct atg ccc agc cca gat gtg gta tac 432
Arg Leu Tyr Pro Ala Ala Pro Ala Met Pro Ser Pro Asp Val Val Tyr
    130                 135                 140
ggg gcc tta ccg cca cca ggc tgt gac ccc agt ggg ctt ggg gaa gca 480
Gly Ala Leu Pro Pro Pro Gly Cys Asp Pro Ser Gly Leu Gly Glu Ala
145                 150                 155                 160
ctg ccc gcc cag agc cgc cca tct ccc ttc cga cca gca gcc ccc aaa 528
Leu Pro Ala Gln Ser Arg Pro Ser Pro Phe Arg Pro Ala Ala Pro Lys
                165                 170                 175

gcc ggg ccc cca ggc ctg gtg cac cct ctc ttc tca cca agc cac ctc 576
Ala Gly Pro Pro Gly Leu Val His Pro Leu Phe Ser Pro Ser His Leu
            180                 185                 190
acc agc aag aca cca ccc cca ctg tac ctg ccg acg gaa ggg cgg agg 624
Thr Ser Lys Thr Pro Pro Pro Leu Tyr Leu Pro Thr Glu Gly Arg Arg
        195                 200                 205
tca gac ctg cct ggt ggc ctg gct ggg ccc cga ggg gga cta aac acc 672
Ser Asp Leu Pro Gly Gly Leu Ala Gly Pro Arg Gly Gly Leu Asn Thr
    210                 215                 220
tcc aga agc ctc tac agt ggc ctg cag aac ccc tgc tcc act gca act 720
Ser Arg Ser Leu Tyr Ser Gly Leu Gln Asn Pro Cys Ser Thr Ala Thr
225                 230                 235                 240
ccc gga ccc cca ctg ggg agc ttc ccc ttc ctc ccc gga ggc ccc cca 768
Pro Gly Pro Pro Leu Gly Ser Phe Pro Phe Leu Pro Gly Gly Pro Pro
                245                 250                 255
gtg ggg gcc gaa gcc tgg gcg agg agg gtc ccc caa ccc gcg gcg cct 816
Val Gly Ala Glu Ala Trp Ala Arg Arg Val Pro Gln Pro Ala Ala Pro
            260                 265                 270
ccc cgc cga ccc ccc cag tca gca tca agt ctg agc gcc tct ctc cgg 864
Pro Arg Arg Pro Pro Gln Ser Ala Ser Ser Leu Ser Ala Ser Leu Arg
        275                 280                 285
ccc ccg ggg gcc ccg gcg act ttc cta aga cct tcc cct atc cct tgc 912
Pro Pro Gly Ala Pro Ala Thr Phe Leu Arg Pro Ser Pro Ile Pro Cys
    290                 295                 300
tcc tcg ccc ggt ccc tgg cag agc ctc tgc ggc ctg ggc ccg ccc tgc 960
Ser Ser Pro Gly Pro Trp Gln Ser Leu Cys Gly Leu Gly Pro Pro Cys
305                 310                 315                 320
gcc ggc tgc cct tgg ccg acg gct ggc ccc ggt agg aga tca ccc ggt 1008
Ala Gly Cys Pro Trp Pro Thr Ala Gly Pro Gly Arg Arg Ser Pro Gly
                325                 330                 335
ggc acc agc cca gag cgc tcg cca ggt acg gcg agg gca cgt ggg gac 1056
Gly Thr Ser Pro Glu Arg Ser Pro Gly Thr Ala Arg Ala Arg Gly Asp
            340                 345                 350
ccc acc tcc ctc cag gcc tct tca gag aag acc caa cag 1095
Pro Thr Ser Leu Gln Ala Ser Ser Glu Lys Thr Gln Gln
        355                 360                 365

<210> 17
<211> 465
<212> PRT
<213> Homo sapiens
<400> 17
Met Gly Arg Lys Lys Ile Gln Ile Thr Arg Ile Met Asp Glu Arg Asn
1               5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Thr Leu Arg Lys Lys Gly Leu Asn Gly Cys
                85                  90                  95
Asp Ser Pro Asp Pro Asp Ala Asp Asp Ser Val Gly His Ser Pro Glu
            100                 105                 110
Ser Glu Asp Lys Tyr Arg Lys Ile Asn Glu Asp Ile Asp Leu Met Ile
        115                 120                 125
Ser Arg Gln Arg Leu Cys Ala Val Pro Pro Pro Asn Phe Glu Met Pro
    130                 135                 140
Val Ser Ile Pro Val Ser Ser His Asn Ser Leu Val Tyr Ser Asn Pro
145                 150                 155                 160
Val Ser Ser Leu Gly Asn Pro Asn Leu Leu Pro Leu Ala His Pro Ser
                165                 170                 175
Leu Gln Arg Asn Ser Met Ser Pro Gly Val Thr His Arg Pro Pro Ser
            180                 185                 190
Ala Gly Asn Thr Gly Gly Leu Met Gly Gly Asp Leu Thr Ser Gly Ala
        195                 200                 205
Gly Thr Ser Ala Gly Asn Gly Tyr Gly Asn Pro Arg Asn Ser Pro Gly
    210                 215                 220
Leu Leu Val Ser Pro Gly Asn Leu Asn Lys Asn Met Gln Ala Lys Ser
225                 230                 235                 240
Pro Pro Pro Met Asn Leu Gly Met Asn Asn Arg Lys Pro Asp Leu Arg

                245                 250                 255
Val Leu Ile Pro Pro Gly Ser Lys Asn Thr Met Pro Ser Val Asn Gln
            260                 265                 270
Arg Ile Asn Asn Ser Gln Ser Ala Gln Ser Leu Ala Thr Pro Val Val
        275                 280                 285
Ser Val Ala Thr Pro Thr Leu Pro Gly Gln Gly Met Gly Gly Tyr Pro
    290                 295                 300
Ser Ala Ile Ser Thr Thr Tyr Gly Thr Glu Tyr Ser Leu Ser Ser Ala
305                 310                 315                 320
Asp Leu Ser Ser Leu Ser Gly Phe Asn Thr Ala Ser Ala Leu His Leu
                325                 330                 335
Gly Ser Val Thr Gly Trp Gln Gln Gln His Leu His Asn Met Pro Pro
            340                 345                 350
Ser Ala Leu Ser Gln Leu Gly Ala Cys Thr Ser Thr His Leu Ser Gln
        355                 360                 365
Ser Ser Asn Leu Ser Leu Pro Ser Thr Gln Ser Leu Asn Ile Lys Ser
    370                 375                 380
Glu Pro Val Ser Pro Pro Arg Asp Arg Thr Thr Thr Pro Ser Arg Tyr
385                 390                 395                 400
Pro Gln His Thr Arg His Glu Ala Gly Arg Ser Pro Val Asp Ser Leu
                405                 410                 415
Ser Ser Cys Ser Ser Ser Tyr Asp Gly Ser Asp Arg Glu Asp His Arg
            420                 425                 430
Asn Glu Phe His Ser Pro Ile Gly Leu Thr Arg Pro Ser Pro Asp Glu
        435                 440                 445
Arg Glu Ser Pro Ser Val Lys Arg Met Arg Leu Ser Glu Gly Trp Ala
    450                 455                 460
Thr
<210> 18
<211> 1395
<212> DNA
<213> Homo sapiens
<220>
<221> CDS
<223> (1)..(1398)
<400> 18

atg ggg aga aaa aag att cag att acg agg att atg gat gaa cgt aac 48
Met Gly Arg Lys Lys Ile Gln Ile Thr Arg Ile Met Asp Glu Arg Asn
1               5                   10                  15
aga cag gtg aca ttt aca aag agg aaa ttt ggg ttg atg aag aag gct 96
Arg Gln Val Thr Phe Thr Lys Arg Lys Phe Gly Leu Met Lys Lys Ala
            20                  25                  30
tat gag ctg agc gtg ctg tgt gac tgt gag att gcg ctg atc atc ttc 144
Tyr Glu Leu Ser Val Leu Cys Asp Cys Glu Ile Ala Leu Ile Ile Phe
        35                  40                  45
aac agc acc aac aag ctg ttc cag tat gcc agc acc gac atg gac aaa 192
Asn Ser Thr Asn Lys Leu Phe Gln Tyr Ala Ser Thr Asp Met Asp Lys
    50                  55                  60
gtg ctt ctc aag tac acg gag tac aac gag ccg cat gag agc cgg aca 240
Val Leu Leu Lys Tyr Thr Glu Tyr Asn Glu Pro His Glu Ser Arg Thr
65                  70                  75                  80
aac tca gac atc gtg gag acg ttg aga aag aag ggc ctt aat ggc tgt 288
Asn Ser Asp Ile Val Glu Thr Leu Arg Lys Lys Gly Leu Asn Gly Cys
                85                  90                  95
gac agc cca gac ccc gat gcg gac gat tcc gta ggt cac agc cct gag 336
Asp Ser Pro Asp Pro Asp Ala Asp Asp Ser Val Gly His Ser Pro Glu
            100                 105                 110
tct gag gac aag tac agg aaa att aac gaa gat att gat cta atg atc 384
Ser Glu Asp Lys Tyr Arg Lys Ile Asn Glu Asp Ile Asp Leu Met Ile
        115                 120                 125
agc agg caa aga ttg tgt gct gtt cca cct ccc aac ttc gag atg cca 432
Ser Arg Gln Arg Leu Cys Ala Val Pro Pro Pro Asn Phe Glu Met Pro
    130                 135                 140
gtc tcc atc cca gtg tcc agc cac aac agt ttg gtg tac agc aac cct 480
Val Ser Ile Pro Val Ser Ser His Asn Ser Leu Val Tyr Ser Asn Pro
145                 150                 155                 160
gtc agc tca ctg gga aac ccc aac cta ttg cca ctg gct cac cct tct 528
Val Ser Ser Leu Gly Asn Pro Asn Leu Leu Pro Leu Ala His Pro Ser
                165                 170                 175
ctg cag agg aat agt atg tct cct ggt gta aca cat cga cct cca agt 576
Leu Gln Arg Asn Ser Met Ser Pro Gly Val Thr His Arg Pro Pro Ser
            180                 185                 190

gca ggt aac aca ggt ggt ctg atg ggt gga gac ctc acg tct ggt gca 624
Ala Gly Asn Thr Gly Gly Leu Met Gly Gly Asp Leu Thr Ser Gly Ala
        195                 200                 205
ggc acc agt gca ggg aac ggg tat ggc aat ccc cga aac tca cca ggt 672
Gly Thr Ser Ala Gly Asn Gly Tyr Gly Asn Pro Arg Asn Ser Pro Gly
    210                 215                 220
ctg ctg gtc tca cct ggt aac ttg aac aag aat atg caa gca aaa tct 720
Leu Leu Val Ser Pro Gly Asn Leu Asn Lys Asn Met Gln Ala Lys Ser
225                 230                 235                 240
cct ccc cca atg aat tta gga atg aat aac cgt aaa cca gat ctc cga 768
Pro Pro Pro Met Asn Leu Gly Met Asn Asn Arg Lys Pro Asp Leu Arg
                245                 250                 255
gtt ctt att cca cca ggc agc aag aat acg atg cca tca gtg aat caa 816
Val Leu Ile Pro Pro Gly Ser Lys Asn Thr Met Pro Ser Val Asn Gln
            260                 265                 270
agg ata aat aac tcc cag tcg gct cag tca ttg gct acc cca gtg gtt 864
Arg Ile Asn Asn Ser Gln Ser Ala Gln Ser Leu Ala Thr Pro Val Val
        275                 280                 285
tcc gta gca act cct act tta cca gga caa gga atg gga gga tat cca 912
Ser Val Ala Thr Pro Thr Leu Pro Gly Gln Gly Met Gly Gly Tyr Pro
    290                 295                 300
tca gcc att tca aca aca tat ggt acc gag tac tct ctg agt agt gca 960
Ser Ala Ile Ser Thr Thr Tyr Gly Thr Glu Tyr Ser Leu Ser Ser Ala
305                 310                 315                 320
gac ctg tca tct ctg tct ggg ttt aac acc gcc agc gct ctt cac ctt 1008
Asp Leu Ser Ser Leu Ser Gly Phe Asn Thr Ala Ser Ala Leu His Leu
                325                 330                 335
ggt tca gta act ggc tgg caa cag caa cac cta cat aac atg cca cca 1056
Gly Ser Val Thr Gly Trp Gln Gln Gln His Leu His Asn Met Pro Pro
            340                 345                 350
tct gcc ctc agt cag ttg gga gct tgc act agc act cat tta tct cag 1104
Ser Ala Leu Ser Gln Leu Gly Ala Cys Thr Ser Thr His Leu Ser Gln
        355                 360                 365
agt tca aat ctc tcc ctg cct tct act caa agc ctc aac atc aag tca 1152
Ser Ser Asn Leu Ser Leu Pro Ser Thr Gln Ser Leu Asn Ile Lys Ser
    370                 375                 380

gaa cct gtt tct cct cct aga gac cgt acc acc acc cct tcg aga tac    1200
Glu Pro Val Ser Pro Pro Arg Asp Arg Thr Thr Thr Pro Ser Arg Tyr
385                 390                 395                 400
cca caa cac acg cgc cac gag gcg ggg aga tct cct gtt gac agc ttg    1248
Pro Gln His Thr Arg His Glu Ala Gly Arg Ser Pro Val Asp Ser Leu
                405                 410                 415
agc agc tgt agc agt tcg tac gac ggg agc gac cga gag gat cac cgg    1296
Ser Ser Cys Ser Ser Ser Tyr Asp Gly Ser Asp Arg Glu Asp His Arg
            420                 425                 430
aac gaa ttc cac tcc ccc att gga ctc acc aga cct tcg ccg gac gaa    1344
Asn Glu Phe His Ser Pro Ile Gly Leu Thr Arg Pro Ser Pro Asp Glu
        435                 440                 445
agg gaa agt ccc tca gtc aag cgc atg cga ctt tct gaa gga tgg gca    1392
Arg Glu Ser Pro Ser Val Lys Arg Met Arg Leu Ser Glu Gly Trp Ala
    450                 455                 460
aca                                                                1395
Thr
465
<210> 19
<211> 521
<212> PRT
<213> Homo sapiens
<400> 19
Met Gly Arg Lys Lys Ile Gln Ile Gln Arg Ile Thr Asp Glu Arg Asn
 1               5                  10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn His Ser Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ala Asp Ile Ile Glu Thr Leu Arg Lys Lys Gly Phe Asn Gly Cys
                85                  90                  95
Asp Ser Pro Glu Pro Asp Gly Glu Asp Ser Leu Glu Gln Ser Pro Leu

            100                 105                 110
Leu Glu Asp Lys Tyr Arg Arg Ala Ser Glu Glu Leu Asp Gly Leu Phe
        115                 120                 125
Arg Arg Tyr Gly Ser Thr Val Pro Ala Pro Asn Phe Ala Met Pro Val
    130                 135                 140
Thr Val Pro Val Ser Asn Gln Ser Ser Leu Gln Phe Ser Asn Pro Ser
145                 150                 155                 160
Gly Ser Leu Val Thr Pro Ser Leu Val Thr Ser Ser Leu Thr Asp Pro
                165                 170                 175
Arg Leu Leu Ser Pro Gln Gln Pro Ala Leu Gln Arg Asn Ser Val Ser
            180                 185                 190
Pro Gly Leu Pro Gln Arg Pro Ala Ser Ala Gly Ala Met Leu Gly Gly
        195                 200                 205
Asp Leu Asn Ser Ala Asn Gly Ala Cys Pro Ser Pro Val Gly Asn Gly
    210                 215                 220
Tyr Val Ser Ala Arg Ala Ser Pro Gly Leu Leu Pro Val Ala Asn Gly
225                 230                 235                 240
Asn Ser Leu Asn Lys Val Ile Pro Ala Lys Ser Pro Pro Pro Pro Thr
                245                 250                 255
His Ser Thr Gln Leu Gly Ala Pro Ser Arg Lys Pro Asp Leu Arg Val
            260                 265                 270
Ile Thr Ser Gln Ala Gly Lys Gly Leu Met His His Leu Thr Glu Asp
        275                 280                 285
His Leu Asp Leu Asn Asn Ala Gln Arg Leu Gly Val Ser Gln Ser Thr
    290                 295                 300
His Ser Leu Thr Thr Pro Val Val Ser Val Ala Thr Pro Ser Leu Leu
305                 310                 315                 320
Ser Gln Gly Leu Pro Phe Ser Ser Met Pro Thr Ala Tyr Asn Thr Asp
                325                 330                 335
Tyr Gln Leu Thr Ser Ala Glu Leu Ser Ser Leu Pro Ala Phe Ser Ser
            340                 345                 350
Pro Gly Gly Leu Ser Leu Gly Asn Val Thr Ala Trp Gln Gln Pro Gln
        355                 360                 365
Gln Pro Gln Gln Pro Gln Gln Pro Gln Pro Pro Gln Gln Gln Pro Pro
    370                 375                 380
Gln Pro Gln Gln Pro Gln Pro Gln Gln Pro Gln Gln Pro Gln Gln Pro

385                 390                 395                 400
Pro Gln Gln Gln Ser His Leu Val Pro Val Ser Leu Ser Asn Leu Ile
                405                 410                 415
Pro Gly Ser Pro Leu Pro His Val Gly Ala Ala Leu Thr Val Thr Thr
            420                 425                 430
His Pro His Ile Ser Ile Lys Ser Glu Pro Val Ser Pro Ser Arg Glu
        435                 440                 445
Arg Ser Pro Ala Pro Pro Pro Pro Ala Val Phe Pro Ala Ala Arg Pro
    450                 455                 460
Glu Pro Gly Asp Gly Leu Ser Ser Pro Ala Gly Gly Ser Tyr Glu Thr
465                 470                 475                 480
Gly Asp Arg Asp Asp Gly Arg Gly Asp Phe Gly Pro Thr Leu Gly Leu
                485                 490                 495
Leu Arg Pro Ala Pro Glu Pro Glu Ala Glu Gly Ser Ala Val Lys Arg
            500                 505                 510
Met Arg Leu Asp Thr Trp Thr Leu Lys
        515                 520
<210> 20
<211> 1563
<212> DNA
<213> Homo sapiens
<220>
<221> CDS
<223> (1)..(1566)
<400> 20
atg ggg agg aaa aag att cag atc cag cga atc acc gac gag cgg aac     48
Met Gly Arg Lys Lys Ile Gln Ile Gln Arg Ile Thr Asp Glu Arg Asn
 1               5                  10                  15
cga cag gtg act ttc acc aag cgg aag ttt ggc ctg atg aag aag gcg     96
Arg Gln Val Thr Phe Thr Lys Arg Lys Phe Gly Leu Met Lys Lys Ala
            20                  25                  30
tat gag ctg agc gtg cta tgt gac tgc gag atc gca ctc atc atc ttc     144
Tyr Glu Leu Ser Val Leu Cys Asp Cys Glu Ile Ala Leu Ile Ile Phe
        35                  40                  45
aac cac tcc aac aag ctg ttc cag tac gcc agc acc gac atg gac aag     192
Asn His Ser Asn Lys Leu Phe Gln Tyr Ala Ser Thr Asp Met Asp Lys

50                  55                  60
gtg ctg ctc aag tac acg gag tac aat gag cca cac gag agc cgc acc    240
Val Leu Leu Lys Tyr Thr Glu Tyr Asn Glu Pro His Glu Ser Arg Thr
65                  70                  75                  80
aac gcc gac atc atc gag acc ctg agg aag aag ggc ttc aat ggc tgc    288
Asn Ala Asp Ile Ile Glu Thr Leu Arg Lys Lys Gly Phe Asn Gly Cys
                85                  90                  95
gac agc ccc gag ccc gac ggg gag gac tcg ctg gaa cag agc ccc ctg    336
Asp Ser Pro Glu Pro Asp Gly Glu Asp Ser Leu Glu Gln Ser Pro Leu
            100                 105                 110
ctg gag gac aag tac cga cgc gcc agc gag gag ctc gac ggg ctc ttc    384
Leu Glu Asp Lys Tyr Arg Arg Ala Ser Glu Glu Leu Asp Gly Leu Phe
        115                 120                 125
cgg cgc tat ggg tca act gtc ccg gcc ccc aac ttt gcc atg cct gtc    432
Arg Arg Tyr Gly Ser Thr Val Pro Ala Pro Asn Phe Ala Met Pro Val
    130                 135                 140
acg gtg ccc gtg tcc aat cag agc tca ctg cag ttc agc aat ccc agc    480
Thr Val Pro Val Ser Asn Gln Ser Ser Leu Gln Phe Ser Asn Pro Ser
145                 150                 155                 160
ggc tcc ctg gtc acc cct tcc ctg gtg aca tca tcc ctc acg gac ccg    528
Gly Ser Leu Val Thr Pro Ser Leu Val Thr Ser Ser Leu Thr Asp Pro
                165                 170                 175
cgg ctc ctg tcc ccc cag cag cca gca cta cag agg aac agt gtg tct    576
Arg Leu Leu Ser Pro Gln Gln Pro Ala Leu Gln Arg Asn Ser Val Ser
            180                 185                 190
cct ggc ctg ccc cag cgg cca gct agt gcg ggg gcc atg ctg ggg ggt    624
Pro Gly Leu Pro Gln Arg Pro Ala Ser Ala Gly Ala Met Leu Gly Gly
        195                 200                 205
gac ctg aac agt gct aac gga gcc tgc ccc agc cct gtt ggg aat ggc    672
Asp Leu Asn Ser Ala Asn Gly Ala Cys Pro Ser Pro Val Gly Asn Gly
    210                 215                 220
tac gtc agt gct cgg gct tcc cct ggc ctc ctc cct gtg gcc aat ggc    720
Tyr Val Ser Ala Arg Ala Ser Pro Gly Leu Leu Pro Val Ala Asn Gly
225                 230                 235                 240
aac agc cta aac aag gtc atc cct gcc aag tct ccg ccc cca cct acc    768
Asn Ser Leu Asn Lys Val Ile Pro Ala Lys Ser Pro Pro Pro Pro Thr

                245                 250                 255
cac agc acc cag ctt gga gcc ccc agc cgc aag ccc gac ctg cga gtc 816
His Ser Thr Gln Leu Gly Ala Pro Ser Arg Lys Pro Asp Leu Arg Val
            260                 265                 270
atc act tcc cag gca gga aag ggg tta atg cat cac ttg act gag gac 864
Ile Thr Ser Gln Ala Gly Lys Gly Leu Met His His Leu Thr Glu Asp
        275                 280                 285
cat tta gat ctg aac aat gcc cag cgc ctt ggg gtc tcc cag tct act 912
His Leu Asp Leu Asn Asn Ala Gln Arg Leu Gly Val Ser Gln Ser Thr
    290                 295                 300
cat tcg ctc acc acc cca gtg gtt tct gtg gca acg ccg agt tta ctc 960
His Ser Leu Thr Thr Pro Val Val Ser Val Ala Thr Pro Ser Leu Leu
305                 310                 315                 320
agc cag ggc ctc ccc ttc tct tcc atg ccc act gcc tac aac aca gat 1008
Ser Gln Gly Leu Pro Phe Ser Ser Met Pro Thr Ala Tyr Asn Thr Asp
                325                 330                 335
tac cag ttg acc agt gca gag ctc tcc tcc tta cca gcc ttt agt tca 1056
Tyr Gln Leu Thr Ser Ala Glu Leu Ser Ser Leu Pro Ala Phe Ser Ser
            340                 345                 350
cct ggg ggg ctg tcg cta ggc aat gtc act gcc tgg caa cag cca cag 1104
Pro Gly Gly Leu Ser Leu Gly Asn Val Thr Ala Trp Gln Gln Pro Gln
        355                 360                 365
cag ccc cag cag ccg cag cag cca cag cct cca cag cag cag cca ccg 1152
Gln Pro Gln Gln Pro Gln Gln Pro Gln Pro Pro Gln Gln Gln Pro Pro
    370                 375                 380
cag cca cag cag cca cag cca cag cag cct cag cag ccg caa cag cca 1200
Gln Pro Gln Gln Pro Gln Pro Gln Gln Pro Gln Gln Pro Gln Gln Pro
385                 390                 395                 400
cct cag caa cag tcc cac ctg gtc cct gta tct ctc agc aac ctc atc 1248
Pro Gln Gln Gln Ser His Leu Val Pro Val Ser Leu Ser Asn Leu Ile
                405                 410                 415
ccg ggc agc ccc ctg ccc cac gtg ggt gct gcc ctc aca gtc acc acc 1296
Pro Gly Ser Pro Leu Pro His Val Gly Ala Ala Leu Thr Val Thr Thr
            420                 425                 430
cac ccc cac atc agc atc aag tca gaa ccg gtg tcc cca agc cgt gag 1344
His Pro His Ile Ser Ile Lys Ser Glu Pro Val Ser Pro Ser Arg Glu

435                 440                 445
cgc agc cct gcg cct ccc cct cca gct gtg ttc cca gct gcc cgc cct    1392
Arg Ser Pro Ala Pro Pro Pro Pro Ala Val Phe Pro Ala Ala Arg Pro
    450                 455                 460
gag cct ggc gat ggt ctc agc agc cca gcc ggg gga tcc tat gag acg    1440
Glu Pro Gly Asp Gly Leu Ser Ser Pro Ala Gly Gly Ser Tyr Glu Thr
465                 470                 475                 480
gga gac cgg gat gac gga cgg ggg gac ttc ggg ccc aca ctg ggc ctg    1488
Gly Asp Arg Asp Asp Gly Arg Gly Asp Phe Gly Pro Thr Leu Gly Leu
                485                 490                 495
ctg cgc cca gcc cca gag cct gag gct gag ggc tca gct gtg aag agg    1536
Leu Arg Pro Ala Pro Glu Pro Glu Ala Glu Gly Ser Ala Val Lys Arg
            500                 505                 510
atg cgg ctt gat acc tgg aca tta aag                                1563
Met Arg Leu Asp Thr Trp Thr Leu Lys
        515                 520
<210> 21
<211> 217
<212> PRT
<213> Rattus norvegicus
<400> 21
Met Ser Leu Val Gly Gly Phe Pro His His Pro Val Val His His Glu
  1               5                  10                  15
Gly Tyr Pro Phe Ala Ala Ala Ala Ala Ala Ala Ala Ala Ala Ala Ala
             20                  25                  30
Ser Arg Cys Ser His Glu Glu Asn Pro Tyr Phe His Gly Trp Leu Ile
         35                  40                  45
Gly His Pro Glu Met Ser Pro Pro Asp Tyr Ser Met Ala Leu Ser Tyr
     50                  55                  60
Ser Pro Glu Tyr Ala Ser Gly Ala Ala Gly Leu Asp His Ser His Tyr
 65                  70                  75                  80
Gly Gly Val Pro Pro Gly Ala Gly Pro Pro Gly Leu Gly Gly Pro Arg
                 85                  90                  95
Pro Val Lys Arg Arg Gly Thr Ala Asn Arg Lys Glu Arg Arg Arg Thr
            100                 105                 110
Gln Ser Ile Asn Ser Ala Phe Ala Glu Leu Arg Glu Cys Ile Pro Asn

        115                 120                 125
Val Pro Ala Asp Thr Lys Leu Ser Lys Ile Lys Thr Leu Arg Leu Ala
    130                 135                 140
Thr Ser Tyr Ile Ala Tyr Leu Met Asp Leu Leu Ala Lys Asp Asp Gln
145                 150                 155                 160
Asn Gly Glu Ala Glu Ala Phe Lys Ala Glu Ile Lys Lys Thr Asp Val
                165                 170                 175
Lys Glu Glu Lys Arg Lys Lys Glu Leu Asn Glu Ile Leu Lys Ser Thr
            180                 185                 190
Val Ser Ser Asn Asp Lys Lys Thr Lys Gly Arg Thr Gly Trp Pro Gln
        195                 200                 205
His Val Trp Ala Leu Glu Leu Lys Gln
    210                 215
<210> 22
<211> 651
<212> DNA
<213> Rattus norvegicus
<220>
<221> CDS
<223> (1)..(654)
<400> 22
   atg agt ctg gtg ggg ggc ttt ccc cac cac ccc gtg gtg cac cat gag 48
   Met Ser Leu Val Gly Gly Phe Pro His His Pro Val Val His His Glu
   1 5 10 15
   ggc tac ccg ttc gcc gca gcc gca gcc gcc gct gct gct gcc gcc gcc 96
   Gly Tyr Pro Phe Ala Ala Ala Ala Ala Ala Ala Ala Ala Ala Ala Ala
   20 25 30
   agc cgc tgc agt cac gag gag aac ccc tat ttc cac ggc tgg ctt att 144
   Ser Arg Cys Ser His Glu Glu Asn Pro Tyr Phe His Gly Trp Leu Ile
   35 40 45
   ggc cac ccg gag atg tcg ccc ccc gac tac agc atg gcc ctg tcc tac 192
   Gly His Pro Glu Met Ser Pro Pro Asp Tyr Ser Met Ala Leu Ser Tyr
   50 55 60
   agt ccc gag tac gcc agc ggt gcc gcg ggc ctg gac cac tcc cat tat 240
   Ser Pro Glu Tyr Ala Ser Gly Ala Ala Gly Leu Asp His Ser His Tyr
   65 70 75 80
   ggg gga gtg ccg ccc ggt gcc ggg cct ccc ggc ctg ggg ggg ccg cgc 288
   Gly Gly Val Pro Pro Gly Ala Gly Pro Pro Gly Leu Gly Gly Pro Arg
   85 90 95
   ccg gtg aag cgt cgg ggc acc gcc aac cgc aag gag cgg cgc agg act 336
   Pro Val Lys Arg Arg Gly Thr Ala Asn Arg Lys Glu Arg Arg Arg Thr
   100 105 110
   cag agc atc aac agc gcc ttc gcc gag ctg cgc gag tgc atc ccc aac 384
   Gln Ser Ile Asn Ser Ala Phe Ala Glu Leu Arg Glu Cys Ile Pro Asn
   115 120 125
   gtg ccc gcc gac acc aaa ctc tcc aaa atc aag act ctg cgc ctg gcc 432
   Val Pro Ala Asp Thr Lys Leu Ser Lys Ile Lys Thr Leu Arg Leu Ala
   130 135 140
   acc agc tac atc gcc tac ctc atg gat ctg ctg gcc aag gac gac cag 480
   Thr Ser Tyr Ile Ala Tyr Leu Met Asp Leu Leu Ala Lys Asp Asp Gln
   145 150 155 160
   aac gga gag gcg gag gcc ttc aag gcg gag atc aag aag acc gac gtg 528
   Asn Gly Glu Ala Glu Ala Phe Lys Ala Glu Ile Lys Lys Thr Asp Val
   165 170 175
   aaa gag gag aag agg aag aaa gag ctg aat gaa atc ttg aaa agt aca 576
   Lys Glu Glu Lys Arg Lys Lys Glu Leu Asn Glu Ile Leu Lys Ser Thr
   180 185 190
   gtg agc agc aac gac aag aaa acc aaa ggc cgg aca ggc tgg cca cag 624
   Val Ser Ser Asn Asp Lys Lys Thr Lys Gly Arg Thr Gly Trp Pro Gln
   195 200 205
   cac gtc tgg gcc ctg gag ctc aag cag 651
   His Val Trp Ala Leu Glu Leu Lys Gln
   210 215
<210> 23
<211> 215
<212> PRT
<213> Homo sapiens
<400> 23
Met Asn Leu Val Gly Ser Tyr Ala His His His His His His His Pro
1               5                   10                  15
His Pro Ala His Pro Met Leu His Glu Pro Phe Leu Phe Gly Pro Ala
            20                  25                  30

Ser Arg Cys His Gln Glu Arg Pro Tyr Phe Gln Ser Trp Leu Leu Ser
        35                  40                  45
Pro Ala Asp Ala Ala Pro Asp Phe Pro Ala Gly Gly Pro Pro Pro Ala
    50                  55                  60
Ala Ala Ala Ala Ala Thr Ala Tyr Gly Pro Asp Ala Arg Pro Gly Gln
65                  70                  75                  80
Ser Pro Gly Arg Leu Glu Ala Leu Gly Gly Arg Leu Gly Arg Arg Lys
                85                  90                  95
Gly Ser Gly Pro Lys Lys Glu Arg Arg Arg Thr Glu Ser Ile Asn Ser
            100                 105                 110
Ala Phe Ala Glu Leu Arg Glu Cys Ile Pro Asn Val Pro Ala Asp Thr
        115                 120                 125
Lys Leu Ser Lys Ile Lys Thr Leu Arg Leu Ala Thr Ser Tyr Ile Ala
    130                 135                 140
Tyr Leu Met Asp Val Leu Ala Lys Asp Ala Gln Ser Gly Asp Pro Glu
145                 150                 155                 160
Ala Phe Lys Ala Glu Leu Lys Lys Ala Asp Gly Gly Arg Glu Ser Lys
                165                 170                 175
Arg Lys Arg Glu Leu Gln Gln His Glu Gly Phe Pro Pro Ala Leu Gly
            180                 185                 190
Pro Val Glu Lys Arg Ile Lys Gly Arg Thr Gly Trp Pro Gln Gln Val
        195                 200                 205
Trp Ala Leu Glu Leu Asn Gln
210
<210> 24
<211> 645
<212> DNA
<213> Homo sapiens
<220>
<221> CDS
<223> (1)..(648)
<400> 24
atg aac ctc gtg ggc agc tac gca cac cat cac cac cat cac cac ccg 48
Met Asn Leu Val Gly Ser Tyr Ala His His His His His His His Pro
1               5                   10                  15
cac cct gcg cac ccc atg ctc cac gaa ccc ttc ctc ttc ggt ccg gcc 96

His Pro Ala His Pro Met Leu His Glu Pro Phe Leu Phe Gly Pro Ala
            20                  25                  30
tcg cgc tgt cat cag gaa agg ccc tac ttc cag agc tgg ctg ctg agc 144
Ser Arg Cys His Gln Glu Arg Pro Tyr Phe Gln Ser Trp Leu Leu Ser
        35                  40                  45
ccg gct gac gct gcc ccg gac ttc cct gcg ggc ggg ccg ccg ccc gcg 192
Pro Ala Asp Ala Ala Pro Asp Phe Pro Ala Gly Gly Pro Pro Pro Ala
    50                  55                  60
gcc gct gca gcc gcc acc gcc tat ggt cct gac gcc agg cct ggg cag 240
Ala Ala Ala Ala Ala Thr Ala Tyr Gly Pro Asp Ala Arg Pro Gly Gln
65                  70                  75                  80
agc ccc ggg cgg ctg gag gcg ctt ggc ggc cgt ctt ggc cgg cgg aaa 288
Ser Pro Gly Arg Leu Glu Ala Leu Gly Gly Arg Leu Gly Arg Arg Lys
                85                  90                  95
ggc tca gga ccc aag aag gag cgg aga cgc act gag agc att aac agc 336
Gly Ser Gly Pro Lys Lys Glu Arg Arg Arg Thr Glu Ser Ile Asn Ser
            100                 105                 110
gca ttc gcg gag ttg cgc gag tgc atc ccc aac gtg ccg gcc gac acc 384
Ala Phe Ala Glu Leu Arg Glu Cys Ile Pro Asn Val Pro Ala Asp Thr
        115                 120                 125
aag ctc tcc aag atc aag act ctg cgc cta gcc acc agc tac atc gcc 432
Lys Leu Ser Lys Ile Lys Thr Leu Arg Leu Ala Thr Ser Tyr Ile Ala
    130                 135                 140
tac ctg atg gac gtg ctg gcc aag gat gca cag tct ggc gat ccc gag 480
Tyr Leu Met Asp Val Leu Ala Lys Asp Ala Gln Ser Gly Asp Pro Glu
145                 150                 155                 160
gcc ttc aag gct gaa ctc aag aag gcg gat ggc ggc cgt gag agc aag 528
Ala Phe Lys Ala Glu Leu Lys Lys Ala Asp Gly Gly Arg Glu Ser Lys
                165                 170                 175
cgg aaa agg gag ctg cag cag cac gaa ggt ttt cct cct gcc ctg ggc 576
Arg Lys Arg Glu Leu Gln Gln His Glu Gly Phe Pro Pro Ala Leu Gly
            180                 185                 190
cca gtc gag aag agg att aaa gga cgc acc ggc tgg ccg cag caa gtc 624
Pro Val Glu Lys Arg Ile Lys Gly Arg Thr Gly Trp Pro Gln Gln Val
        195                 200                 205
tgg gcg ctg gag tta aac cag 645

Trp Ala Leu Glu Leu Asn Gln
    210                 215
<210> 25
<211> 411
<212> PRT
<213> Homo sapiens
<400> 25
Met Glu Arg Met Ser Asp Ser Ala Asp Lys Pro Ile Asp Asn Asp Ala
  1               5                  10                  15
Glu Gly Val Trp Ser Pro Asp Ile Glu Gln Ser Phe Gln Glu Ala Leu
             20                  25                  30
Ala Ile Tyr Pro Pro Cys Gly Arg Arg Lys Ile Ile Leu Ser Asp Glu
         35                  40                  45
Gly Lys Met Tyr Gly Arg Asn Glu Leu Ile Ala Arg Tyr Ile Lys Leu
     50                  55                  60
Arg Thr Gly Lys Thr Arg Thr Arg Lys Gln Val Ser Ser His Ile Gln
 65                  70                  75                  80
Val Leu Ala Arg Arg Lys Ser Arg Asp Phe His Ser Lys Leu Lys Asp
                 85                  90                  95
Gln Thr Ala Lys Asp Lys Ala Leu Gln His Met Ala Ala Met Ser Ser
            100                 105                 110
Ala Gln Ile Val Ser Ala Thr Ala Ile His Asn Lys Leu Gly Leu Pro
        115                 120                 125
Gly Ile Pro Arg Pro Thr Phe Pro Gly Ala Pro Gly Phe Trp Pro Gly
    130                 135                 140
Met Ile Gln Thr Gly Gln Pro Gly Ser Ser Gln Asp Val Lys Pro Phe
145                 150                 155                 160
Val Gln Gln Ala Tyr Pro Ile Gln Pro Ala Val Thr Ala Pro Ile Pro
                165                 170                 175
Gly Phe Glu Pro Ala Ser Ala Pro Ala Pro Ser Val Pro Ala Trp Gln
            180                 185                 190
Gly Arg Ser Ile Gly Thr Thr Lys Leu Arg Leu Val Glu Phe Ser Ala
        195                 200                 205
Phe Leu Glu Gln Gln Arg Asp Pro Asp Ser Tyr Asn Lys His Leu Phe
    210                 215                 220
Val His Ile Gly His Ala Asn His Ser Tyr Ser Asp Pro Leu Leu Glu

225                 230                 235                 240
Ser Val Asp Ile Arg Gln Ile Tyr Asp Lys Phe Pro Glu Lys Lys Gly
                245                 250                 255
Gly Leu Lys Glu Leu Phe Gly Lys Gly Pro Gln Asn Ala Phe Phe Leu
            260                 265                 270
Val Lys Phe Trp Ala Asp Leu Asn Cys Asn Ile Gln Asp Asp Ala Gly
        275                 280                 285
Ala Phe Tyr Gly Val Thr Ser Gln Tyr Glu Ser Ser Glu Asn Met Thr
    290                 295                 300
Val Thr Cys Ser Thr Lys Val Cys Ser Phe Gly Lys Gln Val Val Glu
305                 310                 315                 320
Lys Val Glu Thr Glu Tyr Ala Arg Phe Glu Asn Gly Arg Phe Val Tyr
                325                 330                 335
Arg Ile Asn Arg Ser Pro Met Cys Glu Tyr Met Ile Asn Phe Ile His
            340                 345                 350
Lys Leu Lys His Leu Pro Glu Lys Tyr Met Met Asn Ser Val Leu Glu
        355                 360                 365
Asn Phe Thr Ile Leu Leu Val Val Thr Asn Arg Asp Thr Gln Glu Thr
    370                 375                 380
Leu Leu Cys Met Ala Cys Val Phe Glu Val Ser Asn Ser Glu His Gly
385                 390                 395                 400
Ala Gln His His Ile Tyr Arg Leu Val Lys Asp
                405                 410
<210> 26
<211> 1233
<212> DNA
<213> Homo sapiens
<220>
<221> CDS
<223> (1)..(1236)
<400> 26
atg gaa agg atg agt gac tct gca gat aag cca att gac aat gat gca      48
Met Glu Arg Met Ser Asp Ser Ala Asp Lys Pro Ile Asp Asn Asp Ala
  1               5                  10                  15
gaa ggg gtc tgg agc ccc gac atc gag caa agc ttt cag gag gcc ctg      96
Glu Gly Val Trp Ser Pro Asp Ile Glu Gln Ser Phe Gln Glu Ala Leu

            20                  25                  30
gct atc tat cca cca tgt ggg agg agg aaa atc atc tta tca gac gaa 144
Ala Ile Tyr Pro Pro Cys Gly Arg Arg Lys Ile Ile Leu Ser Asp Glu
        35                  40                  45
ggc aaa atg tat ggt agg aat gaa ttg ata gcc aga tac atc aaa ctc 192
Gly Lys Met Tyr Gly Arg Asn Glu Leu Ile Ala Arg Tyr Ile Lys Leu
    50                  55                  60
agg aca ggc aag acg agg acc aga aaa cag gtg tct agt cac att cag 240
Arg Thr Gly Lys Thr Arg Thr Arg Lys Gln Val Ser Ser His Ile Gln
65                  70                  75                  80
gtt ctt gcc aga agg aaa tct cgt gat ttt cat tcc aag cta aag gat 288
Val Leu Ala Arg Arg Lys Ser Arg Asp Phe His Ser Lys Leu Lys Asp
                85                  90                  95
cag act gca aag gat aag gcc ctg cag cac atg gcg gcc atg tcc tca 336
Gln Thr Ala Lys Asp Lys Ala Leu Gln His Met Ala Ala Met Ser Ser
            100                 105                 110
gcc cag atc gtc tcg gcc act gcc att cat aac aag ctg ggg ctg cct 384
Ala Gln Ile Val Ser Ala Thr Ala Ile His Asn Lys Leu Gly Leu Pro
        115                 120                 125
ggg att cca cgc ccg acc ttc cca ggg gcg ccg ggg ttc tgg ccg gga 432
Gly Ile Pro Arg Pro Thr Phe Pro Gly Ala Pro Gly Phe Trp Pro Gly
    130                 135                 140
atg att caa aca ggg cag cca gga tcc tca caa gac gtc aag cct ttt 480
Met Ile Gln Thr Gly Gln Pro Gly Ser Ser Gln Asp Val Lys Pro Phe
145                 150                 155                 160
gtg cag cag gcc tac ccc atc cag cca gcg gtc aca gcc ccc att cca 528
Val Gln Gln Ala Tyr Pro Ile Gln Pro Ala Val Thr Ala Pro Ile Pro
                165                 170                 175
ggg ttt gag cct gca tcg gcc cca gct ccc tca gtc cct gcc tgg caa 576
Gly Phe Glu Pro Ala Ser Ala Pro Ala Pro Ser Val Pro Ala Trp Gln
            180                 185                 190
ggt cgc tcc att ggc aca acc aag ctt cgc ctg gtg gaa ttt tca gct 624
Gly Arg Ser Ile Gly Thr Thr Lys Leu Arg Leu Val Glu Phe Ser Ala
        195                 200                 205
ttt ctc gag cag cag cga gac cca gac tcg tac aac aaa cac ctc ttc 672
Phe Leu Glu Gln Gln Arg Asp Pro Asp Ser Tyr Asn Lys His Leu Phe

210                 215                 220
gtg cac att ggg cat gcc aac cat tct tac agt gac cca ttg ctt gaa    720
Val His Ile Gly His Ala Asn His Ser Tyr Ser Asp Pro Leu Leu Glu
225                 230                 235                 240
tca gtg gac att cgt cag att tat gac aaa ttt cct gaa aag aaa ggt    768
Ser Val Asp Ile Arg Gln Ile Tyr Asp Lys Phe Pro Glu Lys Lys Gly
                245                 250                 255
ggc tta aag gaa ctg ttt gga aag ggc cct caa aat gcc ttc ttc ctc    816
Gly Leu Lys Glu Leu Phe Gly Lys Gly Pro Gln Asn Ala Phe Phe Leu
            260                 265                 270
gta aaa ttc tgg gct gat tta aac tgc aat att caa gat gat gct ggg    864
Val Lys Phe Trp Ala Asp Leu Asn Cys Asn Ile Gln Asp Asp Ala Gly
        275                 280                 285
gct ttt tat ggt gta acc agt cag tac gag agt tct gaa aat atg aca    912
Ala Phe Tyr Gly Val Thr Ser Gln Tyr Glu Ser Ser Glu Asn Met Thr
    290                 295                 300
gtc acc tgt tcc acc aaa gtt tgc tcc ttt ggg aag caa gta gta gaa    960
Val Thr Cys Ser Thr Lys Val Cys Ser Phe Gly Lys Gln Val Val Glu
305                 310                 315                 320
aaa gta gag acg gag tat gca agg ttt gag aat ggc cga ttt gta tac    1008
Lys Val Glu Thr Glu Tyr Ala Arg Phe Glu Asn Gly Arg Phe Val Tyr
                325                 330                 335
cga ata aac cgc tcc cca atg tgt gaa tat atg atc aac ttc atc cac    1056
Arg Ile Asn Arg Ser Pro Met Cys Glu Tyr Met Ile Asn Phe Ile His
            340                 345                 350
aag ctc aaa cac tta cca gag aaa tat atg atg aac agt gtt ttg gaa    1104
Lys Leu Lys His Leu Pro Glu Lys Tyr Met Met Asn Ser Val Leu Glu
        355                 360                 365
aac ttc aca att tta ttg gtg gta aca aac agg gat aca caa gaa act    1152
Asn Phe Thr Ile Leu Leu Val Val Thr Asn Arg Asp Thr Gln Glu Thr
    370                 375                 380
cta ctc tgc atg gcc tgt gtg ttt gaa gtt tca aat agt gaa cac gga    1200
Leu Leu Cys Met Ala Cys Val Phe Glu Val Ser Asn Ser Glu His Gly
385                 390                 395                 400
gca caa cat cat att tac agg ctt gta aag gac                        1233
Ala Gln His His Ile Tyr Arg Leu Val Lys Asp

                405                 410
<210> 27
<211> 427
<212> PRT
<213> Homo sapiens
<400> 27
Ile Thr Ser Asn Glu Trp Ser Ser Pro Thr Ser Pro Glu Gly Ser Thr
1               5                   10                  15
Ala Ser Gly Gly Ser Gln Ala Leu Asp Lys Pro Ile Asp Asn Asp Ala
            20                  25                  30
Glu Gly Val Trp Ser Pro Asp Ile Glu Gln Ser Phe Gln Glu Ala Leu
        35                  40                  45
Ala Ile Tyr Pro Pro Cys Gly Arg Arg Lys Ile Ile Leu Ser Asp Glu
    50                  55                  60
Gly Lys Met Tyr Gly Arg Asn Glu Leu Ile Ala Arg Tyr Ile Lys Leu
65                  70                  75                  80
Arg Thr Gly Lys Thr Arg Thr Arg Lys Gln Val Ser Ser His Ile Gln
                85                  90                  95
Val Leu Ala Arg Arg Lys Ala Arg Glu Ile Gln Ala Lys Leu Lys Asp
            100                 105                 110
Gln Ala Ala Lys Asp Lys Ala Leu Gln Ser Met Ala Ala Met Ser Ser
        115                 120                 125
Ala Gln Ile Ile Ser Ala Thr Ala Phe His Ser Ser Met Ala Leu Ala
    130                 135                 140
Arg Gly Pro Gly Arg Pro Ala Val Ser Gly Phe Trp Gln Gly Ala Leu
145                 150                 155                 160
Pro Gly Gln Ala Gly Thr Ser His Asp Val Lys Pro Phe Ser Gln Gln
                165                 170                 175
Thr Tyr Ala Val Gln Pro Pro Leu Pro Leu Pro Gly Phe Glu Ser Pro
            180                 185                 190
Ala Gly Pro Ala Pro Ser Pro Ser Ala Pro Pro Ala Pro Pro Trp Gln
        195                 200                 205
Gly Arg Ser Val Ala Ser Ser Lys Leu Trp Met Leu Glu Phe Ser Ala
    210                 215                 220
Phe Leu Glu Gln Gln Gln Asp Pro Asp Thr Tyr Asn Lys His Leu Phe
225                 230                 235                 240

Val His Ile Gly Gln Ser Ser Pro Ser Tyr Ser Asp Pro Tyr Leu Glu
                245                 250                 255
Ala Val Asp Ile Arg Gln Ile Tyr Asp Lys Phe Pro Glu Lys Lys Gly
            260                 265                 270
Gly Leu Lys Asp Leu Phe Glu Arg Gly Pro Ser Asn Ala Phe Phe Leu
        275                 280                 285
Val Lys Phe Trp Ala Asp Leu Asn Thr Asn Ile Glu Asp Glu Gly Ser
    290                 295                 300
Ser Phe Tyr Gly Val Ser Ser Gln Tyr Glu Ser Pro Glu Asn Met Ile
305                 310                 315                 320
Ile Thr Cys Ser Thr Lys Val Cys Ser Phe Gly Lys Gln Val Val Glu
                325                 330                 335
Lys Val Glu Thr Glu Tyr Ala Arg Tyr Glu Asn Gly His Tyr Ser Tyr
            340                 345                 350
Arg Ile His Arg Ser Pro Leu Cys Glu Tyr Met Ile Asn Phe Ile His
        355                 360                 365
Lys Leu Lys His Leu Pro Glu Lys Tyr Met Met Asn Ser Val Leu Glu
    370                 375                 380
Asn Phe Thr Ile Leu Gln Val Val Thr Asn Arg Asp Thr Gln Glu Thr
385                 390                 395                 400
Leu Leu Cys Ile Ala Tyr Val Phe Glu Val Ser Ala Ser Glu His Gly
                405                 410                 415
Ala Gln His His Ile Tyr Arg Leu Val Lys Glu
            420                 425
<210> 28
<211> 1281
<212> DNA
<213> Homo sapiens
<220>
<221> CDS
<223> (1)..(1284)
<400> 28
att acc tcc aac gag tgg agc tct ccc acc tcc cct gag ggg agc acc 48
Ile Thr Ser Asn Glu Trp Ser Ser Pro Thr Ser Pro Glu Gly Ser Thr
1               5                   10                  15
gcc tct ggg ggc agt cag gca ctg gac aag ccc atc gac aat gac gca 96

Ala Ser Gly Gly Ser Gln Ala Leu Asp Lys Pro Ile Asp Asn Asp Ala
            20                  25                  30
gag ggc gtg tgg agc ccg gat att gag cag agt ttc cag gag gcc ctc 144
Glu Gly Val Trp Ser Pro Asp Ile Glu Gln Ser Phe Gln Glu Ala Leu
        35                  40                  45
gcc atc tac ccg ccc tgt ggc agg cgc aaa atc atc ctg tcg gac gag 192
Ala Ile Tyr Pro Pro Cys Gly Arg Arg Lys Ile Ile Leu Ser Asp Glu
    50                  55                  60
ggc aag atg tat ggt cgg aac gag ctg att gcc cgc tac atc aag ctc 240
Gly Lys Met Tyr Gly Arg Asn Glu Leu Ile Ala Arg Tyr Ile Lys Leu
65                  70                  75                  80
cgg aca ggg aag acc cgc acc agg aag cag gtc tcc agc cac atc cag 288
Arg Thr Gly Lys Thr Arg Thr Arg Lys Gln Val Ser Ser His Ile Gln
                85                  90                  95
gtg ctg gct cgt cgc aaa gct cgc gag atc cag gcc aag cta aag gac 336
Val Leu Ala Arg Arg Lys Ala Arg Glu Ile Gln Ala Lys Leu Lys Asp
            100                 105                 110
cag gca gct aag gac aag gcc ctg cag agc atg gct gcc atg tcg tct 384
Gln Ala Ala Lys Asp Lys Ala Leu Gln Ser Met Ala Ala Met Ser Ser
        115                 120                 125
gca cag atc atc tcc gcc acg gcc ttc cac agt agc atg gcc ctc gcc 432
Ala Gln Ile Ile Ser Ala Thr Ala Phe His Ser Ser Met Ala Leu Ala
    130                 135                 140
cgg ggc ccc ggc cgc cca gca gtc tca ggg ttt tgg caa gga gct ttg 480
Arg Gly Pro Gly Arg Pro Ala Val Ser Gly Phe Trp Gln Gly Ala Leu
145                 150                 155                 160
cca ggc caa gcc gga acg tcc cat gat gtg aag cct ttc tct cag caa 528
Pro Gly Gln Ala Gly Thr Ser His Asp Val Lys Pro Phe Ser Gln Gln
                165                 170                 175
acc tat gct gtc cag cct ccg ctg cct ctg cca ggg ttt gag tct cct 576
Thr Tyr Ala Val Gln Pro Pro Leu Pro Leu Pro Gly Phe Glu Ser Pro
            180                 185                 190
gca ggg ccc gcc cca tcg ccc tct gcg ccc ccg gca ccc cca tgg cag 624
Ala Gly Pro Ala Pro Ser Pro Ser Ala Pro Pro Ala Pro Pro Trp Gln
        195                 200                 205
ggc cgc agc gtg gcc agc tcc aag ctc tgg atg ttg gag ttc tct gcc 672

Gly Arg Ser Val Ala Ser Ser Lys Leu Trp Met Leu Glu Phe Ser Ala
    210                 215                 220
ttc ctg gag cag cag cag gac ccg gac acg tac aac aag cac ctg ttc     720
Phe Leu Glu Gln Gln Gln Asp Pro Asp Thr Tyr Asn Lys His Leu Phe
225                 230                 235                 240
gtg cac att ggc cag tcc agc cca agc tac agc gac ccc tac ctc gaa     768
Val His Ile Gly Gln Ser Ser Pro Ser Tyr Ser Asp Pro Tyr Leu Glu
                245                 250                 255
gcc gtg gac atc cgc caa atc tat gac aaa ttc ccg gag aaa aag ggt     816
Ala Val Asp Ile Arg Gln Ile Tyr Asp Lys Phe Pro Glu Lys Lys Gly
            260                 265                 270
gga ctc aag gat ctc ttc gaa cgg gga ccc tcc aat gcc ttt ttt ctt     864
Gly Leu Lys Asp Leu Phe Glu Arg Gly Pro Ser Asn Ala Phe Phe Leu
        275                 280                 285
gtg aag ttc tgg gca gac ctc aac acc aac atc gag gat gaa ggc agc     912
Val Lys Phe Trp Ala Asp Leu Asn Thr Asn Ile Glu Asp Glu Gly Ser
    290                 295                 300
tcc ttc tat ggg gtc tcc agc cag tat gag agc ccc gag aac atg atc     960
Ser Phe Tyr Gly Val Ser Ser Gln Tyr Glu Ser Pro Glu Asn Met Ile
305                 310                 315                 320
atc acc tgc tcc acg aag gtc tgc tct ttc ggc aag cag gtg gtg gag     1008
Ile Thr Cys Ser Thr Lys Val Cys Ser Phe Gly Lys Gln Val Val Glu
                325                 330                 335
aaa gtt gag aca gag tat gct cgc tat gag aat gga cac tac tct tac     1056
Lys Val Glu Thr Glu Tyr Ala Arg Tyr Glu Asn Gly His Tyr Ser Tyr
            340                 345                 350
cgc atc cac cgg tcc ccg ctc tgt gag tac atg atc aac ttc atc cac     1104
Arg Ile His Arg Ser Pro Leu Cys Glu Tyr Met Ile Asn Phe Ile His
        355                 360                 365
aag ctc aag cac ctc cct gag aag tac atg atg aac agc gtg ctg gag     1152
Lys Leu Lys His Leu Pro Glu Lys Tyr Met Met Asn Ser Val Leu Glu
    370                 375                 380
aac ttc acc atc ctg cag gtg gtc acc aac aga gac aca cag gag acc     1200
Asn Phe Thr Ile Leu Gln Val Val Thr Asn Arg Asp Thr Gln Glu Thr
385                 390                 395                 400
ttg ctg tgc att gcc tat gtc ttt gag gtg tca gcc agt gag cac ggg     1248

Leu Leu Cys Ile Ala Tyr Val Phe Glu Val Ser Ala Ser Glu His Gly
                405                 410                 415
gct cag cac cac atc tac agg ctg gtg aaa gaa 1281
Ala Gln His His Ile Tyr Arg Leu Val Lys Glu
            420                 425
<210> 29
<211> 435
<212> PRT
<213> Homo sapiens
<400> 29
Ile Ala Ser Asn Ser Trp Asn Ala Ser Ser Ser Pro Gly Glu Ala Arg
1               5                   10                  15
Glu Asp Gly Pro Glu Gly Leu Asp Lys Gly Leu Asp Asn Asp Ala Glu
            20                  25                  30
Gly Val Trp Ser Pro Asp Ile Glu Gln Ser Phe Gln Glu Ala Leu Ala
        35                  40                  45
Ile Tyr Pro Pro Cys Gly Arg Arg Lys Ile Ile Leu Ser Asp Glu Gly
    50                  55                  60
Lys Met Tyr Gly Arg Asn Glu Leu Ile Ala Arg Tyr Ile Lys Leu Arg
65                  70                  75                  80
Thr Gly Lys Thr Arg Thr Arg Lys Gln Val Ser Ser His Ile Gln Val
                85                  90                  95
Leu Ala Arg Lys Lys Val Arg Glu Tyr Gln Val Gly Ile Lys Ala Met
            100                 105                 110
Asn Leu Asp Gln Val Ser Lys Asp Lys Ala Leu Gln Ser Met Ala Ser
        115                 120                 125
Met Ser Ser Ala Gln Ile Val Ser Ala Ser Val Leu Gln Asn Lys Phe
    130                 135                 140
Ser Pro Pro Ser Pro Leu Pro Gln Ala Val Phe Ser Thr Ser Ser Arg
145                 150                 155                 160
Phe Trp Ser Ser Pro Pro Leu Leu Gly Gln Gln Pro Gly Pro Ser Gln
                165                 170                 175
Asp Ile Lys Pro Phe Ala Gln Pro Ala Tyr Pro Ile Gln Pro Pro Leu
            180                 185                 190
Pro Pro Thr Leu Ser Ser Tyr Glu Pro Leu Ala Pro Leu Pro Ser Ala
        195                 200                 205

Ala Ala Ser Val Pro Val Trp Gln Asp Arg Thr Ile Ala Ser Ser Arg
    210                 215                 220
Leu Arg Leu Leu Glu Tyr Ser Ala Phe Met Glu Val Gln Arg Asp Pro
225                 230                 235                 240
Asp Thr Tyr Ser Lys His Leu Phe Val His Ile Gly Gln Thr Asn Pro
                245                 250                 255
Ala Phe Ser Asp Pro Pro Leu Glu Ala Val Asp Val Arg Gln Ile Tyr
            260                 265                 270
Asp Lys Phe Pro Glu Lys Lys Gly Gly Leu Lys Glu Leu Tyr Glu Lys
        275                 280                 285
Gly Pro Pro Asn Ala Phe Phe Leu Val Lys Phe Trp Ala Asp Leu Asn
    290                 295                 300
Ser Thr Ile Gln Glu Gly Pro Gly Ala Phe Tyr Gly Val Ser Ser Gln
305                 310                 315                 320
Tyr Ser Ser Ala Asp Ser Met Thr Ile Ser Val Ser Thr Lys Val Cys
                325                 330                 335
Ser Phe Gly Lys Gln Val Val Glu Lys Val Glu Thr Glu Tyr Ala Arg
            340                 345                 350
Leu Glu Asn Gly Arg Phe Val Tyr Arg Ile His Arg Ser Pro Met Cys
        355                 360                 365
Glu Tyr Met Ile Asn Phe Ile His Lys Leu Lys His Leu Pro Glu Lys
    370                 375                 380
Tyr Met Met Asn Ser Val Leu Glu Asn Phe Thr Ile Leu Gln Val Val
385                 390                 395                 400
Thr Ser Arg Asp Ser Gln Glu Thr Leu Leu Val Ile Ala Phe Val Phe
                405                 410                 415
Glu Val Ser Thr Ser Glu His Gly Ala Gln His His Val Tyr Lys Leu
            420                 425                 430
Val Lys Asp
<210> 30
<211> 1305
<212> DNA
<213> Homo sapiens
<220>
<221> CDS
<223> (1)..(1305)

<400> 30
ata gcg tcc aac agc tgg aac gcc agc agc agc ccc ggg gag gcc cgg    48
Ile Ala Ser Asn Ser Trp Asn Ala Ser Ser Ser Pro Gly Glu Ala Arg
1               5                   10                  15
gag gat ggg ccc gag ggc ctg gac aag ggg ctg gac aac gat gcg gag    96
Glu Asp Gly Pro Glu Gly Leu Asp Lys Gly Leu Asp Asn Asp Ala Glu
            20                  25                  30
ggc gtg tgg agc ccg gac atc gag cag agc ttc cag gag gcc ctg gcc    144
Gly Val Trp Ser Pro Asp Ile Glu Gln Ser Phe Gln Glu Ala Leu Ala
        35                  40                  45
atc tac ccg ccc tgc ggc cgg cgg aag atc atc ctg tca gac gag ggc    192
Ile Tyr Pro Pro Cys Gly Arg Arg Lys Ile Ile Leu Ser Asp Glu Gly
    50                  55                  60
aag atg tac ggc cga aat gag ttg att gca cgc tat att aaa ctg agg    240
Lys Met Tyr Gly Arg Asn Glu Leu Ile Ala Arg Tyr Ile Lys Leu Arg
65                  70                  75                  80
acg ggg aag act cgg acg aga aaa cag gtg tcc agc cac ata cag gtt    288
Thr Gly Lys Thr Arg Thr Arg Lys Gln Val Ser Ser His Ile Gln Val
                85                  90                  95
cta gct cgg aag aag gtg cgg gag tac cag gtt ggc atc aag gcc atg    336
Leu Ala Arg Lys Lys Val Arg Glu Tyr Gln Val Gly Ile Lys Ala Met
            100                 105                 110
aac ctg gac cag gtc tcc aag gac aaa gcc ctt cag agc atg gcg tcc    384
Asn Leu Asp Gln Val Ser Lys Asp Lys Ala Leu Gln Ser Met Ala Ser
        115                 120                 125
atg tcc tct gcc cag atc gtc tct gcc agt gtc ctg cag aac aag ttc    432
Met Ser Ser Ala Gln Ile Val Ser Ala Ser Val Leu Gln Asn Lys Phe
    130                 135                 140
agc cca cct tcc cct ctg ccc cag gcc gtc ttc tcc act tcc tcg cgg    480
Ser Pro Pro Ser Pro Leu Pro Gln Ala Val Phe Ser Thr Ser Ser Arg
145                 150                 155                 160
ttc tgg agc agc ccc cct ctc ctg gga cag cag cct gga ccc tct cag    528
Phe Trp Ser Ser Pro Pro Leu Leu Gly Gln Gln Pro Gly Pro Ser Gln
                165                 170                 175
gac atc aag ccc ttt gca cag cca gcc tac ccc atc cag ccg ccc ctg    576
Asp Ile Lys Pro Phe Ala Gln Pro Ala Tyr Pro Ile Gln Pro Pro Leu

            180                 185                 190
ccg ccg acg ctc agc agt tat gag ccc ctg gcc ccg ctc ccc tca gct 624
Pro Pro Thr Leu Ser Ser Tyr Glu Pro Leu Ala Pro Leu Pro Ser Ala
        195                 200                 205
gct gcc tct gtg cct gtg tgg cag gac cgt acc att gcc tcc tcc cgg 672
Ala Ala Ser Val Pro Val Trp Gln Asp Arg Thr Ile Ala Ser Ser Arg
    210                 215                 220
ctg cgg ctc ctg gag tat tca gcc ttc atg gag gtg cag cga gac cct 720
Leu Arg Leu Leu Glu Tyr Ser Ala Phe Met Glu Val Gln Arg Asp Pro
225                 230                 235                 240
gac acg tac agc aaa cac ctg ttt gtg cac atc ggc cag acg aac ccc 768
Asp Thr Tyr Ser Lys His Leu Phe Val His Ile Gly Gln Thr Asn Pro
                245                 250                 255
gcc ttc tca gac cca ccc ctg gag gca gta gat gtg cgc cag atc tat 816
Ala Phe Ser Asp Pro Pro Leu Glu Ala Val Asp Val Arg Gln Ile Tyr
            260                 265                 270
gac aaa ttc ccc gag aaa aag gga gga ttg aag gag ctc tat gag aag 864
Asp Lys Phe Pro Glu Lys Lys Gly Gly Leu Lys Glu Leu Tyr Glu Lys
        275                 280                 285
ggg ccc cct aat gcc ttc ttc ctt gtc aag ttc tgg gcc gac ctc aac 912
Gly Pro Pro Asn Ala Phe Phe Leu Val Lys Phe Trp Ala Asp Leu Asn
    290                 295                 300
agc acc atc cag gag ggc ccg gga gcc ttc tat ggg gtc agc tct cag 960
Ser Thr Ile Gln Glu Gly Pro Gly Ala Phe Tyr Gly Val Ser Ser Gln
305                 310                 315                 320
tac agc tct gct gat agc atg acc atc agc gtc tcc acc aag gtg tgc 1008
Tyr Ser Ser Ala Asp Ser Met Thr Ile Ser Val Ser Thr Lys Val Cys
                325                 330                 335
tcc ttt ggc aaa cag gtg gta gag aag gtg gag act gag tat gcc agg 1056
Ser Phe Gly Lys Gln Val Val Glu Lys Val Glu Thr Glu Tyr Ala Arg
            340                 345                 350
ctg gag aac ggg cgc ttt gtg tac cgt atc cac cgc tcg ccc atg tgc 1104
Leu Glu Asn Gly Arg Phe Val Tyr Arg Ile His Arg Ser Pro Met Cys
        355                 360                 365
gag tac atg atc aac ttc atc cac aag ctg aag cac ctg ccc gag aag 1152
Glu Tyr Met Ile Asn Phe Ile His Lys Leu Lys His Leu Pro Glu Lys

370                 375                 380
tac atg atg aac agc gtg ctg gag aac ttc acc atc ctg cag gtg gtc     1200
Tyr Met Met Asn Ser Val Leu Glu Asn Phe Thr Ile Leu Gln Val Val
385                 390                 395                 400
acg agc cgg gac tcc cag gag acc ttg ctt gtc att gct ttt gtc ttc     1248
Thr Ser Arg Asp Ser Gln Glu Thr Leu Leu Val Ile Ala Phe Val Phe
                405                 410                 415
gaa gtc tcc acc agt gag cac ggg gcc cag cac cat gtc tac aag ctc     1296
Glu Val Ser Thr Ser Glu His Gly Ala Gln His His Val Tyr Lys Leu
            420                 425                 430
gtc aaa gac                                                         1305
Val Lys Asp
        435
<210> 31
<211> 1132
<212> PRT
<213> Homo sapiens
<400> 31
<210> 32
<211> 3396
<212> DNA
<213> Homo sapiens
<220>
<221> CDS
<223> (1)..(3399)
<400> 32
atg ccg cgc gct ccc cgc tgc cga gcc gtg cgc tcc ctg ctg cgc agc 48
cac tac cgc gag gtg ctg ccg ctg gcc acg ttc gtg cgg cgc ctg ggg 96
ccc cag ggc tgg cgg ctg gtg cag cgc ggg gac ccg gcg gct ttc cgc 144
gcg ctg gtg gcc cag tgc ctg gtg tgc gtg ccc tgg gac gca cgg ccg 192
ccc ccc gcc gcc ccc tcc ttc cgc cag gtg tcc tgc ctg aag gag ctg 240
gtg gcc cga gtg ctg cag agg ctg tgc gag cgc ggc gcg aag aac gtg 288
ctg gcc ttc ggc ttc gcg ctg ctg gac ggg gcc cgc ggg ggc ccc ccc 336
gag gcc ttc acc acc agc gtg cgc agc tac ctg ccc aac acg gtg acc 384
gac gca ctg cgg ggg agc ggg gcg tgg ggg ctg ctg ctg cgc cgc gtg 432
ggc gac gac gtg ctg gtt cac ctg ctg gca cgc tgc gcg ctc ttt gtg 480
ctg gtg gct ccc agc tgc gcc tac cag gtg tgc ggg ccg ccg ctg tac 528
cag ctc ggc gct gcc act cag gcc cgg ccc ccg cca cac gct agt gga 576
ccc cga agg cgt ctg gga tgc gaa cgg gcc tgg aac cat agc gtc agg 624
gag gcc ggg gtc ccc ctg ggc ctg cca gcc ccg ggt gcg agg agg cgc 672
ggg ggc agt gcc agc cga agt ctg ccg ttg ccc aag agg ccc agg cgt 720
ggc gct gcc cct gag ccg gag cgg acg ccc gtt ggg cag ggg tcc tgg 768
gcc cac ccg ggc agg acg cgt gga ccg agt gac cgt ggt ttc tgt gtg 816
gtg tca cct gcc aga ccc gcc gaa gaa gcc acc tct ttg gag ggt gcg 864
ctc tct ggc acg cgc cac tcc cac cca tcc gtg ggc cgc cag cac cac 912
gcg ggc ccc cca tcc aca tcg cgg cca cca cgt ccc tgg gac acg cct 960
tgt ccc ccg gtg tac gcc gag acc aag cac ttc ctc tac tcc tca ggc 1008
gac aag gag cag ctg cgg ccc tcc ttc cta ctc agc tct ctg agg ccc 1056
agc ctg act ggc gct cgg agg ctc gtg gag acc atc ttt ctg ggt tcc 1104
agg ccc tgg atg cca ggg act ccc cgc agg ttg ccc cgc ctg ccc cag 1152
cgc tac tgg caa atg cgg ccc ctg ttt ctg gag ctg ctt ggg aac cac 1200
gcg cag tgc ccc tac ggg gtg ctc ctc aag acg cac tgc ccg ctg cga 1248
gct gcg gtc acc cca gca gcc ggt gtc tgt gcc cgg gag aag ccc cag 1296
ggc tct gtg gcg gcc ccc gag gag gag gac aca gac ccc cgt cgc ctg 1344
gtg cag ctg ctc cgc cag cac agc agc ccc tgg cag gtg tac ggc ttc 1392
gtg cgg gcc tgc ctg cgc cgg ctg gtg ccc cca ggc ctc tgg ggc tcc 1440
agg cac aac gaa cgc cgc ttc ctc agg aac acc aag aag ttc atc tcc 1488
ctg ggg aag cat gcc aag ctc tcg ctg cag gag ctg acg tgg aag atg 1536
agc gtg cgg gac tgc gct tgg ctg cgc agg agc cca ggg gtt ggc tgt 1584
gtt ccg gcc gca gag cac cgt ctg cgt gag gag atc ctg gcc aag ttc 1632
ctg cac tgg ctg atg agt gtg tac gtc gtc gag ctg ctc agg tct ttc 1680
ttt tat gtc acg gag acc acg ttt caa aag aac agg ctc ttt ttc tac 1728
cgg aag agt gtc tgg agc aag ttg caa agc att gga atc aga cag cac 1776
ttg aag agg gtg cag ctg cgg gag ctg tcg gaa gca gag gtc agg cag 1824
cat cgg gaa gcc agg ccc gcc ctg ctg acg tcc aga ctc cgc ttc atc 1872
ccc aag cct gac ggg ctg cgg ccg att gtg aac atg gac tac gtc gtg 1920
gga gcc aga acg ttc cgc aga gaa aag agg gcc gag cgt ctc acc tcg 1968
agg gtg aag gca ctg ttc agc gtg ctc aac tac gag cgg gcg cgg cgc 2016
ccc ggc ctc ctg ggc gcc tct gtg ctg ggc ctg gac gat atc cac agg 2064
gcc tgg cgc acc ttc gtg ctg cgt gtg cgg gcc cag gac ccg ccg cct 2112
gag ctg tac ttt gtc aag gtg gat gtg acg ggc gcg tac gac acc atc 2160
ccc cag gac agg ctc acg gag gtc atc gcc agc atc atc aaa ccc cag 2208
aac acg tac tgc gtg cgt cgg tat gcc gtg gtc cag aag gcc gcc cat 2256
ggg cac gtc cgc aag gcc ttc aag agc cac gtc tct acc ttg aca gac 2304
ctc cag ccg tac atg cga cag ttc gtg gct cac ctg cag gag acc agc 2352
ccg ctg agg gat gcc gtc gtc atc gag cag agc tcc tcc ctg aat gag 2400
gcc agc agt ggc ctc ttc gac gtc ttc cta cgc ttc atg tgc cac cac 2448
gcc gtg cgc atc agg ggc aag tcc tac gtc cag tgc cag ggg atc ccg 2496
cag ggc tcc atc ctc tcc acg ctg ctc tgc agc ctg tgc tac ggc gac 2544
atg gag aac aag ctg ttt gcg ggg att cgg cgg gac ggg ctg ctc ctg 2592
cgt ttg gtg gat gat ttc ttg ttg gtg aca cct cac ctc acc cac gcg 2640
aaa acc ttc ctc agg acc ctg gtc cga ggt gtc cct gag tat ggc tgc 2688
gtg gtg aac ttg cgg aag aca gtg gtg aac ttc cct gta gaa gac gag 2736
gcc ctg ggt ggc acg gct ttt gtt cag atg ccg gcc cac ggc cta ttc 2784
ccc tgg tgc ggc ctg ctg ctg gat acc cgg acc ctg gag gtg cag agc 2832
gac tac tcc agc tat gcc cgg acc tcc atc aga gcc agt ctc acc ttc 2880
aac cgc ggc ttc aag gct ggg agg aac atg cgt cgc aaa ctc ttt ggg 2928
gtc ttg cgg ctg aag tgt cac agc ctg ttt ctg gat ttg cag gtg aac 2976
agc ctc cag acg gtg tgc acc aac atc tac aag atc ctc ctg ctg cag 3024
gcg tac agg ttt cac gca tgt gtg ctg cag ctc cca ttt cat cag caa 3072
gtt tgg aag aac ccc aca ttt ttc ctg cgc gtc atc tct gac acg gcc 3120
tcc ctc tgc tac tcc atc ctg aaa gcc aag aac gca ggg atg tcg ctg 3168
ggg gcc aag ggc gcc gcc ggc cct ctg ccc tcc gag gcc gtg cag tgg 3216
ctg tgc cac caa gca ttc ctg ctc aag ctg act cga cac cgt gtc acc 3264
tac gtg cca ctc ctg ggg tca ctc agg aca gcc cag acg cag ctg agt 3312
cgg aag ctc ccg ggg acg acg ctg act gcc ctg gag gcc gca gcc aac 3360
ccg gca ctg ccc tca gac ttc aag acc atc ctg gac 3396
<210> 33
<211> 21
<212> DNA
<213> Artificial Sequence
<220>
<223> Description of Artificial Sequence: artificially synthesized primer
sequence
<400> 33
ttggcttcca ggccataatt g 21
<210> 34
<211> 20
<212> DNA
<213> Artificial Sequence

<220>

<223> Description of Artificial Sequence: artificially synthesized primer
sequence
<400> 34
aagagggcag atctatcgga                                                    20
<210> 35
<211> 20
<212> DNA
<213> Artificial Sequence
<220>
<223> Description of Artificial Sequence: artificially synthesized primer
sequence
<400> 35
atggatctcc tgaaggtgct                                                    20
<210> 36
<211> 20
<212> DNA
<213> Artificial Sequence
<220>
<223> Description of Artificial Sequence: artificially synthesized primer
sequence
<400> 36
aagagggcag atctatcgga                                                    20
<210> 37
<211> 23
<212> DNA
<213> Artificial Sequence
<220>
<223> Description of Artificial Sequence: artificially synthesized primer
sequence
<400> 37
ggaagagtga gcggccatca agg                                                23
<210> 38
<211> 22
<212> DNA
<213> Artificial Sequence
<220>

<223> Description of Artificial Sequence: artificially synthesized primer
sequence
<400> 38
ctgctggaga ggttattcct cg                                                22
<210> 39
<211> 24
<212> DNA
<213> Artificial Sequence
<220>
<223> Description of Artificial Sequence: artificially synthesized primer
sequence
<400> 39
gccaacacca acctgtccaa gttc                                              24
<210> 40
<211> 24
<212> DNA
<213> Artificial Sequence
<220>
<223> Description of Artificial Sequence: artificially synthesized primer
sequence
<400> 40
tgcaaaggct ccaggtctga gggc                                              24
<210> 41
<211> 19
<212> DNA
<213> Artificial Sequence
<220>
<223> Description of Artificial Sequence: artificially synthesized primer
sequence
<400> 41
ctctctctcc tcaggacaa                                                    19
<210> 42
<211> 22
<212> DNA
<213> Artificial Sequence
<220>

<223> Description of Artificial Sequence: artificially synthesized primer
sequence
<400> 42
tggagcaaaa cagaatggct gg 22
<210> 43
<211> 24
<212> DNA
<213> Artificial Sequence
<220>
<223> Description of Artificial Sequence: artificially synthesized primer
sequence
<400> 43
ctgagatgtc tctctctctc ttag 24
<210> 44
<211> 20
<212> DNA
<213> Artificial Sequence
<220>
<223> Description of Artificial Sequence: artificially synthesized primer
sequence
<400> 44
acaatgactg atgagagatg 20
<210> 45
<211> 18
<212> DNA
<213> Artificial Sequence
<220>
<223> Description of Artificial Sequence: artificially synthesized primer
sequence
<400> 45
cagacctgaa ggagacct 18
<210> 46
<211> 18
<212> DNA
<213> Artificial Sequence
<220>

<223> Description of Artificial Sequence: artificially synthesized primer
sequence
<400> 46
gtcagcgtaa acagttgc 18
<210> 47
<211> 20
<212> DNA
<213> Artificial Sequence
<220>
<223> Description of Artificial Sequence: artificially synthesized primer
sequence
<400> 47
gccaagaagc ggatagaagg 20
<210> 48
<211> 20
<212> DNA
<213> Artificial Sequence
<220>
<223> Description of Artificial Sequence: artificially synthesized primer
sequence
<400> 48
ctgtggttca gggctcagtc 20
<210> 49
<211> 20
<212> DNA
<213> Artificial Sequence
<220>
<223> Description of Artificial Sequence: artificially synthesized primer
sequence
<400> 49
cagtggagct ggacaaagcc 20
<210> 50
<211> 20
<212> DNA
<213> Artificial Sequence
<220>

<223> Description of Artificial Sequence: artificially synthesized primer
sequence
<400> 50
tagcgacggt tctggaacca                                                    20
<210> 51
<211> 20
<212> DNA
<213> Artificial Sequence
<220>
<223> Description of Artificial Sequence: artificially synthesized primer
sequence
<400> 51
ctgtcatctc actatgggca                                                    20
<210> 52
<211> 20
<212> DNA
<213> Artificial Sequence
<220>
<223> Description of Artificial Sequence: artificially synthesized primer
sequence
<400> 52
ccaagtccga gcaggaattt                                                    20
<210> 53
<211> 20
<212> DNA
<213> Artificial Sequence
<220>
<223> Description of Artificial Sequence: artificially synthesized primer
sequence
<400> 53
aagacgtcaa gccctttgtg                                                    20
<210> 54
<211> 20
<212> DNA
<213> Artificial Sequence
<220>

<223> Description of Artificial Sequence: artificially synthesized primer
sequence
<400> 54
aaaggagcac actttggtgg                                                    20
<210> 55
<211> 20
<212> DNA
<213> Artificial Sequence
<220>
<223> Description of Artificial Sequence: artificially synthesized primer
sequence
<400> 55
agcaagaata cgatgccatc                                                    20
<210> 56
<211> 20
<212> DNA
<213> Artificial Sequence
<220>
<223>Description of Artificial Sequence: artificially
     synthesized primer sequence
<400> 56
gaaggggtgg tggtacggtc                                                    20
<210> 57
<211> 20
<212> DNA
<213> Artificial Sequence
<220>
<223> Description of Artificial Sequence: artificially synthesized primer
sequence
<400> 57
tgggaatggc tatgtcagtg                                                    20
<210> 58
<211> 20
<212> DNA
<213> Artificial Sequence
<220>

<223> Description of Artificial Sequence: artificially synthesized primer
sequence
<400> 58
ctggtaatct gtgttgtagg                                                    20
<210> 59
<211> 20
<212> DNA
<213> Artificial Sequence
<220>
<223> Description of Artificial Sequence: artificially synthesized primer
sequence
<400> 59
caagggcctc tccaaacttg                                                    20
<210> 60
<211> 20
<212> DNA
<213> Artificial Sequence
<220>
<223> Description of Artificial Sequence: artificially synthesized primer
sequence
<400> 60
gccccagaga cagcattcca                                                    20
<210> 61
<211> 268
<212> PRT
<213> Homo sapiens
<400> 61
Met Ala Gln Pro Leu Cys Pro Pro Leu Ser Glu Ser Trp Met Leu Ser
 1               5                  10                  15

Ala Ala Trp Gly Pro Thr Arg Arg Pro Pro Pro Ser Asp Lys Asp Cys
            20                  25                  30

Gly Arg Ser Leu Val Ser Ser Pro Asp Ser Trp Gly Ser Thr Pro Ala
        35                  40                  45

Asp Ser Pro Val Ala Ser Pro Ala Arg Pro Gly Thr Leu Arg Asp Pro
    50                  55                  60

Arg Ala Pro Ser Val Gly Arg Arg Gly Ala Arg Ser Ser Arg Leu Gly
65                  70                  75                  80

Ser Gly Gln Arg Gln Ser Ala Ser Glu Arg Glu Lys Leu Arg Met Arg
                85                  90                  95

Thr Leu Ala Arg Ala Leu His Glu Leu Arg Arg Phe Leu Pro Pro Ser
            100                 105                 110

Val Ala Pro Ala Gly Gln Ser Leu Thr Lys Ile Glu Thr Leu Arg Leu
        115                 120                 125

Ala Ile Arg Tyr Ile Gly His Leu Ser Ala Val Leu Gly Leu Ser Glu
    130                 135                 140

Glu Ser Leu Gln Arg Arg Cys Arg Gln Arg Gly Asp Ala Gly Ser Pro
145                 150                 155                 160

Arg Gly Cys Pro Leu Cys Pro Asp Asp Cys Pro Ala Gln Met Gln Thr
                165                 170                 175

Arg Thr Gln Ala Glu Gly Gln Gly Gln Gly Arg Gly Leu Gly Leu Val
            180                 185                 190

Ser Ala Val Arg Ala Gly Ala Ser Trp Gly Ser Pro Pro Ala Cys Pro
        195                 200                 205

Gly Ala Arg Ala Ala Pro Glu Pro Arg Asp Pro Pro Ala Leu Phe Ala
    210                 215                 220

Glu Ala Ala Cys Pro Glu Gly Gln Ala Met Glu Pro Ser Pro Pro Ser
225                 230                 235                 240

Pro Leu Leu Pro Gly Asp Val Leu Ala Leu Leu Glu Thr Trp Met Pro
                245                 250                 255

Leu Ser Pro Leu Glu Trp Leu Pro Glu Glu Pro Lys
            260                 265
<210> 62
<211> 804
<212> DNA
<213> Homo sapiens
<220>
<221> CDS
<223> (1)..(807)
<400> 62
atg gcc cag ccc ctg tgc ccg ccg ctc tcc gag tcc tgg atg ctc tct 48
Met Ala Gln Pro Leu Cys Pro Pro Leu Ser Glu Ser Trp Met Leu Ser
1               5                   10                  15

gcg gcc tgg ggc cca act cgg cgg ccg ccg ccc tcc gac aag gac tgc 96
Ala Ala Trp Gly Pro Thr Arg Arg Pro Pro Pro Ser Asp Lys Asp Cys
            20                  25                  30

ggc cgc tcc ctc gtc tcg tcc cca gac tca tgg ggc agc acc cca gcc 144
Gly Arg Ser Leu Val Ser Ser Pro Asp Ser Trp Gly Ser Thr Pro Ala
        35                  40                  45

gac agc ccc gtg gcg agc ccc gcg cgg cca ggc acc ctc cgg gac ccc 192
Asp Ser Pro Val Ala Ser Pro Ala Arg Pro Gly Thr Leu Arg Asp Pro
    50                  55                  60

cgc gcc ccc tcc gta ggt agg cgc ggc gcg cgc agc agc cgc ctg ggc 240
Arg Ala Pro Ser Val Gly Arg Arg Gly Ala Arg Ser Ser Arg Leu Gly
65                  70                  75                  80

agc ggg cag agg cag agc gcc agt gag cgg gag aaa ctg cgc atg cgc 288
Ser Gly Gln Arg Gln Ser Ala Ser Glu Arg Glu Lys Leu Arg Met Arg
                85                  90                  95

acg ctg gcc cgc gcc ctg cac gag ctg cgc cgc ttt cta ccg ccg tcc 336
Thr Leu Ala Arg Ala Leu His Glu Leu Arg Arg Phe Leu Pro Pro Ser
            100                 105                 110

gtg gcg ccc gcg ggc cag agc ctg acc aag atc gag acg ctg cgc ctg 384
Val Ala Pro Ala Gly Gln Ser Leu Thr Lys Ile Glu Thr Leu Arg Leu
        115                 120                 125

gct atc cgc tat atc ggc cac ctg tcg gcc gtg cta ggc ctc agc gag 432
Ala Ile Arg Tyr Ile Gly His Leu Ser Ala Val Leu Gly Leu Ser Glu
    130                 135                 140

gag agt ctc cag cgc cgg tgc cgg cag cgc ggt gac gcg ggg tcc cct 480
Glu Ser Leu Gln Arg Arg Cys Arg Gln Arg Gly Asp Ala Gly Ser Pro
145                 150                 155                 160

cgg ggc tgc ccg ctg tgc ccc gac gac tgc ccc gcg cag atg cag aca 528
Arg Gly Cys Pro Leu Cys Pro Asp Asp Cys Pro Ala Gln Met Gln Thr
                165                 170                 175

cgg acg cag gct gag ggg cag ggg cag ggg cgc ggg ctg ggc ctg gta 576
Arg Thr Gln Ala Glu Gly Gln Gly Gln Gly Arg Gly Leu Gly Leu Val
            180                 185                 190

tcc gcc gtc cgc gcc ggg gcg tcc tgg gga tcc ccg cct gcc tgc ccc 624
Ser Ala Val Arg Ala Gly Ala Ser Trp Gly Ser Pro Pro Ala Cys Pro
        195                 200                 205

gga gcc cga gct gca ccc gag ccg cgc gac ccg cct gcg ctg ttc gcc 672
Gly Ala Arg Ala Ala Pro Glu Pro Arg Asp Pro Pro Ala Leu Phe Ala
    210                 215                 220

gag gcg gcg tgc cct gaa ggg cag gcg atg gag cca agc cca ccg tcc 720
Glu Ala Ala Cys Pro Glu Gly Gln Ala Met Glu Pro Ser Pro Pro Ser
225                 230                 235                 240

ccg ctc ctt ccg ggc gac gtg ctg gct ctg ttg gag acc tgg atg ccc 768
Pro Leu Leu Pro Gly Asp Val Leu Ala Leu Leu Glu Thr Trp Met Pro
                245                 250                 255

ctc tcg cct ctg gag tgg ctg cct gag gag ccc aag 804
Leu Ser Pro Leu Glu Trp Leu Pro Glu Glu Pro Lys
            260                 265
<210> 63
<211> 215
<212> PRT
<213> Homo sapiens
<400> 63
Met Gly Ser Pro Arg Ser Ala Leu Ser Cys Leu Leu Leu His Leu Leu
1               5                   10                  15

Val Leu Cys Leu Gln Ala Gln Val Thr Val Gln Ser Ser Pro Asn Phe
            20                  25                  30

Thr Gln His Val Arg Glu Gln Ser Leu Val Thr Asp Gln Leu Ser Arg
        35                  40                  45

Arg Leu Ile Arg Thr Tyr Gln Leu Tyr Ser Arg Thr Ser Gly Lys His
    50                  55                  60

Val Gln Val Leu Ala Asn Lys Arg Ile Asn Ala Met Ala Glu Asp Gly
65                  70                  75                  80

Asp Pro Phe Ala Lys Leu Ile Val Glu Thr Asp Thr Phe Gly Ser Arg
                85                  90                  95

Val Arg Val Arg Gly Ala Glu Thr Gly Leu Tyr Ile Cys Met Asn Lys
            100                 105                 110

Lys Gly Lys Leu Ile Ala Lys Ser Asn Gly Lys Gly Lys Asp Cys Val
        115                 120                 125

Phe Thr Glu Ile Val Leu Glu Asn Asn Tyr Thr Ala Leu Gln Asn Ala
    130                 135                 140

Lys Tyr Glu Gly Trp Tyr Met Ala Phe Thr Arg Lys Gly Arg Pro Arg
145                 150                 155                 160

Lys Gly Ser Lys Thr Arg Gln His Gln Arg Glu Val His Phe Met Lys
                165                 170                 175

Arg Leu Pro Arg Gly His His Thr Thr Glu Gln Ser Leu Arg Phe Glu
            180                 185                 190

Phe Leu Asn Tyr Pro Pro Phe Thr Arg Ser Leu Arg Gly Ser Gln Arg
        195                 200                 205

Thr Trp Ala Pro Glu Pro Arg
    210

<210> 64
<211> 645
<212> DNA
<213> Homo sapiens
<220>
<221> CDS
<223> (1)..(648)
<400> 64
atg ggc agc ccc cgc tcc gcg ctg agc tgc ctg ctg ttg cac ttg ctg    48
Met Gly Ser Pro Arg Ser Ala Leu Ser Cys Leu Leu Leu His Leu Leu
  1               5                  10                  15

gtc ctc tgc ctc caa gcc cag gta act gtt cag tcc tca cct aat ttt    96
Val Leu Cys Leu Gln Ala Gln Val Thr Val Gln Ser Ser Pro Asn Phe
            20                  25                  30

aca cag cat gtg agg gag cag agc ctg gtg acg gat cag ctc agc cgc    144

Thr Gln His Val Arg Glu Gln Ser Leu Val Thr Asp Gln Leu Ser Arg
        35                  40                  45

cgc ctc atc cgg acc tac caa ctc tac agc cgc acc agc ggg aag cac 192
Arg Leu Ile Arg Thr Tyr Gln Leu Tyr Ser Arg Thr Ser Gly Lys His
    50                  55                  60

gtg cag gtc ctg gcc aac aag cgc atc aac gcc atg gca gag gac ggc 240
Val Gln Val Leu Ala Asn Lys Arg Ile Asn Ala Met Ala Glu Asp Gly
65                  70                  75                  80

gac ccc ttc gca aag ctc atc gtg gag acg gac acc ttt gga agc aga 288
Asp Pro Phe Ala Lys Leu Ile Val Glu Thr Asp Thr Phe Gly Ser Arg
                85                  90                  95

gtt cga gtc cga gga gcc gag acg ggc ctc tac atc tgc atg aac aag 336
Val Arg Val Arg Gly Ala Glu Thr Gly Leu Tyr Ile Cys Met Asn Lys
            100                 105                 110

aag ggg aag ctg atc gcc aag agc aac ggc aaa ggc aag gac tgc gtc 384
Lys Gly Lys Leu Ile Ala Lys Ser Asn Gly Lys Gly Lys Asp Cys Val
        115                 120                 125

ttc acg gag att gtg ctg gag aac aac tac aca gcg ctg cag aat gcc 432
Phe Thr Glu Ile Val Leu Glu Asn Asn Tyr Thr Ala Leu Gln Asn Ala
    130                 135                 140

aag tac gag ggc tgg tac atg gcc ttc acc cgc aag ggc cgg ccc cgc 480
Lys Tyr Glu Gly Trp Tyr Met Ala Phe Thr Arg Lys Gly Arg Pro Arg
145                 150                 155                 160

aag ggc tcc aag acg cgg cag cac cag cgt gag gtc cac ttc atg aag 528
Lys Gly Ser Lys Thr Arg Gln His Gln Arg Glu Val His Phe Met Lys
                165                 170                 175

cgg ctg ccc cgg ggc cac cac acc acc gag cag agc ctg cgc ttc gag 576

Arg Leu Pro Arg Gly His His Thr Thr Glu Gln Ser Leu Arg Phe Glu
            180                 185                 190

ttc ctc aac tac ccg ccc ttc acg cgc agc ctg cgc ggc agc cag agg 624
Phe Leu Asn Tyr Pro Pro Phe Thr Arg Ser Leu Arg Gly Ser Gln Arg
        195                 200                 205

act tgg gcc ccg gaa ccc cga 645
Thr Trp Ala Pro Glu Pro Arg
    210                 215
<210> 65
<211> 212
<212> PRT
<213> Homo sapiens
<400> 65
Met Asp Tyr Leu Leu Met Ile Phe Ser Leu Leu Phe Val Ala Cys Gln
1               5                   10                  15

Gly Ala Pro Glu Thr Ala Val Leu Gly Ala Glu Leu Ser Ala Val Gly
            20                  25                  30

Glu Asn Gly Gly Glu Lys Pro Thr Pro Ser Pro Pro Trp Arg Leu Arg
        35                  40                  45

Arg Ser Lys Arg Cys Ser Cys Ser Ser Leu Met Asp Lys Glu Cys Val
    50                  55                  60

Tyr Phe Cys His Leu Asp Ile Ile Trp Val Asn Thr Pro Glu His Val
65                  70                  75                  80

Val Pro Tyr Gly Leu Gly Ser Pro Arg Ser Lys Arg Ala Leu Glu Asn
                85                  90                  95

Leu Leu Pro Thr Lys Ala Thr Asp Arg Glu Asn Arg Cys Gln Cys Ala
            100                 105                 110

Ser Gln Lys Asp Lys Lys Cys Trp Asn Phe Cys Gln Ala Gly Lys Glu
        115                 120                 125

Leu Arg Ala Glu Asp Ile Met Glu Lys Asp Trp Asn Asn His Lys Lys
    130                 135                 140

Gly Lys Asp Cys Ser Lys Leu Gly Lys Lys Cys Ile Tyr Gln Gln Leu
145                 150                 155                 160

Val Arg Gly Arg Lys Ile Arg Arg Ser Ser Glu Glu His Leu Arg Gln
                165                 170                 175

Thr Arg Ser Glu Thr Met Arg Asn Ser Val Lys Ser Ser Phe His Asp
            180                 185                 190

Pro Lys Leu Lys Gly Lys Pro Ser Arg Glu Arg Tyr Val Thr His Asn
        195                 200                 205

Arg Ala His Trp
    210
<210> 66
<211> 636
<212> DNA
<213> Homo sapiens
<220>
<221> CDS
<223> (1)..(639)
<400> 66
atg gat tat ttg ctc atg att ttc tct ctg ctg ttt gtg gct tgc caa    48
Met Asp Tyr Leu Leu Met Ile Phe Ser Leu Leu Phe Val Ala Cys Gln
1               5                   10                  15

gga gct cca gaa aca gca gtc tta ggc gct gag ctc agc gcg gtg ggt    96
Gly Ala Pro Glu Thr Ala Val Leu Gly Ala Glu Leu Ser Ala Val Gly
            20                  25                  30

gag aac ggc ggg gag aaa ccc act ccc agt cca ccc tgg cgg ctc cgc 144
Glu Asn Gly Gly Glu Lys Pro Thr Pro Ser Pro Pro Trp Arg Leu Arg
        35                  40                  45

cgg tcc aag cgc tgc tcc tgc tcg tcc ctg atg gat aaa gag tgt gtc 192
Arg Ser Lys Arg Cys Ser Cys Ser Ser Leu Met Asp Lys Glu Cys Val
    50                  55                  60

tac ttc tgc cac ctg gac atc att tgg gtc aac act ccc gag cac gtt 240
Tyr Phe Cys His Leu Asp Ile Ile Trp Val Asn Thr Pro Glu His Val
65                  70                  75                  80

gtt ccg tat gga ctt gga agc cct agg tcc aag aga gcc ttg gag aat 288
Val Pro Tyr Gly Leu Gly Ser Pro Arg Ser Lys Arg Ala Leu Glu Asn
                85                  90                  95

tta ctt ccc aca aag gca aca gac cgt gag aat aga tgc caa tgt gct 336
Leu Leu Pro Thr Lys Ala Thr Asp Arg Glu Asn Arg Cys Gln Cys Ala
            100                 105                 110

agc caa aaa gac aag aag tgc tgg aat ttt tgc caa gca gga aaa gaa 384
Ser Gln Lys Asp Lys Lys Cys Trp Asn Phe Cys Gln Ala Gly Lys Glu
        115                 120                 125

ctc agg gct gaa gac att atg gag aaa gac tgg aat aat cat aag aaa 432
Leu Arg Ala Glu Asp Ile Met Glu Lys Asp Trp Asn Asn His Lys Lys
    130                 135                 140

gga aaa gac tgt tcc aag ctt ggg aaa aag tgt att tat cag cag tta 480
Gly Lys Asp Cys Ser Lys Leu Gly Lys Lys Cys Ile Tyr Gln Gln Leu
145                 150                 155                 160

gtg aga gga aga aaa atc aga aga agt tca gag gaa cac cta aga caa 528
Val Arg Gly Arg Lys Ile Arg Arg Ser Ser Glu Glu His Leu Arg Gln
                165                 170                 175

acc agg tcg gag acc atg aga aac agc gtc aaa tca tct ttt cat gat     576
Thr Arg Ser Glu Thr Met Arg Asn Ser Val Lys Ser Ser Phe His Asp
            180                 185                 190

ccc aag ctg aaa ggc aag ccc tcc aga gag cgt tat gtg acc cac aac     624
Pro Lys Leu Lys Gly Lys Pro Ser Arg Glu Arg Tyr Val Thr His Asn
        195                 200                 205

cga gca cat tgg                                                     636
Arg Ala His Trp
    210
<210> 67
<211> 143
<212> PRT
<213> Homo sapiens
<400> 67
Met Gln His Arg Gly Phe Leu Leu Leu Thr Leu Leu Ala Leu Leu Ala
 1               5                  10                  15

Leu Thr Ser Ala Val Ala Lys Lys Lys Asp Lys Val Lys Lys Gly Gly
            20                  25                  30

Pro Gly Ser Glu Cys Ala Glu Trp Ala Trp Gly Pro Cys Thr Pro Ser
        35                  40                  45

Ser Lys Asp Cys Gly Val Gly Phe Arg Glu Gly Thr Cys Gly Ala Gln
    50                  55                  60

Thr Gln Arg Ile Arg Cys Arg Val Pro Cys Asn Trp Lys Lys Glu Phe
65                  70                  75                  80

Gly Ala Asp Cys Lys Tyr Lys Phe Glu Asn Trp Gly Ala Cys Asp Gly
                85                  90                  95

Gly Thr Gly Thr Lys Val Arg Gln Gly Thr Leu Lys Lys Ala Arg Tyr
            100                 105                 110

Asn Ala Gln Cys Gln Glu Thr Ile Arg Val Thr Lys Pro Cys Thr Pro
        115                 120                 125

Lys Thr Lys Ala Lys Ala Lys Ala Lys Lys Gly Lys Gly Lys Asp
    130                 135                 140
<210> 68
<211> 429
<212> DNA
<213> Homo sapiens
<220>
<221> CDS
<223> (1)..(432)
<400> 68
atg cag cac cga ggc ttc ctc ctc ctc acc ctc ctc gcc ctg ctg gcg    48
Met Gln His Arg Gly Phe Leu Leu Leu Thr Leu Leu Ala Leu Leu Ala
  1               5                  10                  15

ctc acc tcc gcg gtc gcc aaa aag aaa gat aag gtg aag aag ggc ggc    96
Leu Thr Ser Ala Val Ala Lys Lys Lys Asp Lys Val Lys Lys Gly Gly
             20                  25                  30

ccg ggg agc gag tgc gct gag tgg gcc tgg ggg ccc tgc acc ccc agc    144
Pro Gly Ser Glu Cys Ala Glu Trp Ala Trp Gly Pro Cys Thr Pro Ser
         35                  40                  45

agc aag gat tgc ggc gtg ggt ttc cgc gag ggc acc tgc ggg gcc cag    192
Ser Lys Asp Cys Gly Val Gly Phe Arg Glu Gly Thr Cys Gly Ala Gln
     50                  55                  60

acc cag cgc atc cgg tgc agg gtg ccc tgc aac tgg aag aag gag ttt    240
Thr Gln Arg Ile Arg Cys Arg Val Pro Cys Asn Trp Lys Lys Glu Phe
 65                  70                  75                  80

gga gcc gac tgc aag tac aag ttt gag aac tgg ggt gcg tgt gat ggg    288
Gly Ala Asp Cys Lys Tyr Lys Phe Glu Asn Trp Gly Ala Cys Asp Gly

                85                  90                  95

ggc aca ggc acc aaa gtc cgc caa ggc acc ctg aag aag gcg cgc tac 336
Gly Thr Gly Thr Lys Val Arg Gln Gly Thr Leu Lys Lys Ala Arg Tyr
            100                 105                 110

aat gct cag tgc cag gag acc atc cgc gtc acc aag ccc tgc acc ccc 384
Asn Ala Gln Cys Gln Glu Thr Ile Arg Val Thr Lys Pro Cys Thr Pro
        115                 120                 125

aag acc aaa gca aag gcc aaa gcc aag aaa ggg aag gga aag gac 429
Lys Thr Lys Ala Lys Ala Lys Ala Lys Lys Gly Lys Gly Lys Asp
    130                 135                 140
<210> 69
<211> 408
<212> PRT
<213> Homo sapiens
<400> 69
Met Ile Pro Gly Asn Arg Met Leu Met Val Val Leu Leu Cys Gln Val
1               5                   10                  15

Leu Leu Gly Gly Ala Ser His Ala Ser Leu Ile Pro Glu Thr Gly Lys
            20                  25                  30

Lys Lys Val Ala Glu Ile Gln Gly His Ala Gly Gly Arg Arg Ser Gly
        35                  40                  45

Gln Ser His Glu Leu Leu Arg Asp Phe Glu Ala Thr Leu Leu Gln Met
    50                  55                  60

Phe Gly Leu Arg Arg Arg Pro Gln Pro Ser Lys Ser Ala Val Ile Pro
65                  70                  75                  80

Asp Tyr Met Arg Asp Leu Tyr Arg Leu Gln Ser Gly Glu Glu Glu Glu
                85                  90                  95

Glu Gln Ile His Ser Thr Gly Leu Glu Tyr Pro Glu Arg Pro Ala Ser
            100                 105                 110

Arg Ala Asn Thr Val Arg Ser Phe His His Glu Glu His Leu Glu Asn
        115                 120                 125

Ile Pro Gly Thr Ser Glu Asn Ser Ala Phe Arg Phe Leu Phe Asn Leu
    130                 135                 140

Ser Ser Ile Pro Glu Asn Glu Ala Ile Ser Ser Ala Glu Leu Arg Leu
145                 150                 155                 160

Phe Arg Glu Gln Val Asp Gln Gly Pro Asp Trp Glu Arg Gly Phe His
                165                 170                 175

Arg Ile Asn Ile Tyr Glu Val Met Lys Pro Pro Ala Glu Val Val Pro
            180                 185                 190

Gly His Leu Ile Thr Arg Leu Leu Asp Thr Arg Leu Val His His Asn
        195                 200                 205

Val Thr Arg Trp Glu Thr Phe Asp Val Ser Pro Ala Val Leu Arg Trp
    210                 215                 220

Thr Arg Glu Lys Gln Pro Asn Tyr Gly Leu Ala Ile Glu Val Thr His
225                 230                 235                 240

Leu His Gln Thr Arg Thr His Gln Gly Gln His Val Arg Ile Ser Arg
                245                 250                 255

Ser Leu Pro Gln Gly Ser Gly Asn Trp Ala Gln Leu Arg Pro Leu Leu
            260                 265                 270

Val Thr Phe Gly His Asp Gly Arg Gly His Ala Leu Thr Arg Arg Arg
        275                 280                 285

Arg Ala Lys Arg Ser Pro Lys His His Ser Gln Arg Ala Arg Lys Lys
    290                 295                 300

Asn Lys Asn Cys Arg Arg His Ser Leu Tyr Val Asp Phe Ser Asp Val
305                 310                 315                 320

Gly Trp Asn Asp Trp Ile Val Ala Pro Pro Gly Tyr Gln Ala Phe Tyr
                325                 330                 335

Cys His Gly Asp Cys Pro Phe Pro Leu Ala Asp His Leu Asn Ser Thr
            340                 345                 350

Asn His Ala Ile Val Gln Thr Leu Val Asn Ser Val Asn Ser Ser Ile
        355                 360                 365

Pro Lys Ala Cys Cys Val Pro Thr Glu Leu Ser Ala Ile Ser Met Leu
    370                 375                 380

Tyr Leu Asp Glu Tyr Asp Lys Val Val Leu Lys Asn Tyr Gln Glu Met
385                 390                 395                 400

Val Val Glu Gly Cys Gly Cys Arg
                405
<210> 70
<211> 1224
<212> DNA
<213> Homo sapiens
<220>
<221> CDS
<223> (1)..(1227)
<400> 70
atg att cct ggt aac cga atg ctg atg gtc gtt tta tta tgc caa gtc    48
Met Ile Pro Gly Asn Arg Met Leu Met Val Val Leu Leu Cys Gln Val
  1               5                  10                  15

ctg cta gga ggc gcg agc cat gct agt ttg ata cct gag acg ggg aag    96

Leu Leu Gly Gly Ala Ser His Ala Ser Leu Ile Pro Glu Thr Gly Lys
            20                  25                  30

aaa aaa gtc gcc gag att cag ggc cac gcg gga gga cgc cgc tca ggg 144
Lys Lys Val Ala Glu Ile Gln Gly His Ala Gly Gly Arg Arg Ser Gly
        35                  40                  45

cag agc cat gag ctc ctg cgg gac ttc gag gcg aca ctt ctg cag atg 192
Gln Ser His Glu Leu Leu Arg Asp Phe Glu Ala Thr Leu Leu Gln Met
    50                  55                  60

ttt ggg ctg cgc cgc cgc ccg cag cct agc aag agt gcc gtc att ccg 240
Phe Gly Leu Arg Arg Arg Pro Gln Pro Ser Lys Ser Ala Val Ile Pro
65                  70                  75                  80

gac tac atg cgg gat ctt tac cgg ctt cag tct ggg gag gag gag gaa 288
Asp Tyr Met Arg Asp Leu Tyr Arg Leu Gln Ser Gly Glu Glu Glu Glu
                85                  90                  95

gag cag atc cac agc act ggt ctt gag tat cct gag cgc ccg gcc agc 336
Glu Gln Ile His Ser Thr Gly Leu Glu Tyr Pro Glu Arg Pro Ala Ser
            100                 105                 110

cgg gcc aac acc gtg agg agc ttc cac cac gaa gaa cat ctg gag aac 384
Arg Ala Asn Thr Val Arg Ser Phe His His Glu Glu His Leu Glu Asn
        115                 120                 125

atc cca ggg acc agt gaa aac tct gct ttt cgt ttc ctc ttt aac ctc 432
Ile Pro Gly Thr Ser Glu Asn Ser Ala Phe Arg Phe Leu Phe Asn Leu
    130                 135                 140

agc agc atc cct gag aac gag gcg atc tcc tct gca gag ctt cgg ctc 480
Ser Ser Ile Pro Glu Asn Glu Ala Ile Ser Ser Ala Glu Leu Arg Leu
145                 150                 155                 160

ttc cgg gag cag gtg gac cag ggc cct gat tgg gaa agg ggc ttc cac 528

Phe Arg Glu Gln Val Asp Gln Gly Pro Asp Trp Glu Arg Gly Phe His
                165                 170                 175

cgt ata aac att tat gag gtt atg aag ccc cca gca gaa gtg gtg cct 576
Arg Ile Asn Ile Tyr Glu Val Met Lys Pro Pro Ala Glu Val Val Pro
            180                 185                 190

ggg cac ctc atc aca cga cta ctg gac acg aga ctg gtc cac cac aat 624
Gly His Leu Ile Thr Arg Leu Leu Asp Thr Arg Leu Val His His Asn
        195                 200                 205

gtg aca cgg tgg gaa act ttt gat gtg agc cct gcg gtc ctt cgc tgg 672
Val Thr Arg Trp Glu Thr Phe Asp Val Ser Pro Ala Val Leu Arg Trp
    210                 215                 220

acc cgg gag aag cag cca aac tat ggg cta gcc att gag gtg act cac 720
Thr Arg Glu Lys Gln Pro Asn Tyr Gly Leu Ala Ile Glu Val Thr His
225                 230                 235                 240

ctc cat cag act cgg acc cac cag ggc cag cat gtc agg att agc cga 768
Leu His Gln Thr Arg Thr His Gln Gly Gln His Val Arg Ile Ser Arg
                245                 250                 255

tcg tta cct caa ggg agt ggg aat tgg gcc cag ctc cgg ccc ctc ctg 816
Ser Leu Pro Gln Gly Ser Gly Asn Trp Ala Gln Leu Arg Pro Leu Leu
            260                 265                 270

gtc acc ttt ggc cat gat ggc cgg ggc cat gcc ttg acc cga cgc cgg 864
Val Thr Phe Gly His Asp Gly Arg Gly His Ala Leu Thr Arg Arg Arg
        275                 280                 285

agg gcc aag cgt agc cct aag cat cac tca cag cgg gcc agg aag aag 912
Arg Ala Lys Arg Ser Pro Lys His His Ser Gln Arg Ala Arg Lys Lys
    290                 295                 300

aat aag aac tgc cgg cgc cac tcg ctc tat gtg gac ttc agc gat gtg 960

Asn Lys Asn Cys Arg Arg His Ser Leu Tyr Val Asp Phe Ser Asp Val
305                 310                 315                 320

ggc tgg aat gac tgg att gtg gcc cca cca ggc tac cag gcc ttc tac     1008
Gly Trp Asn Asp Trp Ile Val Ala Pro Pro Gly Tyr Gln Ala Phe Tyr
                325                 330                 335

tgc cat ggg gac tgc ccc ttt cca ctg gct gac cac ctc aac tca acc     1056
Cys His Gly Asp Cys Pro Phe Pro Leu Ala Asp His Leu Asn Ser Thr
            340                 345                 350

aac cat gcc att gtg cag acc ctg gtc aat tct gtc aat tcc agt atc     1104
Asn His Ala Ile Val Gln Thr Leu Val Asn Ser Val Asn Ser Ser Ile
        355                 360                 365

ccc aaa gcc tgt tgt gtg ccc act gaa ctg agt gcc atc tcc atg ctg     1152
Pro Lys Ala Cys Cys Val Pro Thr Glu Leu Ser Ala Ile Ser Met Leu
    370                 375                 380

tac ctg gat gag tat gat aag gtg gta ctg aaa aat tat cag gag atg     1200
Tyr Leu Asp Glu Tyr Asp Lys Val Val Leu Lys Asn Tyr Gln Glu Met
385                 390                 395                 400

gta gta gag gga tgt ggg tgc cgc                                     1224
Val Val Glu Gly Cys Gly Cys Arg
                405
<210> 71
<211> 24
<212> DNA
<213> Artificial Sequence
<400> 71
gcccgcgctc caactgctct gatg                                          24

<210> 72
<211> 24
<212> DNA

<213> Artificial Sequence
<400> 72
tgcctacggt ggtgcgccct ctgc                                                24

<210> 73
<211> 22
<212> DNA
<213> Artificial Sequence
<400> 73
gaagcgcaac agggccatca cg                                                  22

<210> 74
<211> 22
<212> DNA
<213> Artificial Sequence
<400> 74
ccacgtcacg caggtcccgt tc                                                  22

<210> 75
<211> 22
<212> DNA
<213> Artificial Sequence
<400> 75
gatcctgttc tctgcctctg ga                                                  22

<210> 76
<211> 22
<212> DNA
<213> Artificial Sequence
<400> 76
tcatccactt tgtccacccg ag                                                  22

<210> 77
<211> 21
<212> DNA
<213> Artificial Sequence

<400> 77
ttcctcgtct tggccttttg g                                                21

<210> 78
<211> 21
<212> DNA
<213> Artificial Sequence
<400> 78
gctggatctt cgtaggctcc g                                                21

<210> 79
<211> 19
<212> DNA
<213> Artificial Sequence
<400> 79
ggcaagctga ccctgaagt                                                   19

<210> 80
<211> 19
<212> DNA
<213> Artificial Sequence
<400> 80
gggtgctcag gtagtggtt                                                   19

## Description

### TECHNICAL FIELD

The present invention relates to methods for isolation, purification, expansion, and differentiation of cells having the potential to differentiate into cardiomyocytes. Furthermore, the present invention relates to methods for proliferating cells having the potential to differentiate into cardiomyocytes and for regulating their differentiation into cardiomyocytes using various cytokines and transcription factors. Moreover, the present invention relates to a method for obtaining surface antigens specific for cells having the potential to differentiate into cardiomyocytes, a method for obtaining genes encoding the surface antigens, a method for obtaining antibodies specific for the surface antigens, and a method for obtaining a protein and a gene controlling the proliferation of cells having the potential to differentiate into cardiomyocytes and their differentiation into cardiomyocytes. Also, the present invention relates to therapeutic agents for various heart diseases containing cells having the potential to differentiate into cardiomyocytes.

### BACKGROUND ART

Cardiomyocytes actively divide into daughter cells with spontaneous beating before birth. However, they lose the proliferative activity after birth and never acquire the division potentiality again unlike hepatocytes. Furthermore, unlike skeletal muscles, they does not have undifferentiated precursor cells such as satellite cells. Therefore, when cardiomyocytes are necrotized by myocardial infarction, myocarditis, senility etc., hypertrophy of the remaining cardiomyocytes occurs *in vivo* instead of cell division. Cardiac hypertrophy is a physiological adaptation at the initial stage, but when coupled with the fibrosis of stroma by the growth of cardiac fibroblasts, it comes to lower the diastolic function and the systolic function of heart itself, leading to heart failure. Therapy so far developed for heart failure caused by myocardial infarction, etc. is mainly symptomatic therapy, for example, intensification of the cardiac systolic function, alleviation of the pressure overload and the volume load on heart using a vasodilator drug, and decrease of blood flow using of a diuretic. On the other hand, heart transplantation is alternative therapy for severe heart failure, but is not generally adopted as a common treatment because of problems such as shortage of heart donors, difficulty in judging cerebral death, immune rejection and a great rise in medical cost. At present, heart diseases are the third cause of mortality in Japan *(Annual Report on Health and Welfare,* 1998), and thus success in regeneration of lost cardiomyocytes will lead to a great advance in medical welfare.

As a cell line retaining the characteristics of cardiomyocytes, AT-1 cell line has been obtained from the atrial tumor of the transgenic mouse expressing SV40 promoter large T antigen under the control of atrial natriuretic hormone promoter (*Science, 239*: 1029-1038 (1988)). However, this cell line forms tumors when transplanted *in vivo* and thus is inappropriate for cell transplantation. Under these circumstances, the following methods have been proposed for reconstructing myocardium.

The first method is conversion of cells other than cardiomyocytes into cardiomyocytes, which has been proposed on the analogy of the conversion of fibroblasts into skeletal muscle cells by the introduction of MyoD. Although a successful result has been reported with P19 cell which is a murine embryonal carcinoma cell (*Cell Struc. & Func., 21*: 101-110 (1996)), there has been no report on success with non-carcinomatous cells.

The second method is restoration of proliferative activity to cardiomyocytes, which is based on the fact that beating cardiomyocytes can proliferate in the fetus. No successful example of this method has been reported yet.

The third method is induction of cardiomyocytes from undifferentiated stem cells. It has already been demonstrated that cardiomyocytes can be differentiated from embryonic stem cells (ES cells), but there still remain the problems of carcinoma formation and immune rejection by embryonic stem cells transplanted into an adult tissue. (*Nature Biotechnology, 17:* 139-142 (1999)).

In order to practically utilize embryonic stem cells in medical treatments, it is essential to develop a technique for highly purifying at least cardiomyocyte precursor cells or cardiomyocytes. As for the problem of immune rejection, the possibility of solving the problem by the cloning technique has been suggested, but it is difficult to apply this technique to general medical treatments because of its complicated operation.

It has also been proposed to transplant undifferentiated cardiomyocyte precursor cells obtained from an aborted fetus, and it is known that such cells effectively function as cardiomyocytes in an experiment using animals (*Science*, *264*: 98-101 (1994)). However, it is difficult to obtain a large amount of cardiomyocyte precursor cells in this method, and the method is hardly applicable to general medical treatments also from an ethical viewpoint.

It is known that there exist mesenchymal stem cells besides hematopoietic stem cells and vascular stem cells in adult bone marrow and that mesenchymal stem cells can be induced to differentiate into osteocytes, chondrocytes, tendon cells, ligament cells, skeletal muscle cells, adipocytes, stromal cells and hepatic oval cells (*Science, 284*: 143-147 (1999); *Science, 284*: 1168-1170 (1999)). On the other hand, it has been recently reported that the cells obtained from the bone marrow of an adult mouse can be induced to differentiate into cardiomyocytes *(J. Clinical Investigation, 103*: 10-18 (1999)). This report suggests that the cell therapy which comprises transplanting cells which are obtained from bone marrow fluid taken from a patient followed by in vitro expansion and drug treatment to the damaged part of the patient's heart can be a practical medical treatment (*J. Clinical Investigation, 103*: 591-592 (1999)). However, this report merely indicates that a part of the immortalized cells established from the bone marrow of an adult mouse can differentiate into cardiomyocytes. Furthermore, the report fails to isolate, selectively proliferate, and efficiently differentiate the adult bone marrow cells having the potential to differentiate into cardiomyocytes (*J. Clinical Investigation, 103*: 591-592 (1999)).

Antibodies which recognize various surface antigens are used to isolate the target cells from the tissue of vital body. For example, it is known that immature hematopoietic stem cells have the characteristics of CD34+/CD38-/HLA-DR-/CD90 (Thy-1)+, and CD38 is expressed while CD90(Thy-1) disappears in the process of differentiation (*Protein, Nucleic Acid, Enzyme, 45*: 13, 2056-2062 (2000)). In vascular endothelial cells, markers such as CD34, CD31, Flk-1, Tie-2, E-selectin, etc. are expressed (Molecular Cardiovascular Disease, 1(3): 294-302 (2000)). In bone marrow mesenchymal stem cells, markers such as CD90, CD105, CD140, etc. are expressed (*Science, 284*: 143-147 (1999); *Science, 284*: 1168-1170 (1999)). However, no surface marker of stem cells capable of inducing both myocardium and vascular endothelial cells is known.

### DISCLOSURE OF THE INVENTION

Under the circumstances, a need exists for the development of therapy for heart diseases which therapy is safer and more established than those currently available. It is useful to select cells having the potential to differentiate into cardiomyocytes from a vital tissue such as bone marrow cells or the like or umbilical blood and to control the growth or differentiation of the cells for the development of myocardium-regenerating therapy using vital cells such as bone marrow-derived cells or the like or umbilical blood. For this purpose, it is necessary to separate the cells having the potential to differentiate into cardiomyocytes and to identify cytokines or transcription factors participating in the growth or differentiation of such cells.

The present inventors have made intensive studies aiming at solving the above problems and have obtained the following results. Specifically, various cell lines were obtained by separating mouse bone marrow-derived cells to single cell level. Then, various cell lines have characterized by their potential to differentiate into cardiomyocytes by treating each cell line with 5-azacytidine. Next, by labeling the thus obtained cell line using a retrovirus vector which expresses a GFP (green fluorescent protein) and tracing the cells using a fluorescence microscope, it has been found that the bone marrow-derived cells are pluripotent stem cells which can differentiate into at least two different cells, i.e., cardiomyocytes and adipocytes. Furthermore, it has been found that the stem cells can be differentiated into cardiomyocytes, adipocytes and skeletal muscle cells stochastically by addition of not only 5-azacytidine but also other genomic DNA-demethylating agents, such as DMSO (dimethyl sulfoxide), indicating that demethylation of genomic DNA is effective in inducing the differentiation of bone marrow-derived cells into cardiomyocytes. Moreover, it was found that the expression of myocardium-specific genes, ANP (atrral natriuretic peptide) and cTnI (cardiac Troponin I), can be expressed in the bone marrow-derived cells by adding at least one cytokine of four cytokines, FGF-8, ET1, midkine and BMP4, combined with 5-azacytidine. Also, it was found that differentiation of the bone marrow-derived cells into cardiomyocytes can be promoted about 50-fold by the forced expression of two transcriptional factors, Nkx2.5 and GATA4, in these bone marrow-derived cells using virus vectors followed by 5-azacytidine treatment. Furthermore, it was found that the expression of ANP and cTnI, which are myocardium-specific genes, in the bone marrow-derived cells can be specifically promoted by culturing these bone marrow-derived cells in a culture dish coated with a cardiomyocyte-derived extracellular substrate. Moreover, it was found that the formation of myocardium from the bone marrow-derived cells can be about 10 times or more promoted by co-culturing the bone marrow-derived cells together with primarily cultured cells derived from myocardium. Moreover, it was found that differentiation of the bone marrow-derived cells into cardiomyocytes can be promoted about 500-fold when the forced expression of two transcription factors Nkx2.5 and GATA4 in the bone marrow-derived cells using virus vectors and co-culturing these cells with cardiomyocytes were combined.

Subsequently, the differentiation potency of the bone marrow-derived cells was examined by a transplantation experiment. First, the bone marrow-derived cells were transplanted into an adult mouse heart and it was thus found that these bone marrow-derived cells were differentiated into myocardia and vessels. Furthermore, the bone marrow-derived cells were transplanted into an adult mouse muscle and it was thus found that these bone marrow-derived cells could form skeletal muscles. When the bone marrow-derived cells were transplanted into a mouse blastocyst, tissues derived from these transplanted cells were formed in the central nervous system, liver and heart of the mouse. The central nervous system, liver and heart are tissues of the ectoderm, endoderm and mesoderm, respectively.

These results indicate that the bone marrow-derived cells found in the present invention have properties different from those possessed by hematopoietic stem cells which are differentiated into only hematopoietic stem tissue present in bone marrow and from those possessed by mesenchymal stem cell which are differentiated into only dorsal mesoderm tissue such as skeletal muscle, adipocytes, bone and the like known in the art, that is, a totipotency of differentiating into all of the three germ layers including the ectoderm, mesoderm and endoderm.

Furthermore, the inventors analyzed the expression of surface antigens of bone marrow-derived cells using antibodies which recognize hematopoietic cell surface antigens, CD34, CD117, CD14, CD45, CD90, Sca-1, Ly6c and Ly6g, antibodies which recognize vascular endothelial cell surface antigens, Flk-1, CD31, CD105 and CD144, antibodies which recognize a mesenchymal cell surface antigen, CD140, antibodies which recognize integrin surface antigens, CD49b, CD49d, CD29 and CD41, and antibodies which recognize matrix receptors, CD54, CD102, CD106 and CD44, and the like in these bone marrow cells of the present invention and thus found that they are totipotential stem cells exhibiting a quite novel expression form having been unknown, thereby completing the present invention.

Specifically, the present invention provides the following (1)-(91):
(1) A cell which has been isolated from a living tissue or umbilical blood, and which has the potential to differentiate into at least a cardiomyocyte.
(2) The cell according to (1), wherein the living tissue is bone marrow.
(3) The cell according to (1) or (2), wherein the cell is a multipotential stem cell.
(4) The cell according to any one of (1) to (3), wherein the cell is a multipotential stem cell which differentiates into at least a cardiomyocyte and a vascular endothelial cell.
(5) The cell according to any one of (1) to (4), wherein the cell is a multipotential stem cell which differentiates into at least a cardiomyocyte, an adipocyte, a skeletal muscle cell, an osteoblast, and a vascular endothelial cell.
(6) The cell according to any one of (1) to (5), wherein the cell is a multipotential stem cell which differentiates into at least a cardiomyocyte, an adipocyte, a skeletal muscle cell, an osteoblast, a vascular endothelial cell, a nervous cell, and a hepatic cell.
(7) The cell according to any one of (1) to (3), wherein the cell is a multipotential stem cell which differentiates into any cell in adult tissues.
(8) The cell according to any one of (1) to (7), wherein the cell is CD117-positive and CD140-positive.
(9) The cell according to (8), wherein the cell is further CD34-positive.
(10) The cell according to (9), wherein the cell is further CD144-positive.
(11) The cell according to (9), wherein the cell is further CD140-negative.
(12) The cell according to (8), wherein the cell is CD34-negative.
(13) The cell according to (12), wherein the cell is further CD144-positive.
(14) The cell according to (12), wherein the cell is further CD144-negative.
(15) The cell according to (10), wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.
(16) The cell according to (11), wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.
(17) The cell according to (12), wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.
(18) The cell according to (13), wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.
(19) The cell according to (1), which does not take up Hoechst 33342.
(20) A cardiomyocyte precursor which differentiates into only cardiomyocyte induced from the cell according to any one of (1) to (19).
(21) The cell according to any one of (1) to (20), which has the potential to differentiate into a ventricular cardiac muscle cell.
(22) The cell according to any one of (1) to (20), which has the potential to differentiate into a sinus node cell.
(23) The cell according to any one of (1) to (20), wherein the vital tissue or umbilical blood is derived from a mammal.
(24) The cell according to (23), wherein the mammal is selected from the group consisting of a mouse, a rat, a guinea pig, a hamster, a rabbit, a cat, a dog, a sheep, a swine, cattle, a goat and a human.
(25) The cell according to any one of (1) to (8), which is mouse bone marrow-derived multipotential stem cell BMSC (FERM BP-7043).
(26) The cell according to any one of (1) to (25), which has the potential to differentiate into a cardiomyocyte by demethylation of a chromosomal DNA of the cell.
(27) The cell according to (26), wherein the demethylation is carried out by at least one selected from the group consisting of demethylase, 5-azacytidine, and dimethyl sulfoxide, DMSO.
(28) The cell according to (27), wherein the demethylase comprises the amino acid sequence represented by SEQ ID NO:1.
(29) The cell according to any one of (1) to (28), wherein the differentiation is accelerated by a factor which is expressed in a cardiogenesis region of a fetus or a factor which acts on differentiation into a cardiomyocyte in a cardiogenesis stage of a fetus.
(30) The cell according to (29), wherein the factor which is expressed in a cardiogenesis region of a fetus or the factor which acts on differentiation into a cardiomyocyte in a cardiogenesis stage of a fetus is at least one selected from the group consisting of a cytokine, an adhesion molecule, a vitamin, a transcription factor, and an extracellular matrix.
(31) The cell according to (30), wherein the cytokine is at least one selected from the group consisting of a platelet-derived growth factor, PDGF; a fibroblast growth factor-8, FGF-8; an endothelin 1, ET1; a midkine; and a bone morphogenetic factor, BMP-4.
(32) The cell according to (31), wherein the PDGF, FGF-8, ET1, midkine, and BMP-4 comprise the amino acid sequence represented by SEQ ID NO:3 or 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively.
(33) The cell according to (30), wherein the adhesion molecule is at least one selected from the group consisting of a gelatin, a laminin, a collagen, and a fibronectin.
(34) The cell according to (30), wherein the vitamin is retinoic acid.
(35) The cell according to (30), wherein the transcription factor is at least one selected from the group consisting of Nkx2)5/Csx, GATA4, MEF-2A, MEF-2B, MEF-2C, MEF-2D, dHAND, eHAND, TEF-1, TEF-3, TEF-5, and MesP1.
(36) The cell according to (35), wherein the Nkx2)5/Csx, GATA4, MEF-2A, MEF-2B, MEF-2C, MEF-2D, dHAND, eHAND, TEF-1, TEF-3, TEF-5, and MesP1 comprise the amino acid sequence represented by SEQ ID NO:9, the amino acid sequence represented by SEQ ID NO:11, the amino acid sequence represented by SEQ ID NO:13, the amino acid sequence represented by SEQ ID NO:15, the amino acid sequence represented by SEQ ID NO:17, the amino acid sequence represented by SEQ ID NO:19, the amino acid sequence represented by SEQ ID NO:21, the amino acid sequence represented by SEQ ID NO:23, the amino acid sequence represented by SEQ ID NO:25, the amino acid sequence represented by SEQ ID NO:27, the amino acid sequence represented by SEQ ID NO:29, and the amino acid sequence represented by SEQ ID NO:62, respectively.
(37) The cell according to (30), wherein the extracellular matrix is an extracellular matrix derived from a cardiomyocyte.
(38) The cell according to any one of (1) to (28), wherein the differentiation is inhibited by a fibroblast growth factor-2, FGF-2.
(39) The cell according to (38), wherein the FGF-2 comprises the amino acid sequence represented by SEQ ID NO:7 or 8.
(40) The cell according to any one of (1) to (28), which is capable of differentiating into a cardiomyocyte or a blood vessel by transplantation into a heart.
(41) The cell according to any one of (1) to (28), which is capable of differentiating into a cardiac muscle by transplantation into a blastocyst or by co-culturing with a cardiomyocyte.
(42) The cell according to any one of (1) to (28), which is capable of differentiating into an adipocyte by an activator of a nuclear receptor, PPAR-γ.
(43) The cell according to (42), wherein the activator is a compound having a thiazolidione skeleton.
(44) The cell according to (43), wherein the compound is at least one selected from the group consisting of troglitazone, pioglitazone, and rosiglitazone.
(45) The cell according to any one of (1) to (28), which is capable of differentiating into a nervous cell by transplantation into a blastocyst or by transplantation into an encephalon or a spinal cord.
(46) The cell according to any one of (1) to (28), which is capable of differentiating into a hepatic cell by transplantation into a blastocyst or by transplantation into a liver.
(47) A method for differentiting the cell according to any one of (1) to (28) into a cardiac muscle, comprising using a chromosomal DNA-dimethylating agent.
(48) A method for redifferentiating the cell according to (9) into the cell according to (12), comprising using a chromosomal DNA-dimethylating agent.
(49) A method for redifferentiating a cell which is CD117-negative and CD140-positive into the cell according to (8), comprising using a chromosomal DNA-dimethylating agent.
(50) The method according to (48) or (49), wherein the chromosomal DNA-dimethylating agent is selected from the group consisting of a demethylase, 5-azacytidine, and DMSO.
(51) The method according to (50), wherein the demethylase comprises the amino acid sequence represented by SEQ ID NO:1.
(52) A method for differentiating the cell according to any one of (1) to (28) into a cardiac muscle, comprising using a factor which is expressed in a cardiogenesis region of a fetus or a factor which acts on differentiation into a cardiomyocyte in a cardiogenesis stage of a fetus.
(53) The method according to (52), wherein the factor which is expressed in a cardiogenesis region of a fetus or the factor which acts on differentiation into a cardiomyocyte in a cardiogenesis stage of a fetus is at least one selected from the group consisting of a cytokine, an adhesion molecule, a vitamin, a transcription factor, and an extracellular matrix.
(54) The method according to (53), wherein the cytokine is at least one selected from the group consisting of a platelet-derived growth factor, PDGF; a fibroblast growth factor-8, FGF-8; an endothelin 1, ET1; a midkine; and a bone morphogenetic factor, BMP-4.
(55) The method according to (54), wherein the PDGF, FGF-8, ET1, midkine, and BMP-4 comprise the amino acid sequence represented by SEQ ID NO:3 or 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively.
(56) The method according to (53), wherein the adhesion molecule is at least one selected from the group consisting of a gelatin, a laminin, a collagen, and a fibronectin.
(57) The method according to (53), wherein the vitamin is retinoic acid.
(58) The method according to (53), wherein the transcription factor is at least one selected from the group consisting of Nkx2)5/Csx, GATA4, MEF-2A, MEF-2B, MEF-2C, MEF-2D, dHAND, eHAND, TEF-1, TEF-3, TEF-5, and MesP1
(59) The method according to (58), wherein the Nkx2)5/Csx, GATA4, MEF-2A, MEF-2B, MEF-2C, MEF-2D, dHAND, eHAND, TEF-1, TEF-3, TEF-5, and MesP1 comprise the amino acid sequence represented by SEQ ID NO:9, the amino acid sequence represented by SEQ ID NO:11, the amino acid sequence represented by SEQ ID NO:13, the amino acid sequence represented by SEQ ID NO:15, the amino acid sequence represented by SEQ ID NO:17, the amino acid sequence represented by SEQ ID NO:19, the amino acid sequence represented by SEQ ID NO:21, the amino acid sequence represented by SEQ ID NO:23, the amino acid sequence represented by SEQ ID NO:25, the amino acid sequence represented by SEQ ID NO:27, the amino acid sequence represented by SEQ ID NO:29, the amino acid sequence represented by SEQ ID NO:62, respectively.
(60) The method according to (53), wherein the extracellular matrix is an extracellular matrix derived from a cardiomyocyte.
(61) A method for differentiating the cell according to any one of (1) to (28) into an adipocyte, comprising using an activator of a nuclear receptor, PPAR-γ.
(62) The method according to (61), wherein the activator is a compound having a thiazolidione skeleton.
(63) The method according to (62), wherein the compound is at least one selected from the group consisting of troglitazone, pioglitazone, and rosiglitazone.
(64) A myocardium-forming agent, comprising, as an active ingredient, a chromosomal DNA-demethylating agent.
(65) The myocardium-forming agent according to (64), wherein the chromosomal DNA-demethylating agent is at least one selected from the group consisting of a demethylase, 5-azacytidine, and DMSO.
(66) The myocardium-forming agent according to (65), wherein the demethylase comprises the amino acid sequence represented by SEQ ID NO:1.
(67) A myocardium-forming agent, comprising, as an active ingredient, a factor which is expressed in a cardiogenesis region of a fetus or a factor which acts on differentiation into a cardiomyocyte in a cardiogenesis stage of a fetus.
(68) The myocardium-forming agent according to (67), wherein the factor which is expressed in a cardiogenesis region of a fetus or the factor which acts on differentiation into a cardiomyocyte in a cardiogenesis stage of a fetus is at least one selected from the group consisting of a cytokine, an adhesion molecule, a vitamin, a transcription factor, and an extracellular matrix.
(69) The myocardium-forming agent according to (68), wherein the cytokine is at least one selected from the group consisting of a platelet-derived growth factor, PDGF; a fibroblast growth factor-8, FGF-8; an endothelin 1, ET1; a midkine; and a bone morphogenetic factor, BMP-4.
(70) The myocardium-forming agent according to (69), wherein the PDGF, FGF-8, ET1, midkine, and BMP-4 comprise the amino acid sequence represented by SEQ ID NO:3 or 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively.
(71) The myocardium-forming agent according to (68), wherein the adhesion molecule is selected from the group consisting of a gelatin, a laminin, a collagen, and a fibronectin.
(72) The myocardium-forming agent according to (71), wherein the vitamin is retinoic acid.
(73) The myocardium-forming agent according to (68), wherein the transcription factor is at least one selected from the group consisting of Nkx2)5/Csx, GATA4, MEF-2A, MEF-2B, MEF-2C, MEF-2D, dHAND, eHAND, TEF-1, TEF-3, TEF-5, and MesP1.
(74) The myocardium-forming agent according to (73),
   wherein the Nkx2)5/Csx, GATA4, MEF-2A, MEF-2B, MEF-2C, MEF-2D, dHAND, eHAND, TEF-1, TEF-3, TEF-5, and MesP1 comprise the amino acid sequence represented by SEQ ID NO:9, the amino acid sequence represented by SEQ ID NO:11, the amino acid sequence represented by SEQ ID NO:13, the amino acid sequence represented by SEQ ID NO:15, the amino acid sequence represented by SEQ ID NO:17, the amino acid sequence represented by SEQ ID NO:19, the amino acid sequence represented by SEQ ID NO:21, the amino acid sequence represented by SEQ ID NO:23, the amino acid sequence represented by SEQ ID NO:25, the amino acid sequence represented by SEQ ID NO:27, the amino acid sequence represented by SEQ ID NO.29, and the amino acid sequence represented by SEQ ID NO:62, respectively.
(75) The myocardium-forming agent according to (68), wherein the extracellular matrix is an extracellular matrix derived from a cardiomyocyte.
(76) A method for regenerating a heart damaged by a heart disease, comprising using the cell according to any one of (1) to (46).
(77) An agent for cardiac regeneration, comprising, as an active ingredient, the cell according to any one of (1) to (46).
(78) A method for specifically transfecting a wild-type gene corresponding to a mutant gene in a congenital genetic disease to a myocardium, comprising using the cell according to any one of (1) to (46) into which the wild-type gene corresponding to a mutant gene in a congenital genetic disease of a heart has been introduced.
(79) A therapeutic agent for a heart disease, comprising, as an active ingredient, the cell according to any one of (1) to (46) into which a wild-type gene corresponding to a mutant gene in a congenital genetic disease of a heart has been introduced.
(80) A method for producing an antibody which specifically recognizes the cell according to any one of (1) to (46), comprising using the cell as an antigen.
(81) A method for isolating a cell having the potential to differentiate into a cardiomyocyte according to any one of (1) to (46), comprising using an antibody obtained by the method according to (80).
(82) A method for obtaining a surface antigen specific for the cell according to any one of (1) to (46), comprising using the cell.
(83) A method for screening a factor which proliferates the cell according to any one of (1) to (46), comprising using the cell.
(84) A method for screening a factor which induces the cell according to any one of (1) to (46) to differentiate into a cardiomyocyte, comprising using the cell.
(85) A method for screening a factor which immortalizes the cell according to any one of (1) to (46), comprising using the cell.
(86) A method for immortalizing the cell according to any one of (1) to (46), comprising expressing a telomerase in the cell.
(87) The method according to (86), wherein the telomerase comprises the amino acid sequence represented by SEQ ID NO:31.
(88) A therapeutic agent for a heart disease, comprising, as an active ingredient, the cell according to any one of (1) to (46) which has been immortalized by expressing a telomerase.
(89) The therapeutic agent according to (88), wherein the telomerase comprises the amino acid sequence represented by SEQ ID NO:31.
(90) A culture supernatant comprising the cell according to any one of (1) to (46).
(91) A method for inducing the cell according to any one of (1) to (46) to differentiate into a cardiomyocyte, comprising using the culture supernatant according to (90).

The cells having the potential to differentiate into cardiomyocytes according to the present invention can be isolated from adult tissues such as bone marrow, muscle, brain, pancreas, liver and kidney or umbilical blood, and preferred examples include bone marrow and umbilical blood.

Any cell can be used as the pluripotent stem of the present invention, so long as it has the potential to differentiate into cardiomyocytes and other cells. Preferable examples thereof include cells having the potential to differentiate into at least cardiomyocytes, adipocytes, skeletal muscle cells and osteoblasts; cells having the potential to differentiate into at least cardiomyocyte and vascular endothelial cells; cells having the potential to differentiate into at least cardiomyocytes, adipocytes, skeletal muscle cells, osteoblasts and vascular endothelial cells; and cells having the potential to differentiate into at least cardiomyocytes, adipocytes, skeletal muscle cells, vascular endothelial cells, osteoblasts, neural cells and hepatocytes.

Also, even if cells originally have the potential to differentiate into adipocytes, skeletal muscle cells and osteoblasts but do not have the potential to differentiate into cardiomyocytes, those cells to which the potential to differentiate into cardiomyocytes can be added by the following induction method or the like, are included in the invention.

The cells of the present invention having the potential to differentiate into cardiomyocytes include cells which are CD117-positive and CD140-positive. The cells which are CD117-positive and CD140-positive preferably cells which are CD34-positive, CD117-positive and CD140-positive, and cells which are CD34-negative, CD117-positive and CD140-positive; more preferably cells which are CD144-positive, CD34-positive, CD117-positive and CD140-positive, cell which are CD144-negative, CD34-positive, CD117-positive and CD140-positive, cells which are CD144-positive, CD34-negative, CD117-positive and CD140-positive, and cells which are CD144-negative, CD34-negative, CD117-positive and CD140-positive; still more preferably cells which are CD34-positive, CD117-positive, CD14-negative, CD45-negative, CD90-negative, Flk-1-negative, CD31-negative, CD105-negative, CD144-positive, CD140-positive, CD49b-negative, CD49d-negative, CD29-positive, CD54-negative, CD102-negative, CD106-negative and CD44-positive, cells which are CD34-positive, CD117-positive, CD14-negative, CD45-negative, CD90-negative, Flk-1-negative, CD31-negative, CD105-negative, CD144-negative, CD140-positive, CD49b-negative, CD49d-negative, CD29-positive, CD54-negative, CD102-negative, CD106-negative and CD44-positive, cells which are CD34-negative, CD1l7-positive CD14-negative, CD45-negative, CD90-negative, Flk-1-negative, CD31-negative, CD105-negative, CD144-positive, CD140-positive, CD49b-negative, CD49d-negative, CD29-positive, CD54-negative, CD102-negative, CD106-negative and CD44-positive, and cells which are CD34-positive, CD117-positive, CD14-negative, CD45-negative, CD90-negative, Flk-1-negative, CD31-negative, CD105-negative, CD144-negative, CD140-positive, CD49b-negative, CD49d-negative, CD29-positive, CD54-negative, CD102-negative, CD106-negative and CD44-positive. The cells which are CD1l7-positive and CD140-positive include mouse marrow multipotential stem cells, BMSC. Mouse bone marrow-derived pluripotent stem cells (BMSC) have been deposited on February 22, 2000, in National Institute of Bioscience and Human Technology, Agency of Industrial Science and Technology (Higashi 1-1-3, Tsukuba-shi, Ibaraki, Japan) as FERM BP-7043.

Examples of the cells which originally have the potential to differentiate into adipocytes, skeletal muscle cells and osteoblasts but do not have the potential to differentiate into cardiomyocytes, to which the potential to differentiate into heart muscle cells can be added by the following induction method or the like include cells which are CD117-negative and CD140-positive, preferably cells which are CD144-negative, CD34-negative, CD117-negative and CD140-positive, more preferably cells which are CD34-negative, CD117-negative, CD14-positive, CD45-negative, CD90-negative, Flk-1-negative, CD31-negative, CD105-negative, CD144-negative, CD140-positive, CD49b-positive, CD49d-negative, CD29-positive, CD54-positive, CD102-negative, CD106-positive and CD44-positive. KUM2 cells can be exemplified as the cells which are CD34-negative, CD117-negative CD14-positive, CD45-negative, CD90-negative, Flk-1-negative, CD31-negative, CD105-negative, CD144-negative, CD140-positive, CD49b-positive, CD49d-negative, CD29-positive, CD54-positive, CD102-negative, CD106-positive and CD44-positive.

As the species of the vital tissue or umbilical blood used in the invention, vertebrate animals, preferably warm blooded animals, and more preferably mammals such as mouse, rat, guinea pig, hamster, rabbit, cat, dog, sheep, pig, cattle, goat, monkey and human are used. Those derived from a human is preferred for human therapeutic use.

Any adult tissue or umbilical blood can be used, so long as it is derived from the above animal . In therapeutic use for the human body, it is preferred to employ those derived from humans.

Myocardial cells can be obtained by isolating cells having the potential to differentiate into cardiomyocytes from an adult tissue or umbilical blood of a mammal, such as mouse, rat or human, culturing these cells and then inducing the differentiation of cells having the potential to differentiate into cardiomyocytes.

The differentiation into not only cardiomyocytes but also vascular endothelial cells, smooth muscles, skeletal muscle cells, adipocytes, bones, cartilages, pancreatic endocrine cells, pancreatic exocrine cells, hepatocytes, glomerular cells, renal tubular cells, neurons, glial cells, oligodendrocytes, etc. can be induced using the pluripotent stem cell to obtain various cells.

Now, the present invention will be described in greater detail.

### 1. Isolation of cells having the potential to differentiate into cardiomyocytes

The cells having the potential to differentiate into cardiomyocytes according to the present invention can be isolated from any tissue (for example, an adult tissue, umbilical blood), so long as cells having the potential to differentiate into cardiomyocytes can be obtained. Next, a method for isolating cells having the potential to differentiate into cardiomyocytes from bone marrow will be illustrated.

### (1) Method for isolating bone marrow cells having the potential to differentiate into cardiomyocytes

The method for obtaining human cells having the potential to differentiate into cardiomyocytes from bone marrow is not particularly limited, so long as it is a safe and efficient method. For example, the method described in S. E. Haynesworth, *et al., Bone, 13*: 81 (1992) can be employed.

Bone marrow puncture is conducted by sternal or iliac puncture. After skin disinfection of the part for puncture, a donor is subjected to local anesthesia. Particularly, subpeiosteum is thoroughly anesthetized. The inner tube of a bone marrow puncture needle is pulled out and a 10 ml syringe containing 5000 units of heparin is attached to the needle. A required amount, normally 10-20 ml, of the bone marrow fluid is quickly taken by suction and the puncture needle is removed, followed by pressure hemostasis for about 10 minutes. The obtained bone marrow fluid is centrifuged at 1000 x g to recover bone marrow cells, which are then washed with PBS (phosphate buffered saline). After this centrifugation step is repeated twice, the obtained bone marrow cells are suspended in a cell culture medium such as α-MEM (α-modification of MEM), DMEM (Dulbecco's modified MEM) or IMDM (Isocove's modified Dulbeccos's medium) each containing 10% FBS (fetal bovine serum) to prepare a bone marrow cell suspension.

For the isolation of the bone marrow cells having the potential to differentiate into cardiomyocytes from the obtained bone marrow cell suspension, any method can be employed, so long as it is effective for removing other cells existing in the cell suspension such as hematocytes, hematopoietic stem cells, vascular stem cells and fibroblasts. For example, based on the method described in M.F. Pittenger *et al., Science, 284*: 143 (1999), the desired cells can be isolated by subjecting the cell suspension layered over Percoll having the density of 1.073 g/ml to centrifugation at 1100 × g for 30 minutes, and the cells on the interface are recovered. Furthermore, a bone marrow cell mixture containing the cells having the potential to differentiate into cardiomyocytes can be obtained by mixing the above cell suspension with an equal amount of Percoll solution diluted to 9/10 with 10× PBS, followed by centrifugation at 20000 × g for 30 minutes, and recovering the fraction having the density of 1.075-1.060.

The thus obtained bone marrow cell mixture containing the bone marrow cells having the potential to differentiate into cardiomyocytes is diluted into single cell using 96-well culture plates to prepare a number of clones respectively derived from single cells. The clones having the potential to differentiate into cardiomyocyte can be selected by the observation of spontaneously beating cells generated by the treatment to induce cardiomyocytes from the cells having the potential to differentiate into cardiomyocytes described below.

Rat- or mouse-derived bone marrow cells having the potential to differentiate into cardiomyocytes can be obtained, for example, in the following manner. A rat or a mouse is sacrificed by cervical dislocation and thoroughly disinfected with 70% ethanol. After the skin on the femur and quadriceps femuris are excised, the femur is put out of the knee joint with scissors and the muscle on the back side of the femur is removed. Then, the femur is put out of the hip joint with scissors and taken out. After the muscle on the femur is removed with scissors as completely as possible, the femur is cut at both ends using scissors. A needle having a size appropriate for the thickness of the bone is attached to a 2.5 ml syringe containing about 1.5 ml of a cell culture medium such as α-MEM, DMEM or IMDM each containing 10% FBS followed by injecting into the pore of femur. The needle of the syringe is put into the femur from the cut end of the knee joint side and the culture medium is injected into bone marrow, whereby bone marrow cells are pressed out of the bone from the cut end of the hip joint side. The thus obtained bone marrow cells are suspended in a culture medium by pipetting. The bone marrow cells having the potential to differentiate into cardiomyocytes can be isolated from the resulting cell suspension in the same manner as in the above isolation of the human bone marrow cells.

### (2) Method for isolating cells having the potential to differentiate into cardiomyocytes from tissue other than bone marrow

According to the separation method using antibodies as described in 12 hereinafter, cells having the potential to differentiate into cardiomyocytes can be obtained form tissues other than bone marrow.

Preferred examples of the tissues other than bone marrow include umbilical blood. More specifically, it can be isolated in the following method.

First, umbilical blood is separated from the cord, followed by addition of heparin to give a final concentration of 500 units/ml. After thoroughly mixing, cells are separated from the umbilical blood by centrifugation and re-suspended in a cell culture medium, such as α-MEM (α-modified MEM), DMEM (Dulbecco's modified MEM) or IMDM (Isocove's modified Dulbecco's medium), each containing 10% FBS. From the cell suspension thus obtained, cells having the potential to differentiate into cardiomyocytes can be separated using the antibodies described below.

### 2. Methods for culturing the cells having the potential to differentiate into cardiomyocytes

The cells having the potential to differentiate into cardiomyocytes isolated by the methods described in the above 1 can be usually cultured using media of known compositions (*Technical Standard of Tissue Culture,* Third Edition, Asakura Shoten (1996)). Preferred media are cell culture media such as α-MEM, DMEM and IMDM supplemented with a serum such as 5-20% bovine serum. Culturing can be carried out under any conditions suitable for cell culture, but is preferably carried out at a temperature of 33-37°C in an incubator filled with 5-10% carbon dioxide gas. It is preferred to culture the cells having the potential to differentiate into cardiomyocytes in a plastic culture dish used for ordinary tissue culture so that the grown cells adhere to the dish. When cells become confluent on the dish, the medium is removed and a trypsin-EDTA solution is added to suspend the cells therein. The suspended cells may be washed with PBS or a medium for culturing the cells, diluted 5-20 times with the medium and then added to another culture dish for subculture.

### 3. Methods for inducing cardiomyocytes from cells having the potential to differentiate into cardiomyocytes

The methods for inducing cardiomyocytes from the cells having the potential to differentiate into cardiomyocytes include the following: (1) induction of differentiation by the treatment with a DNA-demethylating agent, (2) induction of differentiation using a factor which is expressed in the cardiogenesis region of a fetus or a factor which controls differentiation into cardiomyocytes in the cardiogenesis stage of a fetus, and (3) induction of differentiation using a culture supernatant of the cells having the potential to differentiate into cardiomyocytes or cardiomyocytes differentiated from the cells. Cardiomyocytes can be induced from the cells having the potential to differentiate into cardiomyocytes using such a method alone or in combination. Also, according to these methods, even mesenchymal cells which originally do not have the potential to differentiate into cardiomyocytes can be differentiated into cells having the potential to differentiate into cardiomyocytes, and cardiomyocytes can be induced.

Any DNA-demethylating agent can be used, so long as it is a compound which causes demethylation of DNA. Suitable DNA-demethylating agents include demethylase which is an enzyme which specifically removes the methylation of the cytosine residue in the GpC sequence in a chromosomal DNA, 5-azacytidine (hereinafter referred to as "5-aza-C") and DMSO (dimethyl sulfoxide). Examples of the demethylase enzymes include demethylase having the amino acid sequence represented by SEQ ID NO:1 (*Nature, 397*: 579-583 (1999)). Differentiation can be induced by the treatment with a DNA-demethylating agent, for example, in the following manner.

The cells having the potential to differentiate into cardiomyocytes are cultured in the presence of 3 µmol/l to 10 (µmol/1 of 5-aza-C for 24 hours. After 5-aza-C is removed by replacing the culture supernatant with a fresh medium, the cells are cultured for further 2-3 weeks to obtain cardiomyocytes. The cardiomyocytes produced by culturing for 2-3 weeks are mainly sinus node cells, but culturing for more than 4 weeks induces differentiation into ventricular cardiomyocytes.

Examples of the factors which are expressed in the cardiogenesis region of a fetus and the factors which act on differentiation into cardiomyocytes in the cardiogenesis stage of a fetus include cytokines, vitamins, adhesion molecules and transcription factors.

Any cytokine can be used, so long as it stimulates the cardiomyogenic differentiation of the cells having the potential to differentiate into cardiomyocytes in the cardiogenesis stage.

The examples include platelet-derived growth factor (hereinafter referred to as "PDGF"), fibroblast growth factor 8 (FGF8), endothelin 1 (ET1), midkine, and bone morphogenic protein 4 (BMP4). Preferred examples of the PDGF include PDGF A, PDGF B, PDGF C and the like, and specific examples include those the amino acid sequences represented by SEQ ID NOS:3 and 5. Preferred examples of the FGF8, ET1, midkine, BMP4 include the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively. The cytokine can be used, e.g., at a concentration of 10 to 40 ng/ml.

It is also possible to stimulate the cardiomyogenic differentiation of the cells having the potential to differentiate into cardiomyocytes into cardiomyocytes in the cardiogenesis stage using an inhibitor against a cytokine which suppresses the cardiomyogenic differentiation.

The cytokines which suppress the cardiomyogenic differentiation include fibroblast growth factor-2 (hereinafter referred to as "FGF-2"), specifically, FGF-2 having the amino acid sequence represented by SEQ ID NO:7 or 8.

The inhibitors against the cytokines which suppress the cardiomyogenic differentiation include substances which inhibit the signal transduction of the cytokines, such as antibodies and low molecular weight compounds which neutralize the cytokines activities.

Any vitamin can be used, so long as it stimulates the cardiomyogenic differentiation of the cells having the potential to differentiate into cardiomyocytes in the cardiogenesis stage. Retinoic acid can be used, e.g., at a concentration of 10⁻⁹ M.

Any adhesion molecule can be used, so long as it is expressed in the cardiogenesis region in the cardiogenesis stage. Examples include extracellular matrices such as gelatin, laminin, collagen, fibronectin and the like. For example, the cardiomyogenic differentiation of the cells having the potential to differentiate into cardiomyocytes can be stimulated by culturing the cells on a culture dish coated with fibronectin.

Examples of the transcription factors include a homeobox-type transcription factor, Nkx2.5/Csx (SEQ ID NO:9, amino acid sequence; SEQ ID NO:10, nucleotide sequence); a zinc finger-type transcription factor belonging to the GATA family, GATA4 (SEQ ID NO:11, amino acid sequence; SEQ ID NO:12, nucleotide sequence); transcription factors belonging to the myocyte enhance factor-2 (MEF-2) family, MEF-2A (SEQ ID NO:13, amino acid sequence; SEQ ID NO:14, nucleotide sequence), MEF-2B (SEQ ID NO:15, amino acid sequence; SEQ ID NO:16, nucleotide sequence), MEF-2C (SEQ ID NO:17, amino acid sequence; SEQ ID NO:18, nucleotide sequence) and MEF-2D (SEQ ID NO:19, amino acid sequence; SEQ ID NO:20, nucleotide sequence); transcription factors belonging to the basic helix loop helix-type transcription factors, dHAND (SEQ ID NO:21, amino acid sequence; SEQ ID NO:22, nucleotide sequence) and eHAND (SEQ ID NO:23, amino acid sequence; SEQ ID NO:24, nucleotide sequence); and transcription factors belonging to the family of TEA-DNA binding-type transcription factors, TEF-1 (SEQ ID NO:25, amino acid sequence; SEQ ID NO:26, nucleotide sequence), TEF-3 (SEQ ID NO:27, amino acid sequence; SEQ ID NO:28, nucleotide sequence) and TEF-5 (SEQ ID NO:29, amino acid sequence; SEQ ID NO:30, nucleotide sequence).

The cardiomyogenic differentiation of the cells having the potential to differentiate into cardiomyocytes can be induced by introducing DNA encoding one or combination of the above-described factors into the cells and expressing the DNA therein.

It is also possible to induce the cardiomyogenic differentiation of the cells having the potential to differentiate into cardiomyocytes by culturing them using a culture dish coated with an extracellular matrix obtained from spontaneously beating cardiomyocytes, co-culturing with spontaneously beating cardiomyocytes or adding a culture supernatant of spontaneously beating cardiomyocytes.

Furthermore, a factor which induces differentiation of cardiomyocytes which are obtained by the method described in 4 below (hereinafter referred to as "the cardiomyogenic differentiation-inducing factor") can also be used in inducing the cardiomyogenic differentiation of the cells having the potential to differentiate into cardiomyocytes.

### 4. Methods for obtaining cardiomyogenic differentiation-inducing factors

A cardiomyogenic differentiation-inducing factor can be obtained by adding various protease inhibitors to a culture supernatant of spontaneously beating cardiomyocytes, followed by combinations of treatments, such as dialysis, salting-out and chromatography.

Genes encoding such cardiomyogenic differentiation-inducing factors can be obtained by determining partial amino acid sequences of these factors using a microsequencer followed by screening a cDNA library prepared from the spontaneously beating cells using DNA probes designed based on the determined amino acid sequences.

### 5. Therapeutic agents for cardiac regeneration and therapeutic agents for heart diseases comprising cells having the potential to differentiate into cardiomyocytes

The cells having the potential to differentiate into cardiomyocytes according to the present invention can be used as therapeutic agents for cardiac regeneration or for heart diseases.

The heart diseases include myocardial infarction, ischemic heart disease, congestive heart failure, arrhythmia, hypertrophic cardiomyopathy, dilated cardiomyopathy, myocarditis and valvular disease.

The agents for cardiac regeneration contain the cells having the potential to differentiate into cardiomyocytes of high purity which cells have been proliferated *in vitro* according to the position and size of the damaged part of the heart. The preferred cells having the potential to differentiate into cardiomyocytes are those which can be induced to differentiate into various cells constituting the heart such as endocardial endothelial cells, cushion cells, ventricular cardiomyocytes, atrial cardiomyocytes and sinus node cells.

The therapeutic agents can be prepared by purifying the cells having the potential to differentiate into cardiomyocytes from the bone marrow fluid taken from myocardial infarction patients according to the above-described density gradient centrifugation, the panning method (*J. Immunol., 141*(8): 2797-800 (1988)) or the FACS method (*Int. Immuno1.,* 275-83 (1998)) using the antibodies described below which specifically recognize the cells having the potential to differentiate into cardiomyocytes, or a method for constructing a reporter system using the promoter of a gene specifically expressed in the cell having the potential to differentiate into cardiomyocytes.

The therapeutic agents include cardiomyocytes derived from the cells having the potential to differentiate into cardiomyocytes using the myocardium-forming agent described below as well as the cells having the potential to differentiate into cardiomyocytes which are obtained by activating the division potential of the bone marrow cells taken from the bone marrow of aged persons by utilizing the immortalization method described below.

The purity of the therapeutic agents prepared according to the above methods can be tested by the FACS method combined with the antibodies which specifically recognize the cells having the potential to differentiate into cardiomyocytes.

The therapeutic agents can be transported to the damaged parts by a method using a catheter or the like. For example, in the case of ischemic heart disease, the therapeutic agents are transported according to the following procedure. Since the cardiomyocytes damaged by ischemic heart disease exist downstream of vascular stricture, it is necessary to locate the vascular stricture by coronary arteriography (*Illustrated Pathological Internal Medical Course Circulateory Organ,* 1, MEDICAL VIEW, 1993) prior to the injection of the above cells. Organic stricture is classified as concentric stricture, eccentric stricture or multiple mural asymmetry according to type of stricture, and eccentric stricture is further classified into two types, i.e. type I and type II. It is known that the types of stricture are related to the course and prognosis of angina; for instance, eccentric stricture of type II and multiple mural asymmetry are often observed in unstable angina which is liable to shift into myocardial infarction. In cases where blood vessels are completely strictured, there is the possibility that the injected cells can not reach the damaged parts. In such cases, the strictured parts must be reopened by means of percutaneous transluminal coronary angioplasty (PTCA), thrombolytic treatment or the like prior to the cell injection. The type of the cells to be injected such as ventricular or atrial can be selected according to the position of the damaged cardiomyocytes. The insertion of a catheter can be performed by the Sones method (*Illustrated Pathological Internal Medical Course Circulateory Organ,* 1, MEDICAL VIEW, 1993) through the artery of the right upper arm or by the Jundkins method (*Illustrated Pathological Internal Medical Course Circulateory Organ,* 1, MEDICAL VIEW, 1993) through the femural artery.

### 6. Myocardium-forming agents

The myocardium-forming agents according to the present invention comprise, as an active ingredient, at least one cardiomyogenic differentiation-inducing factor selected from the group consisting of a chromosomal DNA-demethylating agent, a factor which is expressed in the cardiogenesis region of a fetus, and a factor which acts on differentiation into cardiomyocytes in the cardiogenesis stage of a fetus, and are capable of inducing the bone marrow-derived cells to differentiate into cardiomyocytes.

Examples of the cardiomyogenic differentiation-inducing factors include cytokines, vitamins, adhesion molecules and transcription factors.

Any cytokine can be used, so long as it stimulates the cardiomyogenic differentiation of the cells having the potential to differentiate into cardiomyocytes in the cardiogenesis stage.

For example, PDGF, FGF-8, endotherin 1 (ET1), Midkine and Bone Marrow Protein 4 (BMP4) can be used. Preferable examples of the PDGF, FGF8, ET1, Midkine, BMP4 include those the amino acid sequences represented by SEQ ID NOS:3 and 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively. The cytokine can be used, e.g., at a concentration of 10 to 40 ng/ml.

Any vitamin can be used, so long as it stimulates the cardiomyogenic differentiation of the cells having the potential to differentiate into cardiomyocytes in the cardiogenesis stage. Retinoic acid can be used, e.g., at a concentration of 10⁻⁹ M.

Any adhesion molecule can be used so far as it is expressed in the cardiogenesis region in the cardiogenesis stage. Examples include gelatin, laminin, collagen, fibronectin and the like. For example, the cardiomyogenic differentiation of the cells having the potential to differentiate into cardiomyocytes can be stimulated by culturing the cells in a culture dish coated with fibronectin.

Examples of the transcription factors include a homeobox-type transcription factor, Nkx2.5/Csx (SEQ ID NO:9, amino acid sequence; SEQ ID NO:10, nucleotide sequence); a zinc finger-type transcription factor belonging to the GATA family, GATA4 (SEQ ID NO:11, amino acid sequence; SEQ ID NO:12, nucleotide sequence); transcription factors belonging to the myocyte enhancer factor-2 (MEF-2) family, MEF-2A (SEQ ID NO:13, amino acid sequence; SEQ ID NO:14, nucleotide sequence), MEF-2B (SEQ ID NO:15, amino acid sequence; SEQ ID NO:16, nucleotide acid sequence), MEF-2C (SEQ ID NO:17, amino acid sequence; SEQ ID NO:18, nucleotide sequence) and MED-2D (SEQ ID NO:19, amino acid sequence; SEQ ID NO:20, nucleotide sequence); transcription factors belonging to the basic helix loop helix-type transcription factors, dHAND (SEQ ID NO:21, amino acid sequence; SEQ ID NO:22, nucleotide sequence), eHAND (SEQ ID NO:23, amino acid sequence; SEQ ID NO:24, nucleotide sequence) and MesP1 (SEQ ID NO:6l, amino acid sequence; SEQ ID NO:62, nucleotide sequence); and transcription factors belonging to the family of TEA-DNA binding-type transcription factors, TEF-1 (SEQ ID NO:25, amino acid sequence; SEQ ID NO:26, nucleotide sequence), TEF-3 (SEQ ID NO:27, amino acid sequence; SEQ ID NO:28, nucleotide sequence) and TEF-5 (SEQ ID NO:29, amino acid sequence; SEQ ID NO:30, nucleotide sequence).

The myocardium-forming agents can contain, as a main component, either a gene encoding a cardiomyogenic differentiation-inducing factor or a protein which is a cardiomyogenic differentiation-inducing factor itself.

### (1) Myocardium-forming agent containing gene as main Component

Methods for preparing the myocardium-forming agents of the present invention which comprise, as a main component, a gene encoding a cardiomyogenic differentiation-inducing factor are described below.

First, a DNA fragment or the full length cDNA of a gene encoding a cardiomyogenic differentiation-inducing factor is inserted downstream of a promoter in a virus vector plasmid to construct a recombinant virus vector plasmid.

Then, the obtained recombinant virus vector plasmid is introduced into a packaging cell which is suitable for the virus vector plasmid.

The recombinant virus vector plasmid lacks at least one of the genes encoding the proteins necessary for the packaging of a virus. As the packaging cell, any cell can be used so far as it can supply the protein encoded by the lacking gene. Suitable packaging cells include HEK293 cell derived from human kidney and mouse fibroblast NIH3T3.

Examples of the proteins supplied by the packaging cells include proteins, such as gag, pol and env, derived from mouse retroviruses for retrovirus vectors; proteins, such as gag, pol, env, vpr, vpu, vif, tat, rev and nef, derived from HIV viruses for lentivirus vectors; proteins, such as E1A and ElB, derived from adenoviruses for adenovirus vectors; and proteins, such as Rep(p5, p19, p40) and Vp(Cap), for adeno-associated viruses.

The virus vector plasmids that can be employed are those capable of producing a recombinant virus in the above packaging cells and comprising a promoter at a position appropriate for the transcription of a wild-type gene corresponding to the causative gene of a congenital genetic heart disease in cardiomyocytes.

Suitable virus vector plasmids include MFG (*Proc. Natl. Acad. Sci. USA, 92*: 6733-6737 (1995)), pBabePuro (*Nucleic Acids Research, 18*: 3587-3596 (1990)), LL-CG, CL-CG, CS-CG and CLG (*Journal of Virology, 72*: 8150-8157 (1998)) and pAdex1 (*Nucleic Acids Res., 23*: 3816-3812 (1995)).

Any promoter can be used as long as it can be expressed in human tissues. Examples of suitable promoters are the promoter of IE (immediate early) gene of cytomegalovirus (human CMV), SV40 early promoter, the promoter of a retrovirus, metallothionein promoter, heat shock protein promoter and SRα promoter. The enhancer of IE gene of human CMV may be used in combination with the promoter. It is possible to express the desired gene specifically in cardiomyocytes using a promoter of a gene specifically expressed in cardiomyocytes such as Nkx2.5/Csx gene.

A recombinant virus vector can be produced by introducing the above recombinant virus vector plasmid into the above packaging cell. Introduction of the virus vector plasmid into the packaging cell can be carried out, for example, by the calcium phosphate method (Japanese Published Unexamined Patent Application No. 227075/90) or the lipofection method (*Proc. Natl. Acad. Sci. USA, 84*: 7413 (1987)).

The above recombinant virus vector can be formulated into myocardium-forming agents by admixture with a carrier used in pharmaceutical compositions for gene therapy (*Nature Genet., 8*: 42 (1994)). Any carrier can be used so long as it is usually used in injections. Suitable carriers include distilled water, salt solutions of sodium chloride or mixtures of sodium chloride and inorganic salts, solutions of mannitol, lactose, dextran, glucose, etc., solutions of amino acids such as glycine and arginine, and mixtures of organic acid solutions or salt solutions and a glucose solution. Injections may be prepared in the form of solutions, suspensions or dispersed solutions according to conventional methods using the above carriers as well as auxiliaries, for example, osmotic pressure adjusting agents, pH adjusting agents, vegetable oils such as sesame oil and soybean oil, lecithin, and surfactants such as nonionic surfactants. If desired, the injections may be prepared in the form of powdered or freeze-dried preparations which are dissolved in a solvent before each use. The myocardium-forming agents in the form of liquid preparations can be used as such for gene therapy, and those in the form of solid preparations are dissolved, immediately before use, in the above carriers which are sterilized if necessary. Administration of the myocardium-forming agents is made locally using a catheter or the like so that the agents can be absorbed into the myocardium of a patient.

The cells having the potential to differentiate into cardiomyocytes infected with the above recombinant virus vector *in vitro* can also be formulated into the above myocardium-forming agents and administered to a patient. Furthermore, the recombinant virus vector can be directly administered to the diseased part of a patient.

### (2) Myocardium-forming agent containing protein as main component

Methods for preparing the myocardium-forming agents of the present invention which contains as a main component, a protein which is a cardiomyogenic differentiation-inducing factor are described below.

On the basis of the full length cDNA encoding a cardiomyogenic differentiation-inducing factor, if necessary, a DNA fragment having an appropriate length containing a region encoding the protein is prepared.

The prepared DNA fragment or the full length cDNA is inserted downstream of a promoter in an expression vector to construct a recombinant expression vector for the protein.

Then, the recombinant expression vector is introduced into a host cell suited for the expression vector.

Any cell can be used so long as it is capable of expressing the desired gene products. Examples of the host cells include bacteria belonging to the genus *Escherichia*, the genus *Serratia,* the genus *Corynebacterium,* the genus *Brevibacterium,* the genus *Pseudomonas,* the genus *Bacillus* and the genus *Microbacterium,* yeasts belonging to the genus *K1uyveromyces,* the genus *Saccharomyces,* the genus *Shizosaccharomyces,* the genus *Trichosporon* and the genus *Schwanniomyces,* animal cells and insect cells.

The expression vectors that can be employed are those capable of autonomous replication or integration into chromosome in the above host cells and containing a promoter at a position suitable for the transcription of a gene of a cardiomyogenic differentiation-inducing factor.

When bacteria are used as the host cell, it is preferred that the recombinant expression vector for a gene encoding a cardiomyogenic differentiation-inducing factor is a recombinant vector which is capable of autonomous replication in the bacterial cell and which comprises a promoter, a ribosome binding sequence, a DNA encoding a protein which can induce cardiomyogenic differentiation, and a transcription termination sequence. The vector can further comprise a gene regulating the promoter.

Examples of suitable expression vectors include pBTrp2, pBTac1 and pBTac2 (manufactured by Boehringer Mannheim), pKK233-2 (manufactured by Amersham Pharmacia Biotech), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 (*Agricultural Biological Chemistry, 48*: 669 (1984)), pLSAl (*Agric. Bio1. Chem., 53*: 277 (1989)), pGELl (*Proc. Natl. Acad. Sci. USA, 82*: 4306 (1985)), pBluescript II SK (-) (manufactured by Stratagene), pGEX (manufactured by Amersham Pharmacia Biotech), pET-3 (manufactured by Novagen), pTerm2 (U.S. Patents 4,686,191, 4,939,094 and 5,160,735), and pSupex, pUB110, pTP5, pC194 and pEG400 (*J. Bacteriol., 172*: 2392 (1990)).

It is preferred to use a plasmid in which the distance between the Shine-Dalgarno sequence (ribosome binding sequence) and the initiation codon is adjusted to a suitable length (e.g., 6-18 bases).

Any promoter can be used so long as it can be expressed in the host cell. For example, promoters derived from *Escherichia coli* or a phage, such as *trp* promoter (Pₜᵣₚ)/ *lac* promoter (P_{lac}), P_{L} promoter, P_{R} promoter and T7 promoter, SPO1 promoter, SPO2 promoter and penP promoter can be used. Artificially modified promoters such as a promoter in which two Ptrp are combined in tandem (Pₜᵣₚ×2), tac promoter, *let*I promoter (*Gene, 44*: 29 (1986)) and *lacT*7 promoter can also be used.

The yield of the desired protein can be improved by replacing a nucleotide in the nucleotide sequence of the protein-encoding region in the gene of the cardiomyogenic differentiation-inducing factor of the present invention so as to make a codon most suitable for the expression in a host cell.

The transcription termination sequence is not essential for the expression of the gene encoding the cardiomyogenic differentiation-inducing factor of the present invention, but it is preferred that the transcription termination sequence is located immediately downstream of the structural gene.

Examples of suitable host cells are cells of microorganisms belonging to the genus *Escherichia,* the genus *Serratia,* the genus *Corynebacterium,* the genus *Brevibacterium,* the genus *Pseudomonas,* the genus *Bacillus* and the genus *Microbacterium,* specifically, *Escherichia coli* XL1-Blu *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Bacillus subtilis, Bacillus amylollquefaciens, Brevibacterium ammoniagenes, Brevibacterium immariophilum* ATCC 14068, *Brevibacterium saccharolyticum* ATCC 14066, *Corynebacterium glutamicum* ATCC 13032, *Corynebacterium glutamicum* ATCC 14067, *Corynebacterium glutamicum* ATCC 13869, *Corynebacterium acetoacidophilum* ATCC 13870, *Microbacterlum ammmoniaphilum* ATCC 15354 and *Pseudomonas* sp. D-0110.

Introduction of the recombinant vector can be carried out by any of the methods for introducing DNA into the above host cells, for example, the method using calcium ion (*Proc. Natl. head. Sci. USA, 69*: 2110 (1972)), the protoplast method (Japanese Published Unexamined Patent Application No. 248394/88) and the methods described in *Gene, 17*: 107 (1982) and *Molecular & General Genetics, 168*: 111 (1979).

When yeast is used as the host cell, YEp13 (ATCC 37115), YEp24 (ATCC 37051), YCp50 (ATCC 37419), pHS19, pHS15, etc. can be used as the expression vector.

Any promoter can be used, so long as it can be expressed in the yeast. Suitable promoters include PH05 promoter, PGK promoter, GAP promoter, ADH promoter, gal 1 promoter, gal 10 promoter, heat shock protein promoter, MFα 1 promoter and CUP 1 promoter.

Examples of suitable host cells include cells of *Saccharomyces cerevisiae, Schizosaccharomyces pombe, Kluyveromyces 1actis, Trichosporon pullulans* and *Schwannlomyces alluvius.*

Introduction of the recombinant vector can be carried out by any of the methods for introducing DNA into yeast cells, for example, electroporation (*Methods. Enzymo1, 194*: 182 (1990)), the spheroplast method (*Proc. Natl. Acad. Sci. USA, 75*: 1929 (1978)) and the lithium acetate method (*J. Bacterio1., 153*: 163 (1983), *Proc. Natl. Acad. Sci. USA, 75*: 1929 (1978)).

When an animal cell is used as the host cell, pcDNAI (manufactured by Invitorogen), pcDM8 (manufactured by Invitorogen), pAGE107 (Japanese Published Unexamined Patent Application No. 22979/91, *Cytotechnology, 3*: 133 (1990)), pAS3-3 (Japanese Published Unexamined Patent Application No. 227075/90), pCDM8 (*Nature, 329*: 840 (1987)), pcDNAI/Amp (manufactured by Invitrogen), pREP4 (manufactured by Invitrogen), pAGE103 (*J. Biochem. 101*: 1307 (1987)), pAGE210, *etc.* can be used as the expression vector.

As the promoter, any promoters capable of expression in animal cells can be used. Suitable promoters include the promoter of IE (immediate early) gene of cytomegalovinus (human CMV), SV40 early promoter, the promoter of a retrovirus, metallothionein promoter, heat shock protein promoter and SRα promoter. The enhancer of IE gene of human CMV may be used in combination with the promoter.

Examples of suitable host cells are human Namalwa cell, monkey COS cell, Chinese hamster CHO cell and HBT5637 (Japanese Published Unexamined Patent Application No. 299/88).

Introduction of the recombinant vector can be carried out by any of the methods for introducing DNA into animal cells, for example, electroporation method (*Cytotechnology, 3*: 133 (1990)), the calcium phosphate method (Japanese Published Unexamined Patent Application No. 227075/90), and lipofection method (*Proc. Natl. Acad. Sci. USA, 84*: 7413 (1987), *Virology, 52*: 456 (1973)). A transformant can be obtained and cultured according to the methods described in Japanese Published Unexamined Patent Application Nos. 227075/90 and 257891/90.

When an insect cell is used as the host cell, the protein can be expressed using the methods descried in *Baculovirus Expression Vectors, A Laboratory Manual,* W.H. Freeman and Company, New York (1992), *Current Protocols in* *Molecular Biology,* Supplement 1-38 (1987-1997), *Bio*/*Technology, 6*: 47 (1988), *etc*.

Specifically, the recombinant gene transfection vector and a baculovirus are cotransfected into an insect cell to obtain a recombinant virus in the culture supernatant of the insect cell, and then an insect cell is infected with the recombinant virus to express the protein.

Examples of the gene transfection vectors suitable for use in this method are pVL1392, pVL1393 and pBlueBacIII (manufactured by Invitrogen).

Examples of the baculovirus include Autographa californica nuclear polyhedrosis virus with which an insect belonging to the family *Barathra* is infected.

Examples of the insect cells include Sf9 and Sf21 (*Baculovirus Expression Vectors, A Laboratory Manual,* W.H. Freeman and Company, New York (1992)), which are ovary cells of *Spodoptera frugiperda,* and High 5 (manufactured by Invitrogen), which is an ovary cell of *Trichoplusia ni.*

Cotransfection of the recombinant gene transfection vector and the baculovirus into an insect cell for the preparation of the recombinant virus can be carried out by the calcium phosphate method (Japanese Published Unexamined Patent Application No. 227075/90), the lipofection method (*Proc. Natl Acad. Sci. USA, 84*: 7413 (1987)), *etc.*

Expression of the gene can be carried out not only by direct expression but also by secretory production, fused protein expression, *etc.* according to the methods described in *Molecular Cloning, A Laboratory Manual,* Second Edition, Cold Spring Harbor Laboratory Press (1989) (hereinafter referred to as *"Molecular Cloning, A Laboratory Manual,* 2nd ed.") *etc.*

When the gene is expressed in yeast, an animal cell or an insect cell, a glycoprotein or glycosylated protein can be obtained.

The protein as the cardiomyogenic differentiation-inducing factor can be produced by culturing the transformant carrying the recombinant DNA containing the DNA encoding the protein as the cardiomyogenic differentiation-inducing factor in a medium, allowing the protein to accumulate in the culture, and recovering the protein from the culture.

Culturing of the transformant for the production of the protein as the cardiomyogenic differentiation-inducing faactor can be carried out by conventional methods for culturing the host cell of the transformant.

For the culturing of the transformant prepared using a procaryotic cell such as *E. coli* or a eucaryotic cell such as yeast as the host cell, any of natural media and synthetic media can be used, so long as it is a medium suitable for efficient culturing of the transformant which contains a carbon source, a nitrogen source, an inorganic substance, etc. which can be assimilated by the host used.

Any carbon source can be used, so long as it can be assimilated by the host. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

Examples of the nitrogen sources include ammonia, ammonium salts of inorganic or organic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented cells and digested products thereof.

Examples of the inorganic substances include potassium dihydorgenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration, at 15-40°C for 16 hours to 7 days. The pH is maintained at 3.0-9.0 during the culturing. The pH is adjusted using an organic or inorganic acid, an alkali solution, urea, calcium carbonate, ammonia, *etc*.

If necessary, antibiotics, such as ampicillin and tetracycline, can be added to the medium during the culturing.

When a microorganism transformed with an expression vector comprising an inducible promoter is cultured, an inducer may be added to the medium, if necessary. For example, in the case of a microorganism transformed with an expression vector containing *lac* promoter, isopropyl-β-D-thiogalactopyranoside (IPTG) or the like can be added to the medium; and in the case of a microorganism transformed with an expression vector containing *trp* promoter, indoleacrylic acid (IAA) or the like can be added.

For the culturing of the transformant prepared using an animal cell as the host cell, generally used media such as RPMI1640 medium (*The Journal of the American Medical Association, 199*: 519 (1967)), Eagles's MEM (*Science, 122*: 501 (1952)), Dulbecco's modified MEM (*Virology, 8*: 396 (1959)) and 199 medium (*Proceeding of the Society for the Biological Medicine, 73*: 1 (1950)), media prepared by adding fetal calf serum to these media, *etc.* can be used as the medium.

Culturing is usually carried out at pH 6-8 at 30-40°C for 1-7 days in the presence of 5% CO₂.

If necessary, antibiotics, such as kanamycin and penicillin, can be added to the medium during the culturing.

For the culturing of the transformant prepared using an insect cell as the host cell, generally used media such as TNM-FH medium (manufactured by Pharmingen), Sf-900II SFM medium (manufactured by Life Technologies), ExCell 400 and ExCell 405 (manufactured by JRH Biosciences) and Grace's Insect Medium (Grace, T.C.C., *Nature, 195*: 788 (1962)) can be used as the medium.

Culturing is usually carried out at pH 6-7 at 25-30°C for 1-5 days.

If necessary, antibiotics, such as gentamicin, can be added to the medium during the culturing.

The protein as the cardiomyogenic differentiation-inducing factor can be isolated and purified from the culture of the transformant by conventional methods for isolating and purifying proteins.

For example, when the protein as the cardiomyogenic differentiation-inducing factor is expressed in a soluble form in cells, the isolation and purification can be carried out in the following manner. After the completion of culturing, the cells are recovered from the culture by centrifugation and suspended in an aqueous buffer, followed by disruption using an ultrasonic disrupter, a French press, a Manton Gaulin homogenizer, a Dyno Mill, etc. to obtain a cell-free extract. The cell-free extract is centrifuged, and a purified protein preparation can be produced from the obtained supernatant using ordinary means for isolation and purification of proteins, for example, extraction with a solvent, salting-out with ammonium sulfate, etc., desalting, precipitation with an organic solvent, anion exchange chromatography using resins such as diethylaminoethyl (DEAE)-Sepharose and DIAION HPA-75 (Mitsubishi Chemical Corporation), cation exchange chromatography using resins such as S-Sepharose FF (manufactured by Amersham Pharmacia Biotech), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the protein is expressed as an insoluble substance in cells, the cells are separated and disrupted, followed by centrifugation to recover the insoluble substance of the protein as a precipitate fraction.

The recovered insoluble substance of the protein is solubilized with a protein-denaturing agent. The solubilized protein solution is diluted or dialyzed to lower the concentration of the protein-denaturing agent therein, thereby restoring the normal tertiary structure of the protein, followed by the same isolation and purification steps as described above to obtain a purified protein preparation.

When the protein as the cardiomyogenic differentiation-inducing factor or its derivatives, such as a glycosylated protein, are extracellularly secreted, they can be recovered from the culture supernatant. That is, the culture is treated by means such as centrifugation and the obtained culture supernatant is subjected to the same isolation and purification steps as mentioned above to obtain a purified protein preparation.

The thus obtained proteins include the proteins having the amino acid sequences represented by SEQ ID NOS:5, 6, 10, 12, 14, 16, 18, 20, 22, 24, 26, 28 and 30.

The proteins expressed by the above methods can also be produced by chemical synthetic methods such as the Fmoc method (the fluorenylmethyloxycarbonyl method) and the tBoc method (the t-butyloxycarbonyl method). Furthermore, the proteins can be synthesized using peptide synthesizers (for example, manufactured by Advanced ChemTech, Perkin-Elmer, Amersham Pharmacia Biotech, Protein Technology Instrument, Synthecell-Vega, PerSeptive, Shimadzu Corporation, *etc.*).

The protein which can induce cardiomyogenic differentiation can be formulated into myocardium-forming agents and administered in the same manner as in the above (1).

### 7. Application to therapy of congenital genetic disease

In some of the diseases leading to heart failure, the deficiency of an essential protein due to the mutation of a single gene causes heart failure. Examples of such diseases are familial hypertrophic cardiomyopathy, Fabri disease, QT elongation syndrome, Marfan syndrome, aortic stenosis, mitochondria cardiomyopathy and Duchenne muscular dystrophy. These diseases are known to be caused by the abnormality in the genes of myosin, troponin, tropomyosin, potential-dependent Na channel, K channel, fibrin, elastin, mitochondria, dystrophin, *etc.* (*Therapeutics, 30*: 1302-1306 (1996)).

The method for treating a patient of the above disease includes a method comprising acquiring the cells having the potential to differentiate into cardiomyocytes of the present invention from a patient of the disease, introducing the wild type gene corresponding to the causative gene of the disease into the cells, and transplanting the cells to the patient's heart. The normal gene is inserted into the vector for gene therapy described in the above 6(1), and then can be introduced into the cells having the potential to differentiate into cardiomyocytes of the present invention using the vector for gene therapy described in the above 6(1).

### 8. Methods for obtaining antibody which specifically recognizes surface antigen specific for cells having the potential to differentiate into cardiomyocytes

Methods for preparing antibodies which specifically recognize surface antigens expressed in the cells having the potential to differentiate into cardiomyocytes of the present invention are described below.

The antibodies which recognize the surface antigens expressed specifically in the cells having the potential to differentiate into cardiomyocytes of the present invention are useful in the purity test and purification of the cells required for applying the cells to the therapy of heart diseases such as myocardial infarction.

In order to obtain the antibody, an antigen is administered subcutaneously, intravenously or intraperitoneally to a non-human mammal, such as rabbit or goat, or 3 to 20-weeks-old rat, mouse or hamster together with an appropriate adjuvant, such as complete Freund's adjuvant, aluminum hydroxide gel or pertussis vaccine. As the antigen, the cells having the potential to differentiate into cardiomyocytes of the present invention (3×10⁵ to 5×10⁵ cells/animal) or the cell membrane fraction prepared from the cells (1-10 mg/animal) is used.

Administration of the antigen is repeated 3 to 10 times after the first administration at intervals of 1 to 2 weeks. On the 3rd to 7th day after each administration, a blood sample is collected from fundus oculi veniplex and the obtained serum is examined from reactivity to the antigen used for immunization according to enzyme immunoassay (*Enzyme-Linked Immuno Adsorbent Assay* (*ELISA*), Igaku Shoin (1976), *Antibodies - A Laboratory Manual,* Cold Spring Harbor Laboratory (1988)). A non-human mammal whose serum shows a sufficient antibody titer against the antigen used for immunization is employed as a source of serum or antibody-producing cell.

The polyclonal antibody can be prepared by separation and purification from the serum.

For the preparation of the monoclonal antibody, the antibody-producing cell and a myeloma cell derived from a non-human mammal are fused to obtain hybridoma, and the hybridoma is cultured or administered to an animal to cause ascites tumor. The monoclonal antibody can be prepared by separation and purification from the resulting culture or ascites.

Examples of the antibody-producing cells include spleen cells and antibody-producing cells in lymph nodes or peripheral blood, and among these, spleen cells are preferably used.

As the myeloma cells, mouse-derived cell lines are preferably used. Examples of suitable cell lines are P3-X63Ag8-U1 (P3-U1) cell line (Current *Topics in Microbiology and Immunology, 18*: 1 (1978)), which is 8-azaguanine-resistant mouse (BALB/c-derived) myeloma cell line, P3-NS1/1-Ag41(NS-1) line (*European J. Immunology, 6*: 511 (1976)), SP2/0-Ag14(SP-2) line (*Nature, 276*: 269 (1978)), P3-X63-Ag8653(653) line (*J. Immunology, 123*: 1548 (1979)) and P3-X63-Ag8(X63) line (Nature, 256: 495 (1975)).

The hybridoma can be prepared in the following manner.

The antibody-producing cells and the myeloma cells are mixed and suspended in HAT medium (a medium prepared by adding hypoxanthine, thymidine and aminopterin to a normal medium), followed by culturing for 7-14 days. After the culturing, a portion of the culture supernatant is subjected to enzyme immunoassay to select cells which react with the antigen and do not react with the protein containing no antigen. Then, cloning is carried out by limiting dilution method, and cells showing a high and stable antibody titer according to enzyme immunoassay are selected as the monoclonal antibody-forming hybridomas.

Separation and purification of the polyclonal antibodies and the monoclonal antibodies can be carried out using means such as centrifugation, ammonium sulfate precipitation, caprylic acid precipitation, and chromatography using DEAE-Sepharose column, anion exchange column, protein A- or G-column or gel filtration column, alone or in combination.

Sampling cells can be easily tested for expression of the surface antigen expressed in the cells having the potential to differentiate into cardiomyocytes by comparing the reactivity of the thus obtained antibody specifically which recognizes the surface antigen to the test cells with that to control cells such as hematopoietic stem cells and neural stem cells.

### 9. Methods for obtaining surface antigen expressed in cells having the potential to differentiate into cardiomyocytes and gene encoding the surface antigen

The genes encoding the surface antigens expressed specifically in the cells having the potential to differentiate into cardiomyocytes can be obtained by the cDNA subtraction method (*Proc. Natl. Acad. Sci. USA, 85*: 5738-5742 (1988)) and the representational difference analysis (*Nucleic Acids Research, 22*: 5640-5648 (1994)), which are methods for obtaining genes showing different expression profiles between two samples of different origins.

First, a cDNA library prepared from the cells having the potential to differentiate into cardiomyocytes is subjected to subtraction using mRNA obtained from control cells other than cells having the potential to differentiate into cardiomyocytes, e.g., hematopoietic stem cells and neural stem cells. Then a subtracted cDNA library with a high content of a gene specifically expressed in the cells having the potential to differentiate into cardiomyocytes is prepared, followed by nucleotide sequence analysis of inserted cDNA in the subtracted cDNA library from the 5' terminal side randomly to select those having the secretion signal sequence (random sequence analysis). The full length nucleotide sequences of the thus obtained cDNAs are determined to distinguish the proteins encoded by the cDNAs into secretory proteins and membrane proteins.

In the above process, the signal sequence trap method can be used instead of the random sequence analysis (*Science, 261*: 600-603 (1993), *Nature Biotechnology, 17*: 487-490 (1999)). The signal sequence trap method is a method for selectively screening for genes having the secretion signal sequence.

In order to efficiently obtain the specific surface antigens, it is preferred to prepare a signal sequence trap library from the cells having the potential to differentiate into cardiomyocytes using a vector suitable for subtraction and to subject the signal sequence trap library to subtraction using mRNA obtained from control cells such as hematopoietic stem cells and neural stem cells. The thus obtained DNA fragments containing the secretion signal sequence can be used as probes for cloning the full length cDNAs.

The proteins encoded by the cDNAs can be distinguished into secretory proteins and membrane proteins by determining the full length nucleotide sequences of the full length cDNAs.

When the obtained clone DNA, whether it is obtained by the random sequence analysis or the signal sequence trap method, codes for a membrane protein, the specific antibody can be obtained by the above method using the synthetic peptide prepared based on the amino acid sequence presumed from the nucleotide sequence as an antigen.

The membrane proteins encoded by the clones include receptors, which may act on the regulation of specific growth of cells having the potential to differentiate into cardiomyocytes or their differentiation into cardiomyocytes. The clone encoding such a receptor can be used in the search for a ligand of the receptor. When the clone codes for a secretion protein, it can be used directly for the growth or differentiation of the cells having the potential to differentiate into cardiomyocytes.

### 10. Methods for screening for growth factor for cells having the potential to differentiate into cardiomyocytes and factor inducing the differentiation into cardiomyocytes

Screening for a growth factor for the cells having the potential to differentiate into cardiomyocytes and a factor inducing their differentiation into cardiomyocytes can be carried out by culturing the cells having the potential to differentiate into cardiomyocytes in a serum-free medium in the presence of a test substance and evaluating the growth or the cardiomyogenic differentiation of the cells.

This screening method is applicable to a wide variety of test substances, for example, secretion proteins such as various cytokines and growth factors, membrane-bound proteins such as cell adhesion molecules, tissue extracts, synthetic peptides, synthetic compounds, and culture broths of microorganisms.

The growth capability can be evaluated by examining the colony forming activity, the BrdU uptake, etc.

The colony forming activity can be examined by scattering the cells having the potential to differentiate into cardiomyocytes of the present invention at a low density.

The BrdU uptake can be examined by immunostaining using an antibody which specifically recognizes BrdU.

The cardiomyogenic differentiation can be evaluated according to a method using spontaneous beating as an indicator, a method using the expression of a reporter gene introduced into the cells as an indicator, and the like.

The method using the expression of a reporter gene introduced into the cells as an indicator is a method in which a vector DNA comprising the promoter of a gene expressed specifically in cardiomyocytes and a reporter gene is introduced into cells having the potential to differentiate into cardiomyocytes and the expression of the reporter gene as an indicator is examined using the cells.

The reporter gene includes genes encoding GFP (gleen fluorescent protein), luciferase or β-galactosidase, and the like.

The promoter of a gene expressed specifically in cardiomyocytes includes cardiac troponin I (cTNI) (*J. Biological Chemistry, 273*: 25371-25380 (1998)).

### 11. Methods for immortalizing bone marrow cells having the potential to differentiate into cardiomyocytes

When the therapeutic agent according to the present invention is administered to cardiac patients, especially aged patients, it is preferred that the proliferative activity of the cells having the potential to differentiate into cardiomyocytes of the present invention should be potentiated without generating cancer.

The proliferative activity of the cells having the potential to differentiate into cardiomyocytes can be increased without cancer generation by expressing telomerase in the cells.

The methods for expressing telomerase in the cells having the potential to differentiate into cardiomyocytes of the present invention include: a method which comprises inserting TERT gene which is the catalytic subunit of telomerase, specifically, the DNA represented by SEQ ID NO:32 into a retrovirus vector and introducing the resulting vector into the cells having the potential to differentiate into cardiomyocytes; a method which comprises administering a factor inducing the expression of the TERT gene inherent in the cells having the potential to differentiate into cardiomyocytes to the cells having the potential to differentiate into cardiomyocytes; and a method which comprises introducing a vector containing DNA encoding a factor inducing the expression of the TERT gene into the cells having the potential to differentiate into cardiomyocytes.

The above-described factors inducing the expression of the TERT gene can be selected by introducing a vector DNA to which a reporter gene such as GFP (green fluorescent protein), luciferase, β-galactosidase or the like has been inserted, into the cells having the potential to differentiate into cardiomyocytes.

### 12. Method of separating cells having the potential to differentiate into cardiomyocytes using antibody

The method for obtaining cells in which a target surface antigen is expressed from extirpated various *in vivo* tissues includes a method using a flow cytometer having a sorting function and a method using magnetic beads.

The sorting function of a flow cytometer can be performed by the droplet charge system, the cell capture system, etc. (*Perfect Command of Flow Cytometer,* p.14-23, Shujunsha, 1999). In using each of these systems, the expression amount of an antigen can be quantitated by converting the fluorescent intensity emitted from an antibody binding to a molecule expressed on the cell surface into an electric signal. When plural fluorescences are used in combination, the cells can be separated using plural surface antigens. Examples of the fluorescence include FITC (fluorescein insothiocyanate), PE (phycoerythrin), APC (Allo-phycocyanin), TR (TexasRed), Cy3, CyChrome, Red613, Red670, PerCP, TRI-Color, QuantumRed, etc. (*Perfect Command of Flow Cytometer,* p.3-13, Shujunsha, 1999).

The staining method includes a method in which cells are centrifugally separated from extirpated various *in vivo* tissues such as bone marrow or umbilical blood, and the cells are stained directly with antibodies, and a method in which the cells are once cultured and proliferated in an appropriate medium and then stained with antibodies.

For staining, the target cells are first mixed with a primary antibody, which recognizes a surface antigen, and incubated on ice for 30 minutes to 1 hour. When the primary antibody is labeled with a fluorescence, the cells are washed and then separated with a flow cytometer. When the primary antibody is not labeled with a fluorescence, the cells are washed and then a secondary antibody labeled with a fluorescence having an activity of binding to the primary antibody is mixed with the cells having reacted with the primary antibody and incubated on ice again for 30 minutes to 1 hour. After washing, the cells stained with the primary and secondary antibodies are separated with a flow cytometer.

By the method using magnetic beads, cells expressing specific target surface antigen can be separated in a large amount. Although this method is inferior in the separation purity to the flow cytometer method as described above, repeated purification ensures a sufficiently high cell purity.

After staining the cells with the primary antibody, the residual primary antibody is eliminated. Then the cells are stained with the secondary antibody bonded to the magnetic beads capable of binding to the primary antibody. After washing away the residual secondary antibody, the cells can be separated using a stand provided with a magnet. The materials and apparatus required in these operations are available from Dynal Biotech.

The magnetic bead method is also usable in eliminating unnecessary cells from cell samples. The StemSep method marketed from Stem Cell Technologies Inc. (Vancouver, Canada) can be used to eliminate these unnecessary cells more efficiently.

Examples of the antibodies to be used in the above-described methods include the antibodies acquired in the above 8, antibodies which recognize hematopoietic cell surface antigens, CD34, CD117, CD14, CD45, CD90, Sca-1, Ly6c or Ly6g, antibodies which recognize vascular endothelial cell surface antigens, Flk-1, CD31, CD105 or CD144, an antibody which recognizes a mesenchymal cell surface antigen, CD140, antibodies which recognize integrin surface antigens, CD49b, CD49d, CD29 or CD41, and antibodies which recognize matrix receptors, CD54, CD102, CD106 or CD44. When these antibodies are used in combination, the target cells can be obtained at a higher purity.

Specifically, in order to obtain CD34-negative, CD117-positive, CD144-negative and CD140-positive cells, CD34-positive cells and CD144-positive cells are eliminated from human bone marrow cells by, for example, the above-described immune magnetic bead method and then a CD117-positive and CD140-positive cell fraction is recovered to separate the target cells.

### 13. Separation of cardiomyocyte precursor cells using myocardium-specific gene prompter reporter vector

In order to efficiently separate cardiomyocytes or cardiomyocyte precursor cells derived from cells having the potential to differentiate into cardiomyocytes, green fluorescent protein (GFP) of luminous Aequorea can be used as a reporter gene for gene transfer.

Specifically, a vector is constructed by ligating the GFP gene to the downstream of a promoter of a gene specifically expressed in myocardium or a gene specifically expressed in the cells having the potential to differentiate into cardiomyocytes obtained in the above 9. Then, the vector is introduced into the cells having the potential to differentiate into cardiomyocytes. The cells introducing the reporter vector are separated depending on, for example, tolerance to antibiotics followed by the induction of cardiomyogenic differentiation. The differentiation-induced cells exhibit the expression of GFP and emit fluorescence. The cardiomyocytes and cardiomyocyte precursor cells emitting the fluorescence can be easily separated using a flow cytometer (*Perfect Command of Flow Cytometer,* p.44-52, Shujunsha, 1999).

Examples of the promoter of the gene specifically expressed in myocardium include MLC2v and troponin I.

Examples of the vector include the above-described plasmid vectors for animal cells, and adenovirus vectors.

### 14. Induction of differentiation of cells having the potential to differentiate into cardiomyocytes, into various cells

### (1) Induction of differentiation of cells having the potential to differentiate into cardiomyocytes into adipocytes

Examples of the method for inducing the differentiation of the cells having the potential to differentiate into cardiomyocytes into adipocytes include a method wherein an activator of a nuclear receptor, PPARγ, is added to the medium to give a final concentration of 0.4 to 2 µM. The activator of a nuclear receptor, PPARγ, includes compounds having a thiazolidione skeleton such as troglitazone, pioglitazone, rosiglitazone and the like.

The examples also include a method wherein the cells are cultured in a medium to which dexamethasone, methyl-isobutylxanthine, insulin and indomethacin have been added to a culture of cells confluently grown over a culture dish to give final concentrations of 1 µM, 0.5 mM, 0.01 mg/ml and 0.2 mM, respectively.

### (2) Induction of differentiation of cells having the potential to differentiate into cardiomyocytes into chondrocytes

Examples of the method for inducing the differentiation of the cells having the potential to differentiate into cardiomyocytes into chondrocytes include a method wherein aggregates obtained by centrifuging 1×10⁵ to 3×10⁵ cells are cultured in a medium containing TGFβ3 in a final concentration of 0.01 µg/ml.

### (3) Induction of differentiation of cells having the potential to differentiate into cardiomyocytes into osteoblasts

Examples of the method for inducing the differentiation of the cells having the potential to differentiate into cardiomyocytes into osteoblasts include a method wherein the cells are cultured in a medium containing dexamethasone, ascorbic acid-2-phosphate and β-glycerophosphate in final concentrations of 0.1 µM, 0.05 mM and 10 mM, respectively.

### 15. Purification of stem cell using Hoechst 33342

Hoechst 33342 is a DNA binding reagents which can stain viable cells. Since the majority of bone marrow cells are vigorously divided, they are stained markedly lightly but immature cells are stained darkly. It is known that this phenomenon becomes significant in cells having immature ability to exclude pigment by ABC (ATP binding cassette) transporter (H. Nakauchi, *Protein, Nucleic Acid and Enzyme, 45*: 13, 2056-2062 (2000)).

Cells which are stained darkly with Hoechst 33342 can be separated from the bone marrow by staining bone marrow cells with Hoechst 33342 and then analyzing them by carrying out double staining of a short wavelength and a long wavelength by applying UV laser using FACS. Immature cells which do not incorporate Hoechst 33342 can be fractionated as side population (Goodell, M.A. *et al., J. Exp. Med., 183*: 1797-1806 (1996), http://www.bcm.tmc.edu/genetherapy/goodell/new_site/index2.html).

### BRIEF EXPLANATION OF THE DRAWINGS

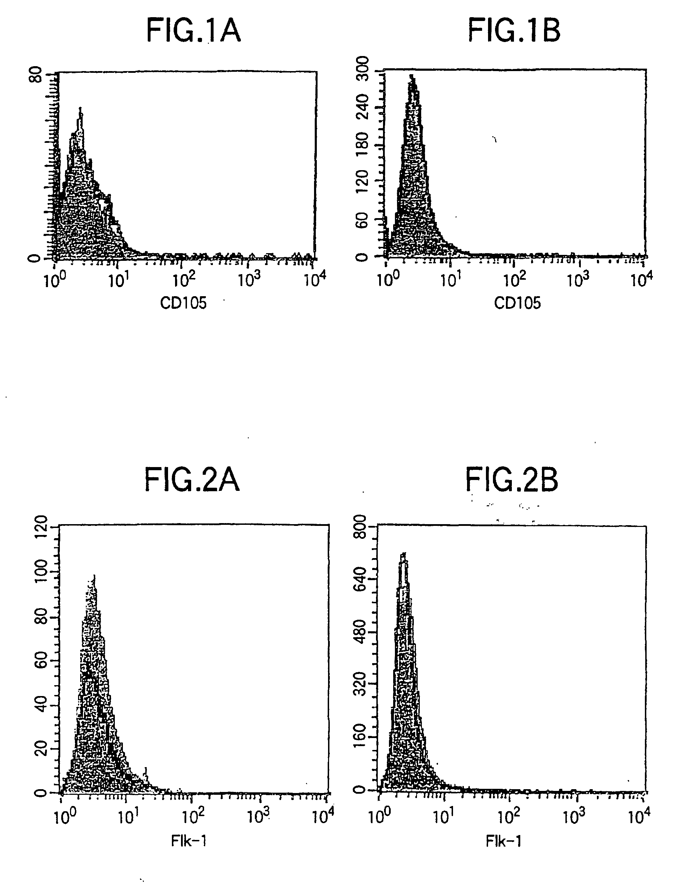

Fig. 1 shows a result of the antibody reaction of KUM2 cell (A) or BMSC cell (B) using a biotinylated anti-mouse CD105 antibody which was measured by a flow cytometer. The ordinate and abscissa show the number of cells and the fluorescence intensity, respectively. The area painted out with gray is a result of the antibody reaction, and the solid line is a result of a negative control.

Fig. 2 shows a result of the antibody reaction of KUM2 cell (A) or BMSC cell (B) using a biotinylated anti-mouse Flk1 antibody which was measured by a flow cytometer. The ordinate and abscissa show the number of cells and the fluorescence intensity, respectively. The area painted out with gray is a result of the antibody reaction, and the solid line is a result of a negative control.

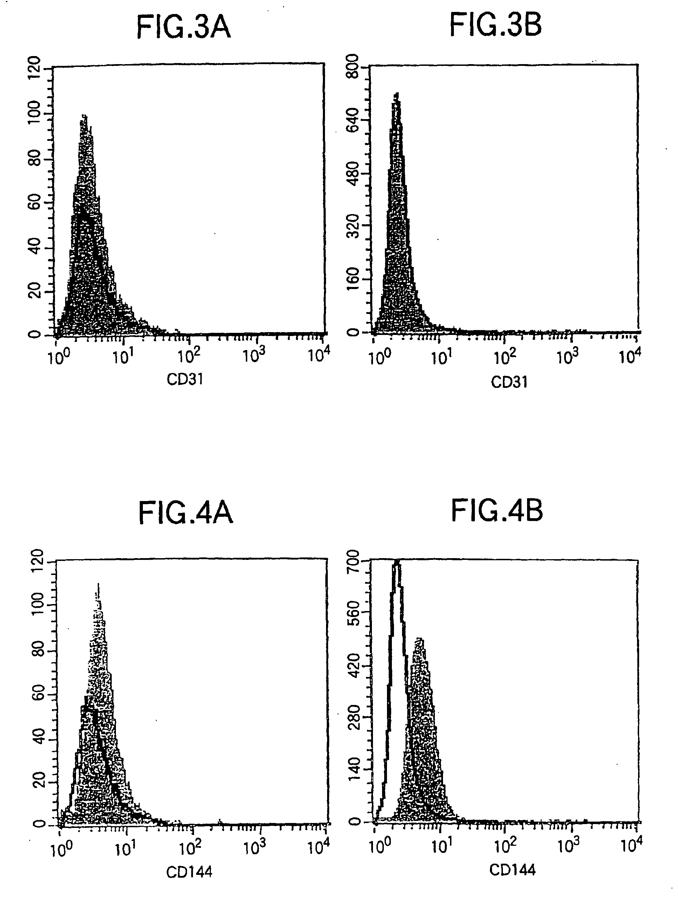

Fig. 3 shows a result of the antibody reaction of KUM2 cell (A) or BMSC cell (B) using an FITC-labeled anti-mouse CD31 antibody which was measured by a flow cytometer. The ordinate and abscissa show the number of cells and the fluorescence intensity, respectively. The area painted out with gray is a result of the antibody reaction, and the solid line is a result of a negative control.

Fig. 4 shows a result of the antibody reaction of KUM2 cell (A) or BMSC cell (B) using a biotinylated anti-mouse CD144 antibody which was measured by a flow cytometer. The ordinate and abscissa show the number of cells and the fluorescence intensity, respectively. The area painted out with gray is a result of the antibody reaction, and the solid line is a result of a negative control.

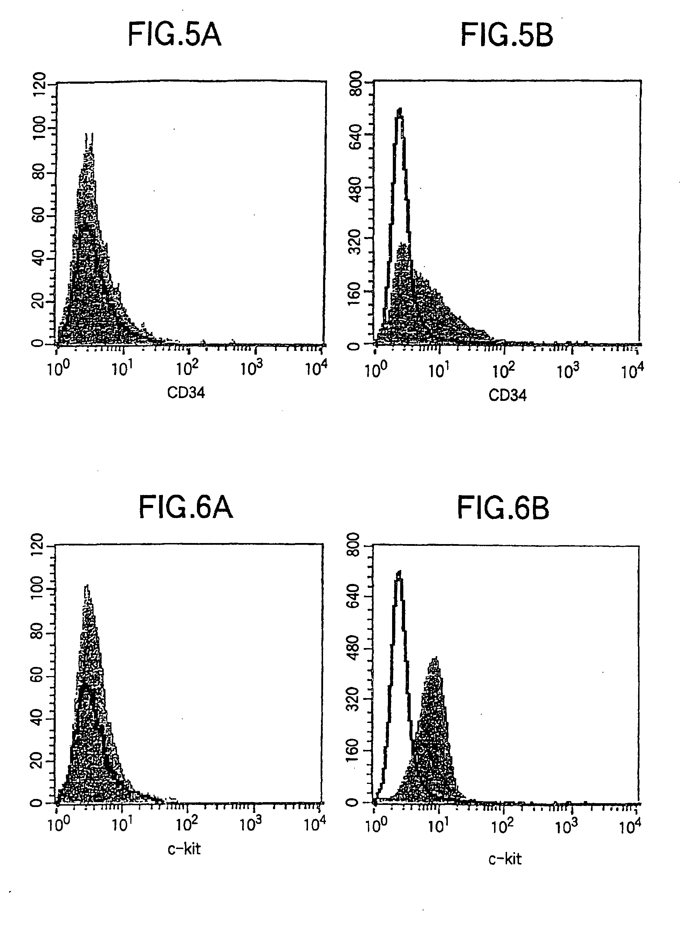

Fig. 5 shows a result of the antibody reaction of KUM2 cell (A) or BMSC cell (B) using an FITC-labeled anti-mouse CD34 antibody which was measured by a flow cytometer. The ordinate and abscissa show the number of cells and the fluorescence intensity, respectively. The area painted out with gray is a result of the antibody reaction, and the solid line is a result of a negative control.

Fig. 6 shows a result of the antibody reaction of KUM2 cell (A) or BMSC cell (B) using an FITC-labeled anti-mouse CD117(c-kit) antibody which was measured by a flow cytometer. The ordinate and abscissa show the number of cells and the fluorescence intensity, respectively. The area painted out with gray is a result of the antibody reaction, and the solid line is a result of a negative control.

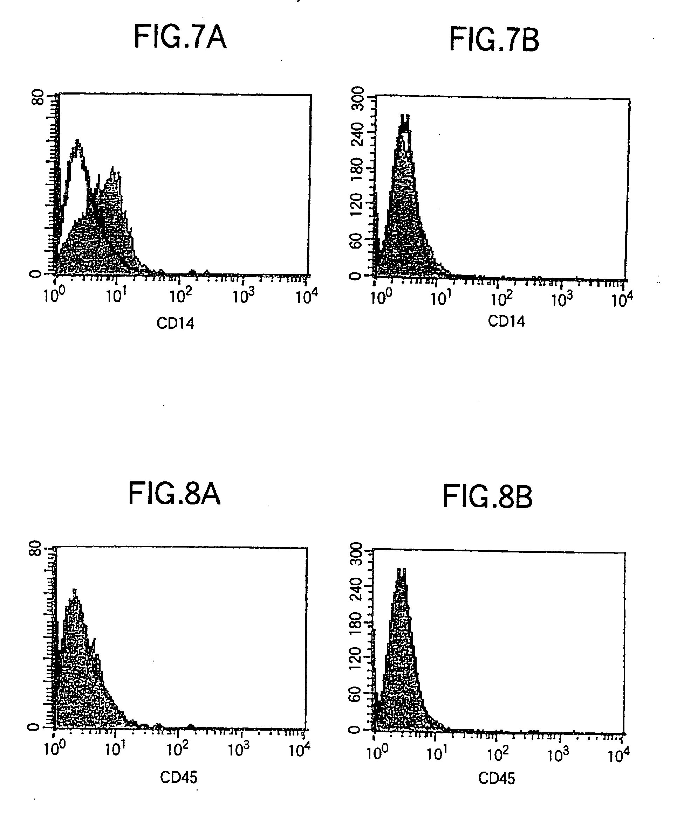

Fig. 7 shows a result of the antibody reaction of KUM2 cell (A) or BMSC cell (B) using an FITC-labeled anti-mouse CD14 antibody which was measured by a flow cytometer. The ordinate and abscissa show the number of cells and the fluorescence intensity, respectively. The area painted out with gray is a result of the antibody reaction, and the solid line is a result of a negative control.

Fig. 8 shows a result of the antibody reaction of KUM2 cell (A) or BMSC cell (B) using an FITC-labeled anti-mouse CD45 antibody which was measured by a flow cytometer. The ordinate and abscissa show the number of cells and the fluorescence intensity, respectively. The area painted out with gray is a result of the antibody reaction, and the solid line is a result of a negative control.

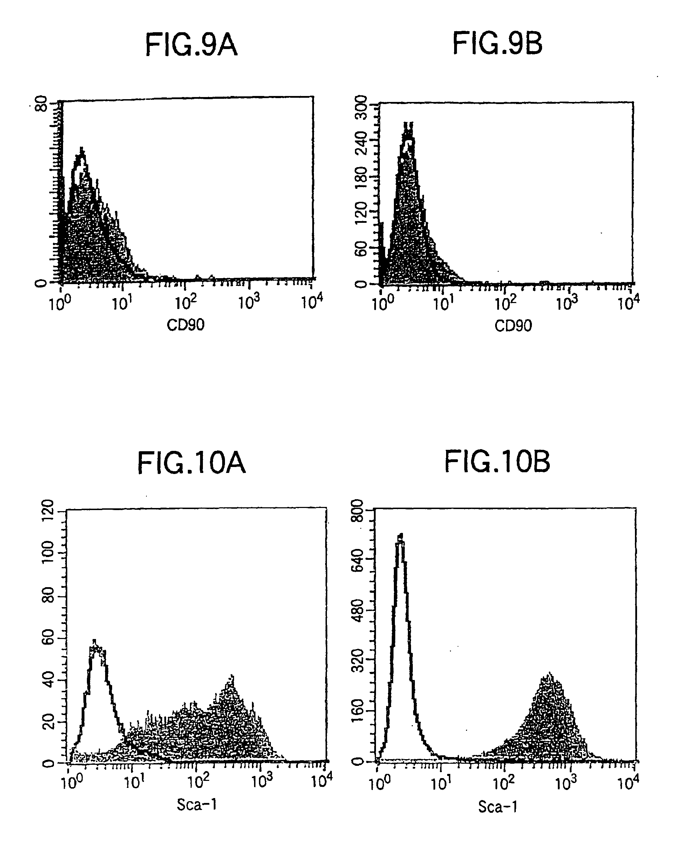

Fig. 9 shows a result of the antibody reaction of KUM2 cell (A) or BMSC cell (B) using an FITC-labeled anti-mouse CD90 antibody which was measured by a flow cytometer. The ordinate and abscissa show the number of cells and the fluorescence intensity, respectively. The area painted out with gray is a result of the antibody reaction, and the solid line is a result of a negative control.

Fig. 10 shows a result of the antibody reaction of KUM2 cell (A) or BMSC cell (B) using an FITC-labeled anti-mouse Ly6A/E(Sca-1) antibody which was measured by a flow cytometer. The ordinate and abscissa show the number of cells and the fluorescence intensity, respectively. The area painted out with gray is a result of the antibody reaction, and the solid line is a result of a negative control.

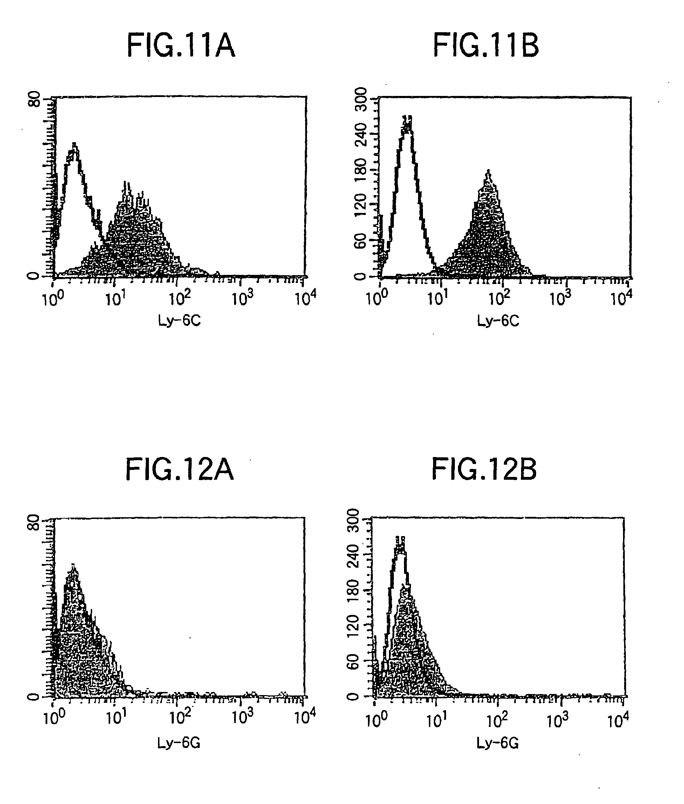

Fig. 11 shows a result of the antibody reaction of KUM2 cell (A) or BMSC cell (B) using an FITC-labeled anti-mouse Ly6c antibody which was measured by a flow cytometer. The ordinate and abscissa show the number of cells and the fluorescence intensity, respectively. The area painted out with gray is a result of the antibody reaction, and the solid line is a result of a negative control.

Fig. 12 shows a result of the antibody reaction of KUM2 cell (A) or BMSC cell (B) using an FITC-labeled anti-mouse Ly6g antibody which was measured by a flow cytometer. The ordinate and abscissa show the number of cells and the fluorescence intensity, respectively. The area painted out with gray is a result of the antibody reaction, and the solid line is a result of a negative control.

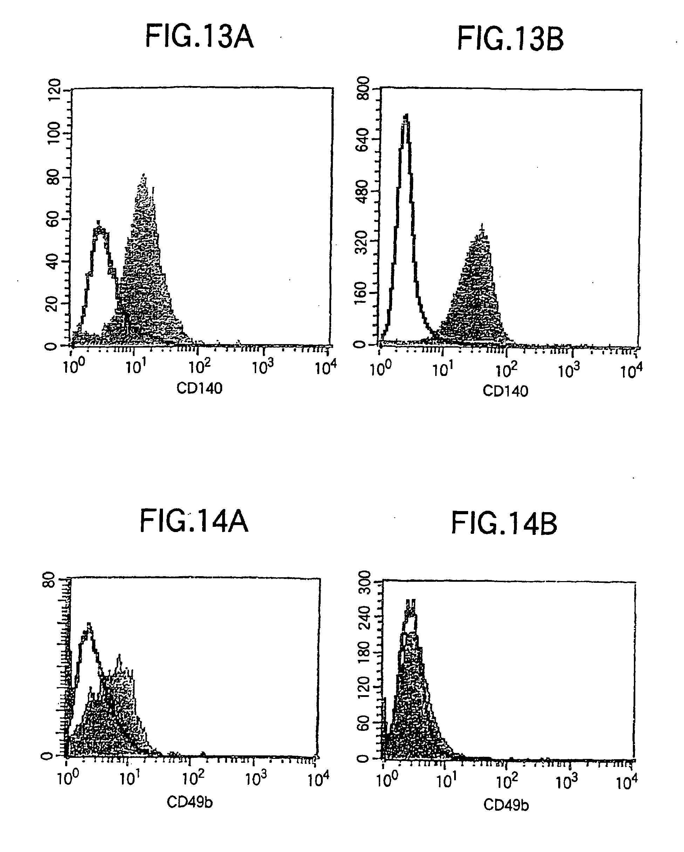

Fig. 13 shows a result of the antibody reaction of KUM2 cell (A) or BMSC cell (B) using a biotinylated anti-mouse CD140 antibody which was measured by a flow cytometer. The ordinate and abscissa show the number of cells and the fluorescence intensity, respectively. The area painted out with gray is a result of the antibody reaction, and the solid line is a result of a negative control.

Fig. 14 shows a result of the antibody reaction of KUM2 cell (A) or BMSC cell (B) using an FITC-labeled anti-mouse CD49b antibody which was measured by a flow cytometer. The ordinate and abscissa show the number of cells and the fluorescence intensity, respectively. The area painted out with gray is a result of the antibody reaction, and the solid line is a result of a negative control.

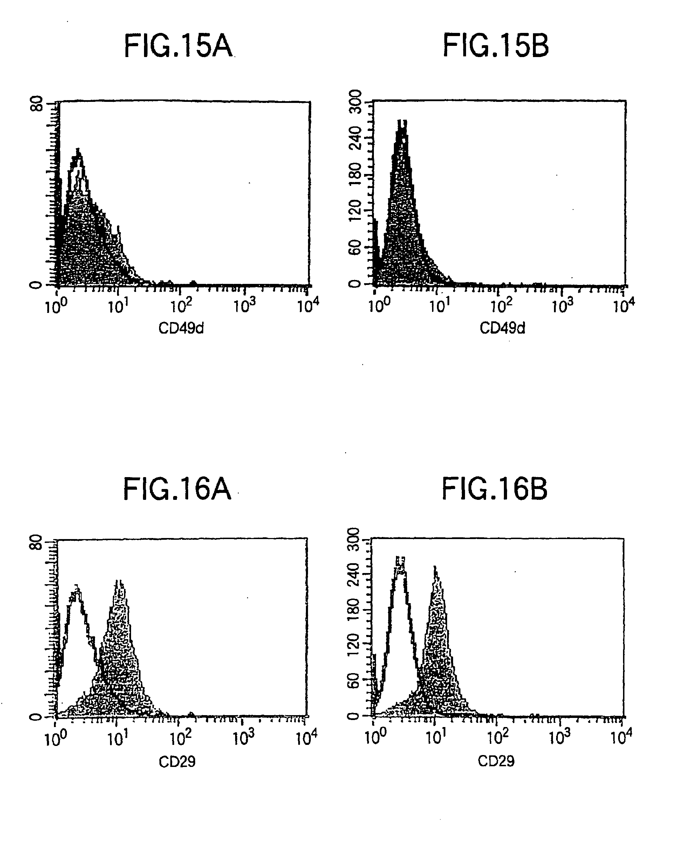

Fig. 15 shows a result of the antibody reaction of KUM2 cell (A) or BMSC cell (B) using an FITC-labeled anti-mouse CD49d antibody which was measured by a flow cytometer. The ordinate and abscissa show the number of cells and the fluorescence intensity, respectively. The area painted out with gray is a result of the antibody reaction, and the solid line is a result of a negative control.

Fig. 16 shows a result of the antibody reaction of KUM2 cell (A) or BMSC cell (B) using an FITC-labeled anti-mouse CD29 antibody which was measured by a flow cytometer. The ordinate and abscissa show the number of cells and the fluorescence intensity, respectively. The area painted out with gray is a result of the antibody reaction, and the solid line is a result of a negative control.

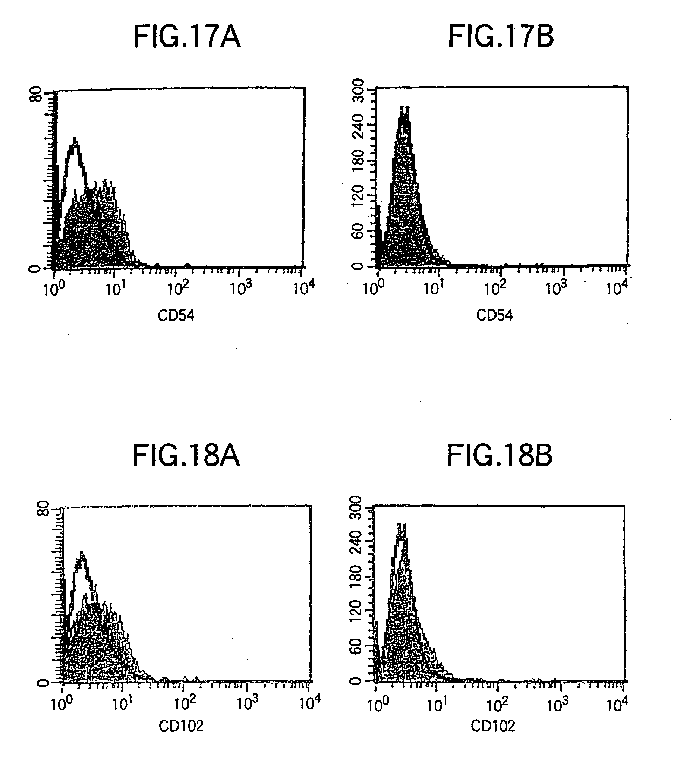

Fig. 17 shows a result of the antibody reaction of KUM2 cell (A) or BMSC cell (B) using an FITC-labeled anti-mouse CD54 antibody which was measured by a flow cytometer. The ordinate and abscissa show the number of cells and the fluorescence intensity, respectively. The area painted out with gray is a result of the antibody reaction, and the solid line is a result of a negative control.

Fig. 18 shows a result of the antibody reaction of KUM2 cell (A) or BMSC cell (B) using an FITC-labeled anti-mouse CD102 antibody which was measured by a flow cytometer. The ordinate and abscissa show the number of cells and the fluorescence intensity, respectively. The area painted out with gray is a result of the antibody reaction, and the solid line is a result of a negative control.

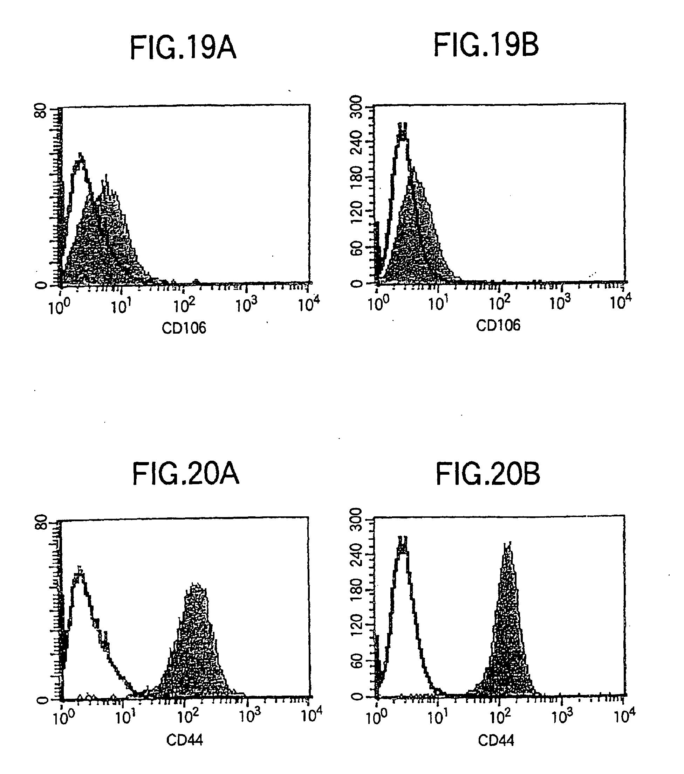

Fig. 19 shows a result of the antibody reaction of KUM2 cell (A) or BMSC cell (B) using an FITC-labeled anti-mouse CD106 antibody which was measured by a flow cytometer. The ordinate and abscissa show the number of cells and the fluorescence intensity, respectively. The area painted out with gray is a result of the antibody reaction, and the solid line is a result of a negative control.

Fig. 20 shows a result of the antibody reaction of KUM2 cell (A) or BMSC cell (B) using an FITC-labeled anti-mouse CD44 antibody which was measured by a flow cytometer. The ordinate and abscissa show the number of cells and the fluorescence intensity, respectively. The area painted out with gray is a result of the antibody reaction, and the solid line is a result of a negative control.

The present invention are illustrated below based on the following examples in more detail.

### BEST MODE FOR CARRYING OUT THE INVENTION

### Example 1

### Isolation and culture of bone marrow cells having the potential to differentiate into cardiomyocytes from mouse bone marrow:

Ten 5-weeks-old C3H/He mice were anesthetized with ether and sacrificed by cervical dislocation. Each mouse was laid in half-lateral position and sufficiently disinfected with 70% ethanol. The skin around the femur was widely opened and the quadriceps femoris covering the femur was excised with scissors. The femur was put out of the knee joint with scissors and the muscle on the back side of the femur was removed. Then, the femur was put out of the hip joint with scissors and taken out. After the muscle on the femur was removed with scissors to expose the whole femur, the femur was cut at both ends using scissors. A needle (23G, TERUMO) was attached to a 2.5 ml syringe and about 1.5 ml of IMDM containing 20% FCS was put into the syringe. The needle of the syringe was put into the femur from the cut end of the knee joint side and the culture medium was injected into the bone marrow, whereby bone marrow cells were pressed out of the bone into a test tube. The thus obtained cell were cultured in IMDM supplemented with 20% FCS, 100 mg/ml penicillin, 250 ng/ml streptomycin and 85 mg/ml amphotericin at 33°C using a 5% CO₂-incubator. As a result of a series of passages, the cells were homogenized into mesenchymal cells and hematopoietic cells disappeared.

After culturing for about 4 months under the above conditions, immortalized cells were selected and diluted to establish 192 cell lines respectively derived from single cells (hereinafter referred to as bone marrow-derived first passage immortalized cell lines). To each of these clone-derived cell lines was added 5-aza-C at a final concentration of 3 µM, and the cells were cultured for 24 hours. After culturing for further 2 weeks in IMDM, clones that produced spontaneously beating cells were selected. Among the bone marrow-derived first passage immortalized cell lines (192 cell lines), three cell lines were found to have the potential to differentiate into cardiomyocytes. One of three cell lines is KUM2. Hereinafter, unless otherwise indicated, the bone marrow cell KUM2 and mouse bone marrow-derived pluripotent stem cells (BMSC) described below were cultured in IMDM supplemented with 20% FCS, 100 mg/ml penicillin, 250 ng/ml streptomycin and 85 mg/ml amphotericin at 33°C using a 5% CO₂-incubator. When the KUM2 cells were exposed to 5-aza-C having a final concentration of 3 µM for 24 hours, nonspecific differentiation into spontaneously beating cardiomyocytes was induced. However, the frequency is very low (one or less per 10⁷ cells).

However, cells surrounding the spontaneously beating cells derived from the KUM2 cells were collected using a cloning syringe to observe at least two cells of the mouse bone marrow-derived pluripotent stem cells (BMSC) having a high proliferation potentiality (FERM BP-7043) and cells differentiated into cardiomyocytes by proliferation under limited times (hereinafter referred to as "cardiomyocyte precursor cells"). BMSC cells isolated by cloning syringe was cloned by selecting immortalized cells in the course of multiple passage. It was observed that the differentiation of the BMSC cells was induced at least 100 times as efficient as the parent cell line, KUM2. And to the cardiomyocyte precursor cells, 5-aza-C was added, followed by culturing for 24 hours, and further culturing in IMDM for 2-3 weeks, so that a larger number of spontaneously beating cells were efficiently obtained. The cardiomyocyte precursor cells showed mononuclear fibroblast-like morphology under the proliferation conditions and expression of myocardial contractile proteins was hardly observed. However, induction of final differentiation with 5-aza-C caused a remarkable change in the morphology of the cells.

About one week after the induction of differentiation, parts of cells showed enlargement of cytoplasm and showed a ball-like or stick-like appearance. Such cells began spontaneously beating afterwards but spontaneous beating was still rare at this stage. Two weeks after the induction of differentiation, the cells began spontaneously beating. The spontaneously beating cells connected lengthwise with one another to form myotube-like structures. Three weeks after the induction of differentiation, many cells were connected in a column and simultaneously contracted. Four weeks after the induction of differentiation, all of the directly connected cells on the culture dish showed simultaneous contraction and formed a myocardial tissue-like structure. The heart of a mouse contracts at a heart rate of 300-400 per minute. On the other hand, the cardiomyocytes differentiated from the cells derived from mouse adult bone marrow showed regular contraction at a rate of 120-250 per minute under the culture conditions.

### Example 2

### Characteristics of the Cardiomyocytes Derived from Mouse Bone Marrow Cells:

The spontaneously beating cardiomyocyte-like cells produced from the bone marrow cells were examined for the characteristics of cardiomyocytes.

Total RNAs were obtained from the bone marrow-derived first passage immortalized cell line, the mouse bone marrow-derived pluripotent stem cells (BMSC), and the cardiomyocytes derived from the cardiomyocyte precursor cells, which were obtained in Example 1, using Trizol Reagents (manufactured by GIBCO BRL). Then, first strand cDNAs were synthesized from the total RNAs as the substrates using SuperscriptII reverse transcriptase (manufactured by GIBCO BRL).

In order to examine the expression of cardiomyocyte-specific genes, quantitative PCR was carried out using the first strand cDNAs as the substrates and using the synthetic DNAs having the nucleotide sequences represented by SEQ ID NOS:33 to 58. As the cardiomyocyte-specific genes, ANP and BNP, which are natriurectic peptides, α-MHC and β-MHC, which are myosin heavy chains, α-skeletal actin and β-skeletal actin, which are actins, MLC-2a and MLC-2v, which are myosin light chains, and Nkx2.5/Csx, GATA4, TEF-1, MEF-2C, MEF-2D and MEF-2A, which are cardiomyocyte-specific transcription factors, were employed.

For the amplification of the above genes, the synthetic DNAs having the nucleotide sequences shown in the following SEQ ID NOS were respectively used: ANP, SEQ ID NOS:33 and 34; BNP, SEQ ID NOS:35 and 36; α-MHC, SEQ ID NOS:37 and 38; β-MHC, SEQ ID NOS:39 and 40; α-skeletal actin, SEQ ID NOS:41 and 42; β-skeletal actin, SEQ ID NOS:43 and 44; MLC-2a, SEQ ID NOS:45 and 46; MLC-2v, SEQ ID NOS:47 and 48; Nkx2.5/Csx, SEQ ID NOS:49 and 50; GATA4, SEQ ID NOS:51 and 52; TEF-1, SEQ ID NOS:53 and 54; MEF-2C, SEQ ID NOS:55 and 56; MEF-2D, SEQ ID NOS:57 and 58; and MEF-2A, SEQ ID NOS:59 and 60.

In cardiomyocytes produced by induced differentiation in vivo, myocardial contractile proteins have different isoforms according to the difference in stage, i.e., fetal period, new-born period or maturation period, or the difference in type, i.e., atrial or ventricular, so that the rate and energy efficiency of myocardial contraction may vary appropriately.

In the case of the bone marrow cells which differentiate into cardiomyocytes *in vitro,* α-skeletal actin was expressed at higher levels than α-cardiac actin in the expression pattern of isoforms; β-MHC was expressed at higher levels than α-MHC in the myosin heavy chain; and MLC-2v was expressed, whereas MLC-2a expression was not observed in the myosin light chain.

After the induction of differentiation of the bone marrow cells into cardiomyocytes in vitro, the expression of the natriuretic peptides, ANP and BNP, was observed. In view of the above expression pattern of myocardial contractile proteins, it is considered that the bone marrow cells which differentiated into cardiomyocytes in vitro have a phenotype specific to fetal ventricular cardiomyocytes.

In the bone marrow cells which differentiated into cardiomyocytes in vitro, the expression of genes coding for Nkx2.5/Csx, GATA4, MEF-2A, MEF-2C, MEF-2D or TEF-1 was observed. The genes coding for these transcription factors were not expressed in the bone marrow-derived first passage immortalized cell lines during proliferation. In the bone marrow-derived cardiomyocyte precursor cells during proliferation, the expression of genes coding for Nkx2.5/Csx, GATA4 or MEF-2C was observed. The expression of MEF-2A and MEF-2D was induced later with the induction of cardiomyogenic differentiation.

The action potentials of the bone marrow cells which differentiated into cardiomyocytes in vitro were recorded using glass microelectrodes. The cells were cultured in IMDM supplemented with 1.49 mM CaCl₂, 4.23 mM KCl and 25 mM HEPES (pH 7.4), and the action potentials of the cells were measured at 25°C under an inverted phase-contrast optic (Diaphoto-300, manufactured by Nikon). The glass microelectrodes were filled with 3M KCl and the electrode resistance was set at 15-30 Ω. in the glass microelectrodes. The membrane potentials were measured with current clamp mode using MEZ-8300 (manufactured by Nihon Kohden). The data were recorded on thermal recording papers using RTA-1100M (manufactured by Nihon Kohden). As a result, it was found that the bone marrow cells which differentiated into cardiomyocytes *in vitro* were classified into two types of action potentials: one is sinus node-like action potential and the other is ventricular myocyte-like action potential. These two type cells of action potentials had the following characteristics in common: (1) a long action potential duration, (2) a relatively shallow resting potential, (3) pacemaker-like slow depolarization of resting potential. The ventricular myocyte-like action potential showed the peak- and dome-like pattern having the phase 1 action potential. The sinus node-like action potential showed the action potential duration, diastolic membrane potential and action potential amplitude which are similar to those previously reported with the action potentials of sinus node cells of rabbits and rats. In comparison, the ventricular myocyte-like action potential had a tendency to show a deep resting membrane potential and a high action potential amplitude. During the 2-3 weeks after the induction of differentiation, the sinus node-like action potential was recorded for all the cells. The ventricular myocyte-like action potential was first recorded about 4 weeks after the induction of differentiation and its incidence gradually increased with the passage of time.

### Example 3

### Stimulation of cardiomyogenic differentiation using cytokine:

The following experiment was conducted to investigate the stimulating effect of cytokines on the cardiomyogenic differentiation of the mouse bone marrow cells having the potential to differentiate into cardiomyocytes induced by 5-aza-C.

The mouse bone marrow-derived pluripotent stem cells (BMSC) having the potential to differentiate into cardiomyocytes were plated into 60-mm culture dishes and 60 mm fibronectin-coated dishes (Becton Dickinson) at a density of 2×10⁴ cells/ml and cultured at 33°C in a 5% CO₂-incubator.

On the next day, 5-aza-C was added to each culture medium in a final concentration of 3 µM, followed by culturing with the following 3 different treatments, with addition of PDGF (culture dish A), both PDGF and retinoic acid (culture dish B) or without addition of any compound (culture dish C) (final concentration: PDGF, 10 ng/ml; retinoic acid, 10⁻⁹ M).

On the next day, the medium was replaced with a fresh medium to remove 5-aza-C therefrom. Then, PDGF was added to the culture dish A until the final concentration of PDGF came to be 10 ng/ml, while PDGF and retinoic acid were added to the culture dish B until the final concentrations of PDGF and retinoic acid came to be 10 ng/ml and 10⁻⁹ M, respectively. Two and four days thereafter, the medium was replaced and the PDGF or retinoic acid was further added.

Four weeks after the addition of the chemicals, the cell morphology was observed with a phase-contrast microscope. As a result, about 30% of the cells in the culture dish containing 5-aza-C alone differentiated into myotubes, while about 40% of the cells in the culture dish containing PDGF and about 50% of the cells in the culture dish containing PDGF together with retinoic acid differentiated into myotubes. In the three groups of the fibronectin-coated dishes, the ratio of the cells differentiated into myotubes was about 10% higher than in the three groups of the culture dishes.

RNAs were collected from the myotubes thus obtained. And genes expressed in the myotubes were analyzed with quantitative PCR analysis using the synthetic oligonucleotides represented by SEQ ID NOS:71 to 78. As a result, PDGF or retinoic acid promoted the expression of MyoD and fTnI genes relating to a skeletal muscle but not cTnI or ANP specifically relating to a myocardium. Next, mouse bone marrow-derived pluripotent stem cells (BMSC) having the potential to differentiate into cardiomyocytes were inoculated in a 60-mm culture dish at a density of 2×10⁴ cells/ml and cultured using an incubator at 33°C under 5% of CO₂.

On the next day, 5-aza-C was added to the liquid culture medium to give a final concentration of 3 µM. Furthermore, five treatments differing from each other were performed by adding FGF-8 to give a final concentration of 10 ng/ml (culture dish D); adding ET-1 to give a final concentration of 10 ng/ml (culture dish E); adding a midkine to give a final concentration of 10 ng/ml (Culture dish F); adding BMP4 to give a final concentration of 10 ng/ml (culture dish G); and adding no compound (culture dish H), followed by culturing.

On the next day, the medium was replaced by a fresh medium to eliminate 5-aza-C therefrom. Then, FGF-8 was added to the culture dish D to give a final concentration of 10 ng/ml; ET-1 was added to the culture dish E to give a final concentration of 10 ng/ml; the midkine was added to the culture dish F to give a final concentration of 10 ng/ml; and BMP4 was added to the culture dish G to give a final concentration of 10 ng/ml, followed by culturing. Two and four days thereafter, the medium was replaced and the FGF-8, ET-1, midkine or BMP4 was further added.

Four weeks after the addition of 5-aza-C, the cell morphology was observed with a phase-contrast microscope. As a result, about 30% of the cells in the culture dish containing 5-aza-C alone differentiated into myotubes, while about 50% of the cells in the culture dishes containing FGF-8, ET-1, midkine or BMP4 differentiated into myotubes respectively.

RNAs were collected from the myotubes thus obtained. And genes expressed in the myotubes were analyzed with quantitative PCR using the synthetic oligonucleotides represented by SEQ ID NOS:71 to 78. As a result, the FGF-8, ET-1, midkine and BMP4 each individually promoted the expression of cTnI and ANP gene which are myocardium-specific genes.

### Example 4

### Induction of differentiation of bone marrow-derived stem cells into cardiomyocytes using DMSO:

According to the method described in Example 1, mouse bone marrow-derived pluripotent stem cells (BMSC) having the potential to differentiate into cardiomyocytes were obtained and cultured for 24 hours in the presence of 10 µM DMSO instead of 3 µM 5-aza-C. The medium was replaced with IMDM, followed by culturing for 6 weeks.

As a result, the stem cells were induced to differentiate into beating cardiomyocytes. The produced cells expressed Nkx2.5/Csx and GATA4 genes and were found to be cardiomyocytes having the same properties as those obtained by the 5-aza-C treatment. This result indicates that cardiomyogenic differentiation requires demethylation of chromosomal DNA, which is a function common to 5-aza-C and DMSO.

### Example 5

### Demonstration that mouse bone marrow-derived pluripotent cells having the potential to differentiate into cardiomyocytes are pluripotent stem cells and cardiomyocyte precursor cells:

It was demonstrated above that the beating cells differentiated from the mouse bone marrow-derived pluripotent stem cell (BMSC) have the properties of cardiomyocytes. In this example, a single cell marking experiment was carried out to examine whether cardiomyocyte precursor cells are present in the mouse bone marrow-derived pluripotent stem cells (BMSC) having the potential to differentiate into cardiomyocytes, or whether more undifferentiated stem cells which can differentiate into not only cardiomyocytes, but also, for example, adipocytes and other cell types are present.

Specifically, a GFP gene was inserted into a virus vector and the vector was transfected into a cell for labeling prior to induction of differentiation, and the labeled cell was induced to differentiate to observe what kind of cell is produced by differentiation.

First, retrovirus vector plasmid GAR3-GFP which expresses the GFP gene products and plasmid vector pCMV-Eco which expresses the Ecotropic gene products were treated according to the alkali neutralization method and the PEG precipitation method described in *Molecular Cloning, A Laboratory Manual,* 2nd ed. to obtain DNAs of high purity.

One day before DNA transfection, 293 cells carrying the gag and pol genes which had reached confluence were passaged into a 10-cm dish by 1/5 dilution and cultured overnight at 37°C in a 5% CO₂-incubator.

Transfection was carried out as follows.

GAR3-GFP retrovirus vector plasmid DNA (15 µg) and pCMV-Eco plasmid vector DNA (5 µg) were dissolved in 0.5 ml of 250 mM CaCl₂ (pH 6.95). The resulting solution was added dropwise to a 15 ml tube containing 0.5 ml of 2x BBS (50 mM BES (N,N-bis(2-hydroxyethl)-2-aminoethanesulfonic acid), 280 mM NaCl and 1.5 mM Na₂HPO₄ (pH 6.95)) and the tube was allowed to stand at room temperature for 10 minutes. The resulting DNA solution was added dropwise to the 293 cell culture prepared on the preceding day, followed by culturing at 37°C in a 5% CO₂-incubator. On the next day, the medium was replaced with a fresh medium, followed by culturing at 37°C in the 5% CO₂-incubator.

Two days after the medium replacement, the culture supernatant was filtered through a 0.45 µm filter (manufactured by Millipore) to recover a solution containing the virus vector. The obtained solution was diluted to 10⁻¹, 10⁻², 10⁻³, 10⁻⁴ and 10⁻⁵ with IMDM.

The mouse bone marrow-derived pluripotent stem cells having the potential to differentiate into cardiomyocytes into which the virus vector was to be introduced were plated into 6-well dishes at a density of 2×10⁴ cells/well on the day before virus infection.

To the diluted virus vector solution, hexadimethine bromide (polybrene) (manufactured by Sigma) was added to give a final concentration of 8 µg/ml. After 2 ml of the culture supernatant of the mouse bone marrow-derived pluripotent stem cells (BMSC) having the potential to differentiate into cardiomyocytes was replaced with 2 ml of the virus solution, culturing was carried out at 33°C in a 5% CO₂-incubator. Five hours later, the culture supernatant was replaced with a fresh IMDM, followed by culturing at 33°C in the 5% CO₂-incubator.

After culturing for 2 days, the cells were observed for GFP expression by a fluorescence microscope to obtain cell populations containing one GFP-positive cell in 1000 cells.

The obtained cells were plated into 35 mm glass base dishes (manufactured by Asahi Techno Glass) at a density of 8×10³ cells/dish followed by culturing at 33°C in a 5% CO₂-incubator.

On the next day, 5-aza-C (manufactured by Sigma), PDGF-BB (manufactured by Peprotech) and all trans retinoic acid (manufactured by Sigma) were added to the dishes to give final concentrations of 3 µM, 10 ng/ml and 10⁻⁹ M, respectively. Two days and four days after the addition, the medium was replaced with a fresh medium and PDGF-BB (hereinafter referred to as "PDGF") and all trans retinoic acid were added at the same concentrations as above.

Four weeks after, the cultures were observed under a fluorescence microscope to examine the mode of differentiation of the GFP-positive cells. As a result, the following three kinds of cell populations were observed; cell populations in which all the GFP-positive cells were cardiomyocytes; cell populations in which cardiomyocytes and undifferentiated stem cells were GFP-positive; and cell populations in which cardiomyocytes, adipocytes and undifferentiated stem cells were GFP-positive. It has thus been found that differentiation is stochastically derived from pluripotent stem cells through myocardial stem cells and then cardiomyocyte precursor cells. This result also indicates that the mouse bone marrow cells having the potential to differentiate into cardiomyocytes comprise pluripotent stem cells.

### Example 6

### Promotion of differentiation info cardiomyocytes by forced expression of transcription factors:

The following experiment was carried out to examine the effect of the forced expression of transcription factors relating to cardiomyogenic differentiation on the cardiomyogenic differentiation of the bone marrow-derived pluripotent stem cells (BMSC) having the potential to differentiate into mouse cardiomyocytes.

That is, the Nkx2.5/Csx or GATA4 gene was introduced into the cells using a virus vector prior to induction of differentiation, and then the cells were induced to differentiate to examine the efficiency of cardiomyogenic differentiation.

In order to express the Nkx2.5/Csx, Nkx2.5/Csx was inserted into retrovirus vector plasmid pCLNCX (manufactured by Imgenex) to prepare pCLNC-Nkx2.5/Csx.

Furthermore, in order to express GATA4, GATA4 was inserted into plasmid pCLPCX in which the G418-resistant gene portion in retrovirus vector plasmid pCLNCX (manufactured by Imgenex) had been replaced with puromycin-resistant genes, to prepare pCLPC-GATA4. The retrovirus vector plasmids pCLNC-Nkx2.5/Csx and pCLPC-GATA4 and plasmid vector pCMV-Eco (manufactured by Imgenex) which expresses the Ecotropic gene were treated according to the alkali neutralization method and the PEG precipitation method described in *Molecular Cloning, A Laboratory Manual,* 2nd ed., *etc.* to obtain DNAs having high purity.

One day before DNA transfection, 293 cells carrying the gag and pol gene which had reached confluence were passaged into a 10-cm dish by 1/5 dilution followed by culturing overnight at 37°C in a 5% CO₂-incubator.

Transfection was carried out as described below.

15 µg of retrovirus vector DNA, pCLNC-Nkx2.5/Csx or pCLPC-GATA4, and 5 µg of plasmid vector, pCMV-Eco, were added and dissolved in 0.5 ml of 250 mM CaCl₂ (pH 6.95). The resulting solution was added dropwise to a 15 ml tube containing 0.5 ml of 2×BBS (50 mM BES (N,N-bis(2-hydroxyethl)-2-aminoethanesulfonic acid), 280 mM NaCl and 1.5 mM Na₂HPO₄ (pH 6.95)) and the tube was allowed to stand at room temperature for 10 minutes. The resulting DNA solution was added dropwise to the 293 cell culture prepared on the preceding day, followed by culturing at 37°C in a 5% CO₂-incubator. On the next day, the medium was replaced with a fresh medium, followed by culturing at 37°C in the 5% CO₂-incubator.

Two days after the medium replacement, the culture supernatant was filtered through a 0.45 µm filter (manufactured by Millipore) to recover a solution containing the virus vector.

The mouse bone marrow-derived pluripotent stem cells (BMSC) having the potential to differentiate into cardiomyocytes into which the virus vector was to be introduced were plated into 6-well dishes at a density of 2×10⁴ cells/well on the day before virus infection.

To the obtained virus vector solution, hexadimethrine bromide (polybrene) (manufactured by Sigma) was added to give a final concentration of 8 µg/ml. The culture medium was replaced with the culture medium for the mouse bone marrow-derived pluripotent stem cells (BMSC) having the potential to differentiate into cardiomyocytes, followed by culturing at 33°C in a 5% CO₂-incubator. Five hours later, the medium was replaced with a fresh IMDM, followed by culturing at 33°C in the 5% CO₂-incubator, and further culturing for 2 days.

G418 was added to the cells infected with the virus produced by transferring pCLNC-Nkx2.5 and pCMV-Eco to give a final concentration of 300 µg/ml, followed by culturing for further 7 days.

Separately, puromycin was added to the cells infected with the virus produced by transferring pCLPC-GATA4 and pCMV-Eco to give a final concentration of 300 ng/ml, followed by culturing for further 7 days.

During this period, both cells partly died and were detached from the dish. The surviving cells were suspended with trypsin followed by plating into new culture dishes.

The obtained stable transformants for expression of Nkx2.5/Csx or GATA4 were induced for differentiation by the method in the above Example 3, and thus the differentiation efficiency into cardiomyocytes was examined.

The NKx2.5 forced expressing bone marrow cells (BMSC-Nkx2.5) having the potential to differentiate into cardiomyocytes and the GATA4 forced expressing bone marrow cells (BMSC-GATA4) having the potential to differentiate into cardiomyocytes were plated into 60-mm culture dishes at a density of 2×10⁴ cells/ml, followed by culturing at 33°C in a 5% CO₂-incubator. On the next day, 5-aza-C was added to each culture medium to give a final concentration of 3 µM After continuing the culturing at 33°C in a 5% CO₂-incubator for further 24 hours, the medium was replaced with a fresh medium to eliminate 5-aza-C, followed by culturing for additional 4 weeks. When observed with a phase-contrast microscope, the number of myotube showed no large change caused by the forced expression of Nkx2.5/Csx or GATA4. Next, RNAs were collected from the myotubes thus obtained and genes expressed in the myotubes were analyzed with quantitative PCR using the synthetic oligonucleotides represented by SEQ ID NOS:71 to 78. As a result, it was observed that the forced expression of Nkx2.5/Csx or GATA4 promoted the expression of cTnI and ANP which are myocardium-specific genes.

To simultaneously express both of the Nkx2.5/Csx and GATA4 genes in bone marrow cells having the potential to differentiate into cardiomyocytes, a retrovirus vector plasmid pCLPC-GATA4 was treated as described above and bone marrow cells (BMSC-NKx2.5) with the forced expression of Nkx2.5/Csx having the potential to differentiate into cardiomyocytes were infected with the recombinant virus thus constructed. Next, puromycin was added to give a final concentration of 300 ng/ml to obtain a drug-tolerant clone (BMSC-Nkx2.5-GATA4).

The Nkx2.5/Csx and GATA4 co-forced expressing bone marrow cells (BMSC-Nkx2.5-GATA4) having the potential to differentiate into cardiomyocytes were plated into a 60-mm culture dish at a density of 2×10⁴ cells/ml, followed by culturing at 33°C in a 5% CO₂-incubator.

On the next day, 5-aza-C was added to the culture medium to give a final concentration of 3 µM. After culturing at 33°C in a 5% CO₂-incubator for further 24 hours, the medium was replaced with a fresh medium to eliminate 5-aza-C, followed by culturing for 4 weeks. When observed with a phase-contrast microscope, the number f myotube showed no large change caused by the forced expression of the Nkx2.5/Csx and GATA4 genes. However, the number of beating cardiomyocyte was 50 times or more elevated than bone marrow cells with no forced expression of these genes having the potential to differentiate into cardiomyocytes. Next, RNAs were collected from the myotubes thus obtained and genes expressed in the myotubes were analyzed with quantitative PCR using the synthetic oligonucleotides represented by SEQ ID NOS:71 to 78. As a result, it was observed that the forced expression of Nkx2.5/Csx and GATA4 promoted the expression of cTnI and ANP which are myocardium-specific genes.

### Example 7

### Promotion of differentiation into cardiomyocytes by combination of the forced expression of transcriptional factors with cytokines:

By combining the above-described transcriptional factors (Nkx2.5/Csx and GATA4) promoting the differentiation into cardiomyocytes with cytokines (FGF-8, ET-1, midkine and BMP4), effects on the differentiation into cardiomyocytes were analyzed.

The Nkx2.5/Csx and GATA4 co-forced expressing bone marrow cells (BMSC-Nkx2.5-GATA4) having the potential to differentiate into cardiomyocytes were plated into a 60-mm culture dish at a density of 2×10⁴ cells/ml and cultured at 33°C in a 5% CO₂-incubator.

On the next day, 5-aza-C was added to the culture medium to give a final concentration of 3 µM. Furthermore, 5 treatments differing from each other were carried out by adding FGF-8 to give a final concentration of 10 ng/ml (culture dish I); adding ET-1 to give a final concentration of 10 ng/ml (culture dish J); adding midkine to give a final concentration of 10 ng/ml (culture dish K); adding BMP4 to give a final concentration of 10 ng/ml (culture dish L); and adding nothing (culture dish M), followed by culturing.

On the next day, the medium was replaced with a fresh medium to eliminate 5-aza-C. Then, FGF-8 was added to the culture dish I to give a final concentration of 10 ng/ml; ET-1 was added to the culture dish J to give a final concentration of 10 ng/ml; midkine was added to the culture dish K to give a final concentration of 10 ng/ml; and BMP4 was added to the culture dish L to give a final concentration of 10 ng/ml, followed by culturing. Two and four days thereafter, furthermore, the medium was replaced, and the FGF-8, ET-1, midkine or BMP4 was added.

Four weeks after the addition of 5-aza-C, the cell morphology was observed with a phase-contrast microscope. As a result, about 30% of the cells in the culture dish containing 5-aza-C alone were converted into myotubes, while about 50% of the cells in the culture dishes containing FGF-8, ET-1, midkine or BMP4 differentiated into myotubes respectively. On the other hand, the addition of FGF-8, ET-1, midkine or BMP4 caused no increase in beating cardiomyocytes.

From the myotubes thus obtained, RNAs were collected and genes expressed in the myotubes were subjected to quantitative PCR analysis using the synthetic oligonucleotides represented by SEQ ID NOS:71 to 78. As a result, the FGF-8, ET-1, midkine and BMP4 did not further promote the expression of cTnI and ANP which had been promoted by the forced expression of Nkx2.5/Csx and GATA4.

### Example 8

### Transplantation of mouse having the potential to differentiate into cardiomyocytes into heart:

In order to examine whether or not bone marrow cells having the potential to differentiate into cardiomyocytes would differentiate into myocardia and thus take into the heart, the GFP labeled bone marrow cells (BMSC-GFP) having the potential to differentiate into cardiomyocytes as prepared in Example 5 were employed as donor cells for the transplantation into mouse. Specifically, the following procedure was performed. The GFP-labeled BMSCs were transiently treated with 5-aza-C for 24 hours, then suspended in PBS to give a concentration of 1×10⁸ cells/ml and stored on ice until immediately before the transplantation. It had been confirmed by 0.05% erythrosine-staining that BMSCs could survive at a ratio of about 95%.

On the other hand, the recipient C3H/He mice (available from Charles River Japan) were anesthetized with ether, and the anesthesia was maintained by intraperitoneally administering 30 mg of thiopental using a Terumo syringe (1 ml) manufactured by Terumo Corp. The legs of each mouse were fixed on a cork board with tape, and its upper jaw was also fixed on the cork board with rubber in such a manner that the neck leaned back. At this stage, electrocardiography electrodes were put into both upper limbs and right side lower limb to monitor the electrocardiogram. Next, the cervix was incised about 1 cm along the trachea using Mayo scissors (NONAKA RIKAKI CO., LTD, NK-174-14), the thyroid gland was stripped to the right and left sides using a baby cotton swab manufactured by Hakujuji, and then muscles around the trachea were incised using micro scissors (NONAKA RIKAKI CO., LTD, NY-334-08) to expose the trachea. Next, the trachea was incised in about 1 mm width using a micro-feather (a surgical knife), a needle of Surflow Flash (22G) manufactured by Terumo deformed into J-shape was inserted into the opening and taken out from the oral cavity, and then the syringe of Surflow Flash (20G) was inserted into the trachea using the needle as a guide. By connecting a respirator (MODEL SN-480-7, manufactured by SHINANO SEISAKUSHO) to the syringe, 100% oxygen was flowed at a rate of 1 ml/minute to start artificial respiration with a tidal volume of 1 ml and a respiration frequency of 120/minute. Since air leaks out from the guide needle-inserted opening, the skin around the trachea was closed by covering the trachea using mosquito forceps (manufactured by NONAKA RIKAKI CO., LTD.). Next, a region of about 2 cm from the sternal pedicel toward the cervix was incised using Mayo scissors and then the sternum was incised about 2 cm from the sternal pedicel toward the cervix. Bleeding was stopped using a bipolar electric knife, and then a 30G needle (metal hub exchange needle N730) manufactured by GL Science was connected to the Terumo syringe (1 ml) manufactured by Terumo Corp and 0.1 ml of a solution prepared by suspending the donor cells in PBS was injected into the apical region. Next, the sternum and the skin were closed using 4-0 ETHIBOND X761 manufactured by ETHICON, and the skin of the cervix was closed using the same suture. After confirmation of the turn up of spontaneous respiration, the respirator was taken out, and an infant warmer was heated to 37°C to wait vigilance of the animal therein. Also, the procedure of this test was carried out using DESIGN FOR VISON 4.5× SURGICAL TELESCOPES.

Tissues were taken out from the mouse 77 days after the transplantation, fixed with 10% formalin and embedded in paraffin. The embedded tissues were sliced with a microtome into pieces of 6 µm in thickness and adhered to slide glasses which had been coated with poly-L-lysine. After eliminating paraffin by immersing in 10% xylene, the samples were washed with ethanol and then immersed in 0.3% H₂O₂ for 30 minutes, followed by a pretreatment for the antibody reaction.

Then, the samples were washed with PBS and blocked by reacting with a 5% normal swine serum solution. After blocking, the samples were washed with PBS and then subjected to the antibody reaction by allowing to stand at 4°C overnight together with a mouse anti-GFP monoclonal antibody (manufactured by CLONTECH). After washing with PBS, the samples were allowed to react with a peroxidase-labeled dextran-bonded goat anti-mouse immunoglobulin antibody (manufactured by DACO) at room temperature for 30 minutes. After washing with PBS again, a coloring solution (10 µg/ml 3,3'-diaminobenzidine (DAB) tetrahydrochloride, 0.01% H₂O₂, 0.05 M Tris-HCl (pH 6.7)) was added and allowed to react for about 10 minutes. Then, the reaction mixture was washed with PBS to stop the reaction. Furthermore, the slide glasses were stained with methyl green. The part of continuous pieces were stained with hematoxylin/eosin to clarify the morphology of the tissue pieces.

As a result, GFP-positive cells were observed in the cardiomyocytes and the vascular endothelial cells.

Thus, it can be concluded that the transplanted mouse bone marrow cells had differentiated into the cardiomyocytes and the vascular endothelial cells.

### Example 9

### Promotion of differentiation into cardiomyocytes by cultured cardiomyocyte-derived factor:

As shown in Example 8, the bone marrow cells (BMSC) having the potential to differentiate into cardiomyocytes were differentiated into the cardiomyocytes when transplanted into the heart. This result suggests that cardiomyocytes per *se* expresses a factor inducing the differentiation of bone marrow cells into cardiomyocytes. To examine this hypothesis, a mouse fetal heart was taken out from a C3H/He mouse on the day 16 of pregnancy and a primary culture cell line of cardiomyocytes (hereinafter referred to as the "cultured cardiomyocytes") was established in accordance with a publicly known method (*Development of Method for Studying Heart and Blood,* ed. by Setsuro Ebashi, Gakkai Shuppan Senta, (1983)).

To examine whether or not a factor secreted from the cultured cardiomyocytes has an activity of promoting heart differentiation, 5×10⁶ cultured cardiomyocytes were cultured in a culture dish for 72 hours. Next, the culture supernatant was filtered through a 0.45 µm filter (manufactured by Millipore). The culture supernatant thus filtered was mixed with the equivalent amount of a medium to give a culture medium (hereinafter referred to as the "conditioned medium") containing the factor secreted from the cultured cardiomyocytes.

Bone marrow cells (BMSC) having the potential to differentiate into cardiomyocytes or Nkx2.5 and GATA4 forced expressing bone marrow cells (BMSC-Nkx2.5-GATA4) having the potential to differentiation into cardiomyocytes were cultured 6-cm culture dishes at a density of 1×10⁵ cells and then the medium was replaced with the conditioned medium. At this point, 5-aza-C was added to give a final concentration of 3 µM. On the next day, the medium was replaced with the fresh conditioned medium, followed by culturing for further 4 weeks. During this period, the medium was replaced with the fresh conditioned medium once 3 days. Thus, it was observed that myotubes derived from the bone marrow cell (BMSC) having the potential to differentiate into cardiomyocytes showed no increase but the expression of the two myocardium-specific genes (ANP and cTnI) was promoted by the addition of the conditioned medium. In case of the Nkx2.5 and GATA forced expressing bone marrow cells (BMSC-Nkx2.5-GATA4) having the potential to differentiate into cardiomyocytes, on the other hand, the myotubes showed no increase and the expression of the two myocardium-specific genes (ANP and cTnI) was promoted at the same level as in Nkx2.5 and GATA4 by the addition of the conditioned medium, showing no promoting effect.

Next, it was examined whether or not cardiomyocyte-expressing extracellular matrix (ECM) has an activity of promoting the differentiation into cardiomyocytes, culture dishes wherein cardiomyocytes had been cultured were treated with 0.45% trypsin/EDTA for about 30 minutes to eliminate the cardiomyocytes. Thus, culture dishes coated with the extracellular matrix of the cultured cardiomyocytes (hereinafter referred to as the "ECM-coated dishes") were prepared. Subsequently, bone marrow cells (BMSC) having the potential to differentiate into cardiomyocytes or compulsively both Nkx2.5 and GATA 4 genes-expressed bone marrow cells (BMSC-Nkx2.5-GATA4) having the potential to differentiate into cardiomyocytes were cultured in these 6-cm culture dishes at a density of 1×10⁵ cells and then 5-aza-C was added to give a final concentration of 3 µM. On the next day, the medium was replaced with a fresh medium to eliminate 5-aza-C and the culture was continued for further 4 weeks. During this period, the medium was replaced with a fresh medium once 3 days. Thus, it was observed that myotubes derived from the bone marrow cells (BMSC) having the potential to differentiate into cardiomyocytes showed no increase but the expression of the two myocardium-specific genes (ANP and cTnI) was promoted by the coated dish. In case of the compulsively both Nkx2.5 and GATA4 genes-expressed bone marrow cells (BMSC-Nkx2.5-GATA4) having the potential to differentiate into cardiomyocytes, on the other hand, the myotubes showed no increase and the expression of the two myocardium-specific genes (ANP and cTnI) was promoted at the same level as in Nkx2.5 and GATA4 by the addition of the conditioned medium, showing no promoting effect.

Next, 2×10⁴ cultured cardiomyocytes were co-cultured together with 8×10⁴ bone marrow cells (BMSC) having the potential to differentiate into cardiomyocytes or 8×10⁴ compulsively both Nkx2.5 and GATA4 genes-expressed bone marrow cells (BMSC-Nkx2.5-GATA4) having the potential to differentiate into cardiomyocytes in 6-cm culture dishes. To distinguish the cultured cardiomyocytes from the bone marrow cells, the two types of bone marrow cells (BMSC and BMSC-Nkx2.5-GATA4) were labeled with GFP as in Example 5. On the next day after the co-culturing, 5-aza-C was added to give a final concentration of 3 µM. On the next day, the medium was replaced with a fresh medium to eliminate 5-aza-C, followed by culturing for further 4 weeks. During this period, the medium was replaced with a fresh medium once 3 day. As a result, beating cardiomyocytes were increased about 10 times or more than the case wherein BMSC or BMSC-Nkx2.5-GATA4 were cultured alone. Thus, it was found that the efficiency of the differentiation into cardiomyocytes can be elevated 500 times or more by combining the forced expression of the Nkx2.5 and GATA4 genes with the co-culturing with cardiomyocytes.

### Example 10

### Surface of surface antigens of cells cells and BMSCs:

Surface antigens of KUM2 cells and BMSCs were analyzed to clearly differentiate KUM2 cells from BMSCs and develop a method for efficiently isolating and purifying cells having the potentiality of forming myocardium from bone marrow.

The surface antigens employed in the analysis included 20 antigens, i.e., CD105, Flk-1, CD31 and CD144 known as surface antigens of vascular endothelial cells, CD34, CD117(c-kit), CD14, CD45, CD90, Ly6A/E(Sca-1), Ly6c and ly6g known as surface antigens in hematopoietic cells, CD140 known as surface antigens of mesenchymal cells, integrins CD49b, CD49d and CD29 and matrix receptors CD54, CD102, CD106 and CD44.

First, 1×10⁴ KUM2 cells or 1×10⁴ BMSC cells were pipetted into a 96-well U-shaped plate. An anti-mouse CD105 antibody (manufactured by Pharmingen), which had been biotin-labeled by a publicly known method (*Enzyme Antibody Technique,* Gakusai Kikaku (1985)), was added to a buffer for FACS (1% BSA-PBS, 0.02% EDTA, 0.05% NaN₃, pH 7.4), then added to the wells and allowed to react on ice for 30 minutes. As a negative control, rat IgG2a, K-purified antibody (manufactured by Pharmingen) was used. After washing with the buffer twice, 20 µl of streptoavidin-PE (manufactured by Nippon Becton Dickinson) was added. Then the mixture was allowed to react in the dark on ice for 30 minutes, washed with the buffer thrice and suspended in 500 µl of the buffer. The fluorescence intensity was measured with a flow cytometer and it was examined whether or not the fluorescence intensity was increased by adding the antibody. The results are shown in Fig. 1. As a result, it was found that the KUM2 cells and the BMSC cells were both CD105-negative.

Regarding the occurrence of the expression of the Flk-1 antigen, an antibody reaction was carried out in the manner similar to that described above, using a biotinylated anti-mouse Flk-1 antibody (PM-28181D, manufactured by Pharmingen), followed by measurement with a flow cytometer. The results are shown in Fig. 2. As a result, it was found that the KUM2 cells and the BMSC cells were both Flk-1-negative.

Regarding the occurrence of the expression of the CD31 antigen, an antibody reaction was carried out using an FITC-labeled anti-mouse CD31 antibody (PM-01954D, manufactured by Pharmingen), followed by measurement with a flow cytometer. The results are shown in Fig. 3. As a result, it was found that the KUM2 cells and the BMSC cells were both CD31-negative.

Regarding the occurrence of the expression of the CD144 antigen, an antibody reaction was carried out using a biotinylated anti-mouse CD144 antibody (PM-28091D, manufactured by Pharmingen) followed by measurement with a flow cytometer. The results are shown in Fig. 4. As a result, it was found that the KUM2 cells were CD144-negative, while the BMSC cells were CD144-weak positive.

Regarding the occurrence of the expression of the CD34 antigen, an antibody reaction was carried out using an FITC-labeled anti-mouse CD34 antibody (PM-09434D, manufactured by Pharmingen), followed by measurement with a flow cytometer. The results are shown in Fig. 5. As a result, it was found that the KUM2 cells were CD34-negative, while the BMSC cells were a mixture of CD34-positive cells and CD34-negative cells.

Regarding the occurrence of the expression of the CD117(c-kit) antigen, an antibody reaction was carried out using an FITC-labeled anti-mouse CD117 antibody (PM-01904D, manufactured by Pharmingen), followed by measurement with a flow cytometer. The results are shown in Fig. 6. As a result, it was found that the KUM2 cells were CD117-negative, while the BMSC cells were CD1l7-positive

Regarding the occurrence of the expression of the CD14 antigen, an antibody reaction was carried out using an FITC-labeled anti-mouse CD14 antibody (PM-09474, manufactured by Pharmingen), followed by measurement with a flow cytometer. The results are shown in Fig. 7. As a result, it was found that the KUM2 cells were CD14-positive, while the BMSC cells were CD14-negative.

Regarding the occurrence of the expression of the CD45 antigen, an antibody reaction was carried out using an FITC-labeled anti-mouse CD45 antibody (PM-01114, manufactured by Pharmingen), followed by the measurement with a flow cytometer. The results are shown in Fig. 8. As a result, it was found that the KUM2 cells and the BMSC cells were both CD45-negative.

Regarding the occurrence of the expression of the CD90 antigen, an antibody reaction was carried out using an FITC-labeled anti-mouse CD90 antibody (PM-22214, manufactured by Pharmingen), followed by measurement with a flow cytometer. The results are shown in Fig. 9. As a result, it was found that the KUM2 cells and the BMSC cells were both CD90-negative.

Regarding the occurrence of the expression of the Ly6A/E(Sca-1) antigen, an antibody reaction was carried out using an FITC-labeled anti-mouse Ly6A/E(Sca-1) antibody (PM-01164A, manufactured by Pharmingen), followed by measurement with a flow cytometer. The results are shown in Fig. 10. As a result, it was found that the KUM2 cells and the BMSC cells were both Ly6A/E(Sca-1)-positive.

Regarding the occurrence of the expression of the Ly6c antigen, an antibody reaction was carried out using an FITC-labeled anti-mouse Ly6c antibody (PM-01152, manufactured by Pharmingen), followed by measurement with a flow cytometer. The results are shown in Fig. 11. As a result, it was found that the KUM2 cells and the BMSC cells were both Ly6c-positive.

Regarding the occurrence of the expression of the Ly6g antigen, an antibody reaction was carried out using an FITC-labeled anti-mouse Ly6g antibody (PM-01214, manufactured by Pharmingen), followed by the measurement with a flow cytometer. The results are shown in Fig. 12. As a result, it was found that the KUM2 cells and the BMSC cells were both Ly6g-negative.

Regarding the occurrence of the expression of the CD140 antigen, an antibody reaction was carried out using a biotinylated anti-mouse CD140 antibody (PM-28011A, manufactured by Pharmingen), followed by measurement with a flow cytometer. The results are shown in Fig. 13. As a result, it was found that the KUM2 cells and the BMSC cells were both CD140-positive.

Regarding the occurrence of the expression of the CD49b antigen, an antibody reaction was carried out using an FITC-labeled anti-mouse CD49b antibody (PM-09794, manufactured by Pharmingen), followed by measurement with a flow cytometer. The results are shown in Fig. 14. As a result, it was found that the KUM2 cells were CD49b-positive, while the BMSC cells were CD49b-negative.

Regarding the occurrence of the expression of the CD49d antigen, an antibody reaction was carried out using an FITC-labeled anti-mouse CD49d antibody (PM-01274, manufactured by Pharmingen), followed by measurement with a flow cytometer. The results are shown in Fig. 15. As a result, it was found that the KUM2 cells and the BMSC cells were both CD49d-negative.

Regarding the occurrence of the expression of the CD29 antigen, an antibody reaction was carried out using an FITC-labeled anti-mouse CD29 antibody (PM-22634, manufactured by Pharmingen), followed by measurement with a flow cytometer. The results are shown in Fig. 16. As a result, it was found that the KUM2 cells and the BMSC cells were both CD29-positive.

Regarding the occurrence of the expression of the CD54 antigen, an antibody reaction was carried out using an FITC-labeled anti-mouse CD54 antibody (PM-01544, manufactured by Pharmingen), followed by measurement with a flow cytometer. The results are shown in Fig. 17. As a result, it was found that the KUM2 cells were CD54-positive, while the BMSC cells were CD54-negative.

Regarding the occurrence of the expression of the CD102 antigen, an antibody reaction was carried out using an FITC-labeled anti-mouse CD102 antibody (PM-01804, manufactured by Pharmingen), followed by measurement with a flow cytometer. The results are shown in Fig. 18. As a result, it was found that the KUM2 cells and the BMSC cells were both CD102-negative.

Regarding the occurrence of the expression of the CD106 antigen, an antibody reaction was carried out using an FITC-labeled anti-mouse CD106 antibody (PM-01814 manufactured by Pharmingen), followed by measurement with a flow cytometer. The results are shown in Fig. 19. As a result, it was found that the KUM2 cells were CD106-positive, while the BMSC cells were CD106-negative.

Regarding the occurrence of the expression of the CD44 antigen, an antibody reaction was carried out using an FITC-labeled anti-mouse CD44 antibody (PM-28154, manufactured by Pharmingen), followed by measurement with a flow cytometer. The results are shown in Fig. 20. As a result, it was found that the KUM2 cells and the BMSC cells were both CD44-positive.

Table 1 shows the summarized analytical data obtained using the flow cytometer.

**Table 1**

| | KUM2 | BMSC |
|---|---|---|
| Hemato | | |
| CD34 | - | ±^{*1} |
| CD117(c-kit) | - | + |
| CD14 | + | - |
| CD45 | - | - |
| CD90(Thyl) | - | - |
| Ly-6a/e(Sca1) | + | + |
| Ly6c | + | + |
| Ly6g | - | - |

| Endothelial | | |
|---|---|---|
| Flk-1 | - | - |
| CD31 | - | - |
| CD105 | - | - |
| CD144 | - | +^{*2} |

| Mesenchyaml | | |
|---|---|---|
| CD140(PDGFR) | + | + |

| Integrin | | |
|---|---|---|
| CD49b(α2) | + | - |
| CD49b(α4) | - | - |
| CD29(β1) | + | + |

| Matrix | | |
|---|---|---|
| CD54(ICAM-1) | + | - |
| CD102(ICAM-2) | - | - |
| CD106(VCAM-1) | + | - |
| CD44(Hyaluronate) | + | + |

| | | |
|---|---|---|
| *1: mixture; | | |
| 2*: weak positive | | |

### Example 11

### Concentration of differentiation precursor cells using mouse MLC2v promoter:

In order to efficiently obtain cells having the potential to differentiate into myocardium from mouse bone marrow-derived cells having the potential to differentiate into cardiomyocytes, a promoter expression system of a mouse MLC2v (myosin light chain-2v) gene showing cardiomyocyte-specific expression was constructed. Specifically, an EGFP gene (manufactured by CLONTECH) was ligated to the downstream of the promoter sequence of the mouse MLC2v gene followed by constructing a pMLC-2-EGFP plasmid containing the expression unit of neomycin-resistance gene. DNA of this plasmid was obtained by the alkali neutralization method described in *Molecular Cloning, A Laboratory Manual,* 2nd ed. *etc.*

2 µg of the above-described DNA was introduced using LIPOFECTAMINE (manufactured by LIFE TECHNOLOGY) into KUM2 cells, which had been cultured in a 6-well plate to give 1×10⁵ cells. Detailed procedure was carried out in accordance with the manufacturer's instructions. Forty-eight hours after the gene transfection, G418 (manufactured by Sigma) was added to give a final concentration of 1 mg/ml followed by selecting survived cells which were transfected by the gene.

On the 14th day after the gene introduction, 5-aza-C was added to give a final concentration of 3 µM, and 24 hours thereafter, the medium was replaced and the differentiation was induced. From the day 3 after the induction of the differentiation, GFP-positive cells were observed. On the day 4 after the induction of the differentiation, GFP-positive cells were exclusively selected from among 1×10⁴ cells using an FACS Caliber (manufactured by Becton Dickinson) and the culturing was further continued. As a result, 90% or more cells had differentiated into cells having a myotube-like structure, which indicates that cells with differentiation potency could be efficiently concentrated. After collecting by FACS, these GFP-positive cells were transplanted in accordance with the method of Example 10. As a result, these cells differentiated not into hemoendothelium but specifically into muscle tissues such as skeletal muscle and myocardium.

### Example 12

### Induction of adipocytes from mouse bone marrow-derived cells having the potential to differentiate into cardiomyocytes:

Bone marrow cells (BMSC) having the potential to differentiate into cardiomyocytes can be induced to differentiate not only into cardiomyocytes but also into adipocytes. To control the differentiation into adipocytes, the conditions for the induction of the differentiation were examined. First, the expression of PPAR-γ receptors was analyzed by the quantitative PCR method. As a result, it was found that PPAR-γ1 receptor was expressed but PPARγ2 receptor was not expressed in the BMSCs. Subsequently, PPAR-γ receptor agonists, pioglitazone and troglitazone, were added at various concentrations to bone marrow cells (BMSC) having the potential to differentiate into cardiomyocytes. As a result, the differentiation into adipocytes was promoted, depending on the concentration, and about 50% and 100% of the BMSCs differentiated into adipocytes respectively at 0.4 µM and 2 µM.

### Example 13

### Induction of differentiation into neurocytes, hapatocytes and cardiomyocytes of mouse bone marrow-derived cells having the potential to differentiate into cardiomyocytes by transplantation into blastocysts:

In order to obtain stable transformants of GFP-labeled bone marrow cells (BMSC) having the potential to differentiate into cardiomyocytes, gene transfection was first performed in the following manner.

GFP was introduced into a retrovirus vector plasmid pCLNCX (manufactured by Imgenex) to prepare pCLNC-GFP. The retrovirus vector plasmid pCLNC-GFP and a pCMV-Eco plasmid vector (manufactured by Imgenex) capable of expressing an ecotropic gene were treated by the alkali neutralization method and the PEG precipitation method described in *Molecular Cloning, A Laboratory Manual,* 2nd ed. *etc.* to obtain DNAs of high purity.

A day before the transfection of these DNAs, 293 cells carrying gag and pol genes, which had become confluent were subculured into a 10 cm dish by a dilution ratio of 1/5 and cultured at 37°C in a 5% CO₂-incubator.

The transfection was carried out in the following manner.

In 0.5 ml of 250 mM CaCl₂ (pH 6.95), 5 µg of the pCLNC-GFP retrovirus vector plasmid DNA and 5 µg of the pCMV-Eco plasmid vector DNA were dissolved. The solution thus obtained was dropped into 0.5 ml of 2× BBS (50 mM BES (N,N-bis(2-hydroxyethl)-2-aminoethanesulfonci acid), 280 mM NaCl, 1.5 mM Na₂HPO₄ (pH 6.95)) in a 15 ml tube and allowed to stand at room temperature for 10 minutes. Subsequently, the DNA solution was dropped into the medium of the 293 cells prepared on the previous day and cultured at 37°C in a 5% CO₂-incubator. On the next day, the medium was replaced and culture was further continued at 37°C in a 5% CO₂-incubator.

Two days after the replacement of the medium, the culture supernatant was filtered through a 0.45 µm filter (manufactured by Millipore) and a solution containing the virus vector was collected.

The mouse bone marrow cells (BMSC) having the potential to differentiate into cardiomyocytes, into which the virus vector was introduced, were plated into a 6-well dish at a density of 2×10⁴ cells/well on the previous day of the infection with the virus.

Hexadimethrine bromide(polybrene) (manufactured by Sigma) was added to the virus vector-containing solution obtained above to give a final concentration of 8 µg/ml. After replacing by the medium of the mouse bone marrow cells (BMSC) having the potential to differentiate into cardiomyocytes, followed by culturing at 33°C in a 5% CO₂-incubator. Five hours thereafter, the medium was replaced with fresh IMDM, followed by further culturing at 33°C in a 5% CO₂-incubator.

After culturing for two days, G418 was added until the final concentration of G418 came to be 300 µg/ml, followed by further culturing for further 7 days. During this period, a part of the cells died to be suspended. The surviving cells were suspended with trypsin and scattered in a fresh culture dish.

The obtained GFP-labeled bone marrow-derived cells having the potential to differentiate into cardiomyocytes were grown in a 6-cm culture dish. After eliminating the medium, 0.5 ml of 0.25% trypsin EDTA was added and the treatment was carried out for 1 minute. Then, 1.5 ml of a fresh medium was added and the cells were suspended. After adding feral bovine serum (manufactured by Lexicon Genetics) and mixing, the cell suspension was poured into mouse blastocyst. The mouse balstocysts were obtained by spontaneously mating female C57B1/6J mice subjected to hyper-ovulation with male mice of the same line, taking out the uterus 4 days thereafter, and perfusing the inside of the uterus with M15 medium. After allowing to stand at 37°C under 5% CO₂ until the balstocyst cavities sufficiently dilated, the balstocyst were transferred into M15 medium containing 20 mM HEPES which was cooled to about 4°C. Then, 10 to 15 BMSCs were microscopically injected into each balstocyst cavity while observing under an inverted microscope (manufactured by Nikon) provided with a microinjector (manufactured by Narumo Kagaku) and a micromanipulator (manufactured by Narumo Kagaku). After allowing to stand at 37°C under 5% CO₂ until the balstocyst cavities sufficiently dilated, the blastocysts were transplanted into the oviducal side of the uterus of female MCH mice with pseudopregnancy, followed by implantation. The female MCH mice with pseudopregnancy were prepared by mating with vasoligated male MCH mice aged 10 weeks or more on 17:00 three days before the transplantation at the ratio of 1:1. On 9:00 on the next morning, vaginal plugs were confirmed, and two days thereafter, the female mice were used for the above-described purpose.

The mice thus born were sacrificed and organs were extirpated for observing the expression of GFP. As a result, the expression of GFP was observed in the brain and the liver, which suggested that the BMSCs had differentiated into the nerve system and the liver. Genomic DNA was obtained from the heart taken out from another individual and subjected to PCR using the primers of SEQ ID NOS:79 and 80. As a result, it was confirmed that BMSCs were also incorporated into the heart. These results indicate that BMSCs have a totipotency of differentiating into all of the three germ layers of nerve, heart and liver.

### Example 14

### Telomerase activity in mouse bone marrow cells having the potential to differentiate into cardiomyocytes:

The mouse bone marrow cells having the potential to differentiate into cardiomyocytes were examined for telomerase activity by the Telomeric Repeat Amplification Protocol (TRAP) method (TRAPeze Telomerase Detection kit, manufactured by Oncor). The measurement of the telomerase activity was carried out as described below according to, in principle, the manufacture's instructions. The mouse bone marrow cells having the potential to differentiate into cardiomyocytes which had been cultured in a 6-cm culture dish (about 10⁶ cells) were washed with PBS, followed by addition of 200 µl of 1× CHAPS solution. After being allowed to stand on ice for 30 minutes, the cells were recovered together with the solution to a 1.5 ml centrifuge tube and centrifuged at 14000 rpm for 20 minutes (4°C; himac CF15, manufactured by Hitachi, Ltd.). The supernatant was recovered as a cell extract and the protein content was determined using Protein Assay (manufactured by BioRad). The protein content of the cell extract made from the mouse bone marrow cells having the potential to differentiate into cardiomyocytes under the above conditions was found to be about 1 mg/ml.

The cell extract was then subjected to telomerase elongation reaction and PCR amplification according to the manufacture's instructions. As the Taq polymerase, EX Taq polymerase (manufactured by Takara Shuzo) was used. After completion of the reactions, the samples were mixed with a 1/10 volume of 10× stain solution (0.25% bromophenol blue, 0.25% xylene cyanol FF, and 30% glycerol) and subjected to electrophoresis or 12.5% polyacrylamide gel (prepared according to the manufacture's instructions of TRAPeze Telomerase Detection Kit) at a constant voltage of 250 mV. After the electrophoresis, the gel was stained with Cyber Green (FMC) and analyzed using a fluorescence image analyzer, FluoroImager (manufactured by Molecular Dynamics). The telomerase activity was detected in the samples of the cell extracts having final concentration of 0.4-4 µg/ml.

### Example 15

### Isolation and culturing of bone marrow cell having the potential to differentiate into cardiomyocyte from rat bone marrow:

Six female Wistar rats of five week age (SLC Japan) were subjected to cervical dislocation and then disinfected by sufficiently applying 70% ethanol. Next, the skin of each leg was incised in a broad range and muscles covering the thighbone and shinbone were cut out to obtain the thighbone and shinbone. The thus obtained thighbone and shinbone were transferred into a culture dish of 10 cm in diameter (manufactured by Iwaki Glass) filled with PBS (manufactured by Gibco BRL) and muscles and joints were completely removed. Next, both ends of these bones were cut out using scissors, and the contents of bone marrow were squeezed out with a water flow of a culture liquid (D-PBS, manufactured by Gibco BRL) using a 10 ml syringe (manufactured by Terumo) equipped with a 20G needle. The thus obtained cell mass was loosened into a homogeneous level by passing through the syringe. The thus obtained cell suspension was recovered into a 50 ml capacity centrifugation tube (manufactured by BECTON DICKINSON) and centrifuged at 1,500 rpm for 10 minutes (a low speed centrifuge manufactured by TOMY), and the precipitated cells were suspended in 6 ml of D-PBS. When the number of cells was counted using a modified Neubauer counting chamber, the recovered cells were 2.6×10⁹ in total. This result means that 1×10⁸ cells were recovered from one thighbone or shinbone. The thus recovered cells were diluted to a density of 1.3×10⁸ cells per 1 ml, 5 ml of the resulting suspension was overlaid on a 1.073 g/ml Percoll (manufactured by Amersham Pharmacia Biotech)/D-PBS solution (25 ml) which had been put into a 50 ml capacity centrifugation tube, followed by centrifugation at room temperature and at 3,100 rpm for 30 minutes. After the centrifugation, cells were recovered from the interface between the Percoll solution and cell suspension, diluted to 4 times with D-PBS and centrifuged at 2,300 rpm for 10 minutes and then the thus fractionated cells were recovered. The thus recovered cells were suspended in IMDM medium (manufactured by Gibco BRL) containing 20% FCS, 100 µg/ml penicillin, 250 ng/ml streptomycin and 85 µg/ml amphotericin (manufactured by Gibco BRL). When the number of cells at this stage was again counted, the recovered bone marrow cells were 4.7×10⁷ in total, meaning that cells corresponding to about 2% of the cells before the treatment were recovered. The fractionated bone marrow cells were plated on three culture dishes for animal cells having a diameter of 10 cm (manufactured by Iwaki Glass, hereinafter referred to as "10-cm culture dish") to a density of 2 to 5×10⁵ cells/cm² and cultured at 33°C in a 5% CO₂-incubator (manufactured by Tabai). A half volume of the medium was exchanged with a fresh medium after 24 hours and 72 hours. Three or 4 days thereafter, a half volume of the medium was exchanged with a fresh medium. Since colonies became dense after a lapse of 15 days, the cells were removed with a trypsin EDTA treatment and a 2/3 part of them was suspended in 4 ml of a stock solution (10% DMSO, 50% bone marrow cell culture supernatant and 40% the above medium which had not been used), dispensed in 1 ml portions into 2 ml capacity tubes (manufactured by Sumitomo Bakelite) and stored in a freezer, and the remaining 1/3 part was again inoculated into two 10-cm culture dishes and subcultured.

### Example 16

### Evaluation of rat bone marrow-derived cell having the potential to differentiate into cardiomyocyte:

The rat bone marrow cells subcultured in the above were again removed with the trypsin EDTA treatment when they became dense and inoculated into a 6 well plate (manufactured by BECTON DICKINSON) in 5×10⁴ cells per well or into a 6 cm diameter culture dish coated with human fibronectin (Biocoat, manufactured by BECTON DICKINSON) in a density of 1.3×10⁵ cells. One day thereafter, culturing was carried out under two different conditions, one in which only 5-azacytidine (manufactured by Sigma, 10 µM in final concentration) was added, and another in which 5-azacytidine, PDGF-BB (manufactured by Pepro Tech EC LTD, 10 ng/ml in final concentration) and all-trans retinoic acid (RA, manufactured by Sigma, 10⁻⁹ M in final concentration) were added, and the medium was exchanged after 2 days of the culturing (in the latter conditions, PDGF and all-trans retinoic acid were again added at the time of the medium exchange and after 2 days and 4 days). Three or 4 days thereafter, the medium was exchanged, followed by culturing for 3 weeks. As a result, differentiation of myotube-like cells was observed in the conditions in which 5-azacytidine, PDGF-BB and retinoic acid were added.

### Example 17

### Forced expression of transcription factor MesP1 and enhancement of cardiomyocyte differentiation by addition of cytokine:

Influences of forced expression of a cardiomyocyte differentiation-related transcription factor MesP1 in a bone marrow-derived pluripotent stem cell (BMSC) having the potential to differentiate into cardiomyocytes upon its differentiation into cardiomyocytes and influences of a combination of forced expression of MesP1 with cytokine (FGF-8, ET-1, Midkine or BMP4) upon differentiation into cardiomyocytes were examined.

A mouse bone marrow-derived pluripotent stem cell (BMSC-MesP1) having the potential to differentiate into cardiomyocytes in which the MesP1 gene was forced-expressed was obtained using a retrovirus vector in the same manner as in Example 6, and then the differentiation was induced to examine efficiency of differentiation into cardiomyocytes.

The bone marrow cell (BMSC-MesP1) having the potential to differentiate into cardiomyocytes in which MesP1 was forced expressed was plated into a 60-mm culture dish in a density of 2×10⁴ cells/ml and cultured at 33°C in a 5% CO₂-incubator. On the next day, 5-aza-C was added to the culture medium to give a final concentration of 3 µM, followed by culturing under five different conditions, namely (i) addition of FGF-8 to give a final concentration of 10 ng/ml (culture dish N), (ii) addition of ET-1 to give a final concentration of 10 ng/ml (culture dish P), (iii) addition of Midkine to give a final concentration of 10 ng/ml (culture dish Q), (iv) addition of BMP4 to give a final concentration of 10 ng/ml (culture dish R), and (v) no addition (culture dish S).

On the next day, the medium was exchanged with a fresh medium in order to eliminate 5-aza-C from the medium, and then the culturing was continued by adding FGF-8 to the culture dish N to give a final concentration of 10 ng/ml, ET-1 to the culture dish P to give a final concentration of 10 ng/ml, Midkine to the culture dish Q to give a final concentration of 10 ng/ml and BMP4 to the culture dish R to give a final concentration of 10 ng/ml. Two days and 4 days thereafter, the medium exchange and addition of FGF-8, ET-1, Midkine or BMP4 were carried out similarly.

Four weeks after the addition of 5-aza-C, morphology of the cells was observed under a phase contrast microscope. As a result, the number of myotube-like cells was not changed greatly by the forced expression of MesP1 In addition, about 50% of the cells became myotube-like cells in the culture dish to which FGF-8, ET-1, Midkine or BMP4 had been added.

Next, RNA was recovered from the thus obtained myotube-like cells, and genes expressing in the myotube-like cells were analyzed by quantitative PCR using the synthetic oligonucleotides shown in SEQ ID NOS:71 to 78. As a result, expression of ANP as a gene specific for a myocardium was accelerated by the forced expression of MesP1 On the other hand, FGF-8, ET-1, Midkine or BMP4 did not further accelerate the expression of ANP accelerated by the forced expression of MesP1.

### INDUSTRIAL APPLICABILITY

The present invention provides a bone marrow cell, a growth factor, a vitamin and an adhesion molecule which are effective for treating a heart disease accompanied with destruction and denaturation of a cardiomyocyte and for screening a therapeutic agent for it, and application methods thereof.

### FREE TEXT OF SEQUENCE LISTINGS:

SEQ ID NO:33-Explanation of artificial sequence: Synthetic DNA
SEQ ID NO:34-Explanation of artificial sequence: Synthetic DNA
SEQ ID NO:35-Explanation of artificial sequence: Synthetic DNA
SEQ ID NO:36-Explanation of artificial sequence: Synthetic DNA
SEQ ID NO:37-Explanation of artificial sequence: Synthetic DNA
SEQ ID NO:38-Explanation of artificial sequence: Synthetic DNA
SEQ ID NO:39-Explanation of artificial sequence: Synthetic DNA
SEQ ID NO:40-Explanation of artificial sequence: Synthetic DNA
SEQ ID NO:41-Explanation of artificial sequence: Synthetic DNA
SEQ ID NO:42-Explanation of artificial sequence: Synthetic DNA
SEQ ID NO:43-Explanation of artificial sequence: Synthetic DNA
SEQ ID NO:44-Explanation of artificial sequence: Synthetic DNA
SEQ ID NO:45-Explanation of artificial sequence: Synthetic DNA
SEQ ID NO:46-Explanation of artificial sequence: Synthetic DNA
SEQ ID NO:47-Explanation of artificial sequence: Synthetic DNA
SEQ ID NO:48-Explanation of artificial sequence: Synthetic DNA
SEQ ID NO:49-Explanation of artificial sequence: Synthetic DNA
SEQ ID NO:50-Explanation of artificial sequence: Synthetic DNA
SEQ ID NO:51-Explanation of artificial sequence: Synthetic DNA
SEQ ID NO:52-Explanation of artificial sequence: Synthetic DNA
SEQ ID NO:53-Explanation of artificial sequence: Synthetic DNA
SEQ ID NO:54-Explanation of artificial sequence: Synthetic DNA
SEQ ID NO:55-Explanation of artificial sequence: Synthetic DNA
SEQ ID NO:56-Explanation of artificial sequence: Synthetic DNA
SEQ ID NO:57-Explanation of artificial sequence: Synthetic DNA
SEQ ID NO:58-Explanation of artificial sequence: Synthetic DNA
SEQ ID NO:59-Explanation of artificial sequence: Synthetic DNA
SEQ ID NO:60-Explanation of artificial sequence: Synthetic DNA
SEQ ID NO:61-Explanation of artificial sequence: Synthetic DNA
SEQ ID NO:62-Explanation of artificial sequence: Synthetic DNA
SEQ ID NO:63-Explanation of artificial sequence: Synthetic DNA
SEQ ID NO:64-Explanation of artificial sequence: Synthetic DNA
SEQ ID NO:65-Explanation of artificial sequence: Synthetic DNA
SEQ ID NO:66-Explanation of artificial sequence: Synthetic DNA
SEQ ID NO:67-Explanation of artificial sequence: Synthetic DNA
SEQ ID NO:68-Explanation of artificial sequence: Synthetic DNA
SEQ ID NO:69-Explanation of artificial sequence: Synthetic DNA
SEQ ID NO:70-Explanation of artificial sequence: Synthetic DNA
SEQ ID NO:71-Explanation of artificial sequence: Synthetic DNA
SEQ ID NO:72-Explanation of artificial sequence: Synthetic DNA
SEQ ID NO:73-Explanation of artificial sequence: Synthetic DNA
SEQ ID NO:74-Explanation of artificial sequence: Synthetic DNA
SEQ ID NO:75-Explanation of artificial sequence: Synthetic DNA
SEQ ID NO:76-Explanation of artificial sequence: Synthetic DNA
SEQ ID NO:77-Explanation of artificial sequence: Synthetic DNA
SEQ ID NO:78-Explanation of artificial sequence: Synthetic DNA
SEQ ID NO:79-Explanation of artificial sequence: Synthetic DNA
SEQ ID NO:80-Explanation of artificial sequence: Synthetic DNA

## Claims

1. A cell which has been isolated from a living tissue or umbilical blood, and which has the potential to differentiate into at least a cardiomyocyte.

2. The cell according to claim 1, wherein the living tissue is bone marrow.

3. The cell according to claim 1 or 2, wherein the cell is a multipotential stem cell.

4. The cell according to any one of claims 1 to 3, wherein the cell is a multipotential stem cell which differentiates into at least a cardiomyocyte and a vascular endothelial cell.

5. The cell according to any one of claims 1 to 4, wherein the cell is a multipotential stem cell which differentiates into at least a cardiomyocyte, an adipocyte, a skeletal muscle cell, an osteoblast, and a vascular endothelial cell.

6. The cell according to any one of claims 1 to 5, wherein the cell is a multipotential stem cell which differentiates into at least a cardiomyocyte, an adipocyte, a skeletal muscle cell, an osteoblast, a vascular endothelial cell, a nervous cell, and a hepatic cell.

7. The cell according to any one of claims 1 to 3, wherein the cell is a multipotential stem cell which differentiates into any cell in adult tissues.

8. The cell according to any one of claims 1 to 7, wherein the cell is CD117-positive and CD140-positive.

9. The cell according to claim 8, wherein the cell is further CD34-positive.

10. The cell according to claim 9, wherein the cell is further CD144-positive.

11. The cell according to claim 9, wherein the cell is further CD140-negative.

12. The cell according to claim 8, wherein the cell is CD34-negative.

13. The cell according to claim 12, wherein the cell is further CD144-positive.

14. The cell according to claim 12, wherein the cell is further CD144-negative.

15. The cell according to claim 10, wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.

16. The cell according to claim 11, wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.

17. The cell according to claim 12, wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.

18. The cell according to claim 13, wherein the cell is further CD14-negative, CD45-negative, CD90-negative, Flk-1-negative, CD31-negative, CD105-negative, CD49b-negative, CD49d-negative, CD29-positive, CD54-negative, CD102-negative, CD106-negative, and CD44-positive.

19. The cell according to claim 1, which does not take up Hoechst 33342.

20. A cardiomyocyte precursor which differentiates into only cardiomyocyte induced from the cell according to any one of claims 1 to 19.

21. The cell according to any one of claims 1 to 20, which has the potential to differentiate into a ventricular cardiac muscle cell.

22. The cell according to any one of claims 1 to 20, which has the potential to differentiate into a sinus node cell.

23. The cell according to any one of claims 1 to 20, wherein the vital tissue or umbilical blood is derived from a mammal.

24. The cell according to claim 23, wherein the mammal is selected from the group consisting of a mouse, a rat, a guinea pig, a hamster, a rabbit, a cat, a dog, a sheep, a swine, cattle, a goat and a human.

25. The cell according to any one of claims 1 to 8, which is mouse bone marrow-derived multipotential stem cell BMSC (FERM BP-7043).

26. The cell according to any one of claims 1 to 25, which has the potential to differentiate into a cardiomyocyte by demethylation of a chromosomal DNA of the cell.

27. The cell according to claim 26, wherein the demethylation is carried out by at least one selected from the group consisting of demethylase, 5-azacytidine, and dimethyl sulfoxide, DMSO.

28. The cell according to claim 27, wherein the demethylase comprises the amino acid sequence represented by SEQ ID NO:1.

29. The cell according to any one of claims 1 to 28, wherein the differentiation is accelerated by a factor which is expressed in a cardiogenesis region of a fetus or a factor which acts on differentiation into a cardiomyocyte in a cardiogenesis stage of a fetus.

30. The cell according to claim 29, wherein the factor which is expressed in a cardiogenesis region of a fetus or the factor which acts on differentiation into a cardiomyocyte in a cardiogenesis stage of a fetus is at least one selected from the group consisting of a cytokine, an adhesion molecule, a vitamin, a transcription factor, and an extracellular matrix.

31. The cell according to claim 30, wherein the cytokine is at least one selected from the group consisting of a platelet-derived growth factor, PDGF; a fibroblast growth factor-8, FGF-8; an endothelin 1, ET1; a midkine; and a bone morphogenetic factor, BMP-4.

32. The cell according to claim 31, wherein the PDGF, FGF-8, Bill, midkine, and BMP-4 comprise the amino acid sequence represented by SEQ ID NO:3 or 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively.

33. The cell according to claim 30, wherein the adhesion molecule is at least one selected from the group consisting of a gelatin, a laminin, a collagen, and a fibronectin.

34. The cell according to claim 30, wherein the vitamin is retinoic acid.

35. The cell according to claim 30, wherein the transcription factor is at least one selected from the group consisting of Nkx2.5/Csx, GATA4, MEF-2A, MEF-2B, MEF-2C, MEF-2D, dHAND, eHAND, TEF-1, TEF-3, TEF-5, and MesP1.

36. The cell according to claim 35, wherein the Nkx2.5/Csx, GATA4, MEF-2A, MEF-2B, MEF-2C, MEF-2D, dHAND, eHAND, TEF-1, TEF-3, TEF-5, and MesP1 comprise the amino acid sequence represented by SEQ ID NO:9, the amino acid sequence represented by SEQ ID NO:11, the amino acid sequence represented by SEQ ID NO:13, the amino acid sequence represented by SEQ ID NO:15, the amino acid sequence represented by SEQ ID NO:17, the amino acid sequence represented by SEQ ID NO:19, the amino acid sequence represented by SEQ ID NO:21, the amino acid sequence represented by SEQ ID NO:23, the amino acid sequence represented by SEQ ID NO:25, the amino acid sequence represented by SEQ ID NO:27, the amino acid sequence represented by SEQ ID NO:29, and the amino acid sequence represented by SEQ ID NO:62, respectively.

37. The cell according to claim 30, wherein the extracellular matrix is an extracellular matrix derived from a cardiomyocyte.

38. The cell according to any one of claims 1 to 28, wherein the differentiation is inhibited by a fibroblast growth factor-2, FGF-2.

39. The cell according to claim 38, wherein the FGF-2 comprises the amino acid sequence represented by SEQ ID NO:7 or 8.

40. The cell according to any one of claims 1 to 28, which is capable of differentiating into a cardiomyocyte or a blood vessel by transplantation into a heart.

41. The cell according to any one of claims 1 to 19, which is capable of differentiating into a cardiac muscle by transplantation into a blastocyst or by co-culturing with a cardiomyocyte.

42. The cell according to any one of claims 1 to 28, which is capable of differentiating into an adipocyte by an activator of a nuclear receptor, PPAR-γ.

43. The cell according to claim 42, wherein the activator is a compound having a thiazolidione skeleton.

44. The cell according to claim 43, wherein the compound is at least one selected from the group consisting of troglitazone, pioglitazone, and rosiglitazone.

45. The cell according to any one of claims 1 to 28, which is capable of differentiating into a nervous cell by transplantation into a blastocyst or by transplantation into an encephalon or a spinal cord.

46. The cell according to any one of claims 1 to 28, which is capable of differentiating into a hepatic cell by transplantation into a blastocyst or by transplantation into a liver.

47. A method for differentiating the cell according to any one of claims 1 to 28 into a cardiac muscle, comprising using a chromosomal DNA-dimethylating agent.

48. A method for redifferentiating the cell according to claim 9 into the cell according to 12, comprising using a chromosomal DNA-dimethylating agent.

49. A method for redifferentiating a cell which is CD117-negative and CD140-positive into the cell according to claim 8, comprising using a chromosomal DNA-dimethylating agent.

50. The method according to claim 48 or 49, wherein the chromosomal DNA-dimethylating agent is selected from the group consisting of a demethylase, 5-azacytidine, and DMSO.

51. The method according to claim 50, wherein the demethylase comprises the amino acid sequence represented by SEQ ID NO:1.

52. A method for differentiating the cell according to any one of claims 1 to 28 into a cardiac muscle, comprising using a factor which is expressed in a cardiogenesis region of a fetus or a factor which acts on differentiation into a cardiomyocyte in a cardiogenesis stage of a fetus.

53. The method according to claim 52, wherein the factor which is expressed in a cardiogenesis region of a fetus or the factor which acts on differentiation into a cardiomyocyte in a cardiogenesis stage of a fetus is at least one selected from the group consisting of a cytokine, an adhesion molecule, a vitamin, a transcription factor, and an extracellular matrix.

54. The method according to claim 53, wherein the cytokine is at least one selected from the group consisting of a platelet-derived growth factor, PDGF; a fibroblast growth factor-8, FGF-8; an endothelin 1, ET1; a midkine; and a bone morphogenetic factor, BMP-4.

55. The method according to claim 54, wherein the PDGF, FGF-8, ET1, midkine, and BMP-4 comprise the amino acid sequence represented by SEQ ID NO:3 or 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively.

56. The method according to claim 53, wherein the adhesion molecule is at least one selected from the group consisting of a gelatin, a laminin, a collagen, and a fibronectin.

57. The method according to claim 53, wherein the vitamin is retinoic acid.

58. The method according to claim 53, wherein the transcription factor is at least one selected from the group consisting of Nkx2.5/Csx, GATA4, MEF-2A, MEF-2B, MEF-2C, MEF-2D, dHAND, eHAND, TEF-1, TEF-3, TEF-5, and MesP1.

59. The method according to claim 58, wherein the Nkx2.5/Csx, GATA4, MEF-2A, MEF-2B, MEF-2C, MEF-2D, dHAND, eHAND, TEF-1, TEF-3, TEF-5, and MesP1 comprise the amino acid sequence represented by SEQ ID NO:9, the amino acid sequence represented by SEQ ID NO:11, the amino acid sequence represented by SEQ ID NO:13, the amino acid sequence represented by SEQ ID NO:15, the amino acid sequence represented by SEQ ID NO:17, the amino acid sequence represented by SEQ ID NO:1 the amino acid sequence represented by SEQ ID NO:21, the amino acid sequence represented by SEQ ID NO:23, the amino acid sequence represented by SEQ ID NO:25, the amino acid sequence represented by SEQ ID NO:27, the amino acid sequence represented by SEQ ID NO: 29, the amino acid sequence represented by SEQ ID NO:62, respectively.

60. The method according to claim 53, wherein the extracellular matrix is an extracellular matrix derived from a cardiomyocyte.

61. A method for differentiating the cell according to any one of claims 1 to 28 into an adipocyte, comprising using an activator of a nuclear receptor, PPAR-γ.

62. The method according to claim 61, wherein the activator is a compound having a thiazolidione skeleton.

63. The method according to claim 62, wherein the compound is at least one selected from the group consisting of troglitazone, pioglitazone, and rosiglitazone.

64. A myocardium-forming agent, comprising, as an active ingredient, a chromosomal DNA-demethylating agent.

65. The myocardium-forming agent according to claim 64, wherein the chromosomal DNA-demethylating agent is at least one selected from the group consisting of a demethylase, 5-azacytidine, and DMSO.

66. The myocardium-forming agent according to claim 65, wherein the demethylase comprises the amino acid sequence represented by SEQ ID NO:1.

67. A myocardium-forming agent, comprising, as an active ingredient, a factor which is expressed in a cardiogenesis region of a fetus or a factor which acts on differentiation into a cardiomyocyte in a cardiogenesis stage of a fetus.

68. The myocardium-forming agent according to claim 67, wherein the factor which is expressed in a cardiogenesis region of a fetus or the factor which acts on differentiation into a cardiomyocyte in a cardiogenesis stage of a fetus is at least one selected from the group consisting of a cytokine, an adhesion molecule, a vitamin, a transcription factor, and an extracellular matrix.

69. The myocardium-forming agent according to claim 68, wherein the cytokine is at least one selected from the group consisting of a platelet-derived growth factor, PDGF; a fibroblast growth factor-8, FGF-8; an endothelin 1, ET1; a midkine; and a bone morphogenetic factor, BMP-4.

70. The myocardium-forming agent according to claim 69, wherein the PDGF, FGF-8, ET1, midkine, and BMP-4 comprise the amino acid sequence represented by SEQ ID NO:3 or 5, the amino acid sequence represented by SEQ ID NO:64, the amino acid sequence represented by SEQ ID NO:66, the amino acid sequence represented by SEQ ID NO:68, and the amino acid sequence represented by SEQ ID NO:70, respectively.

71. The myocardium-forming agent according to claim 68, wherein the adhesion molecule is selected from the group consisting of a gelatin, a laminin, a collagen, and a fibronectin.

72. The myocardium-forming agent according to claim 71, wherein the vitamin is retinoic acid.

73. The myocardium-forming agent according to claim 68, wherein the transcription factor is at least one selected from the group consisting of Nkx2.5/Csx, GATA4, MEF-2A, MEF-2B, MEF-2C, MEF-2D, dHAND, eHAND, TEF-1, TEF-3, TEF-5, and MesP1.

74. The myocardium-forming agent according to claim 73, wherein the Nkx2.5/Csx, GATA4, MEF-2A, MEF-2B, MEF-2C, MEF-2D, dHAND, eHAND, TEF-1, TEF-3, TEF-5, and MesP1 comprise the amino acid sequence represented by SEQ ID NO:9, the amino acid sequence represented by SEQ ID NO:11, the amino acid sequence represented by SEQ ID NO:13, the amino acid sequence represented by SEQ ID NO:15, the amino acid sequence represented by SEQ ID NO:17, the amino acid sequence represented by SEQ ID NO:19, the amino acid sequence represented by SEQ ID NO:21, the amino acid sequence represented by SEQ ID NO:23, the amino acid sequence represented by SEQ ID NO:25, the amino acid sequence represented by SEQ ID NO:27, the amino acid sequence represented by SEQ ID NO:29, and the amino acid sequence represented by SEQ ID NO:62, respectively.

75. The myocardium-forming agent according to claim 68, wherein the extracellular matrix is an extracellular matrix derived from a cardiomyocyte.

76. A method for regenerating a heart damaged by a heart disease, comprising using the cell according to any one of claims 1 to 46.

77. An agent for cardiac regeneration, comprising, as an active ingredient, the cell according to any one of claims 1 to 46.

78. A method for specifically transfecting a wild-type gene corresponding to a mutant gene in a congenital genetic disease to a myocardium, comprising using the cell according to any one of claims 1 to 46 into which the wild-type gene corresponding to a mutant gene in a congenital genetic disease of a heart has been introduced.

79. A therapeutic agent for a heart disease, comprising, as an active ingredient, the cell according to any one of claims 1 to 46 into which a wild-type gene corresponding to a mutant gene in a congenital genetic disease of a heart has been introduced.

80. A method for producing an antibody which specifically recognizes the cell according to any one of claims 1 to 46, comprising using the cell as an antigen.

81. A method for isolating a cell having the potential to differentiate into a cardiomyocyte according to any one of claims 1 to 46, comprising using an antibody obtained by the method according to claim 80.

82. A method for obtaining a surface antigen specific for the cell according to any one of claims 1 to 46, comprising using the cell.

83. A method for screening a factor which proliferates the cell according to any one of claims 1 to 46, comprising using the cell.

84. A method for screening a factor which induces the cell according to any one of claims 1 to 46 to differentiate into a cardiomyocyte, comprising using the cell.

85. A method for screening a factor which immortalizes the cell according to any one of claims 1 to 46, comprising using the cell.

86. A method for immortalizing the cell according to any one of claims 1 to 46, comprising expressing a telomerase in the cell.

87. The method according to claim 86, wherein the telomerase comprises the amino acid sequence represented by SEQ ID NO:31.

88. A therapeutic agent for a heart disease, comprising, as an active ingredient, the cell according to any one of claims 1 to 46 which has been immortalized by expressing a telomerase.

89. The therapeutic agent according to claim 88, wherein the telomerase comprises the amino acid sequence represented by SEQ ID NO:31

90. A culture supernatant comprising the cell according to any one of claims 1 to 46.

91. A method for inducing the cell according to any one of claims 1 to 46 to differentiate into a cardiomyocyte, comprising using the culture supernatant according to claim 90.
